# EUROPEAN PATENT SPECIFICATION

(11) **EP 3 240 494 B1**
(45) Date of publication and mention of the grant of the patent: **17.03.2021**
(21) Application number: 15876302.9
(22) Date of filing: 30.12.2015
(51) Int. Cl.: A61M 25/10, A61M 25/00, A61B 17/22, A61B 18/24

(54) **LASER-INDUCED PRESSURE WAVE EMITTING CATHETER SHEATH**
KATHETERSCHAFT MIT LASERINDUZIERTER DRUCKWELLENEMISSION
GAINE DE CATHÉTER ÉMETTANT DES ONDES DE PRESSION INDUITES PAR LASER

(30) Priority: 30.12.2014 US 201462098242 P; 25.08.2015 US 201562209691 P; 31.08.2015 US 201562212242 P; 30.10.2015 US 201562248753 P; 30.10.2015 US 201562248936 P; 08.12.2015 US 201562264725 P; 17.12.2015 US 201562268797 P
(43) Date of publication of application: 08.11.2017
(73) Proprietor: The Spectranetics Corporation, Colorado Springs CO 80921 (US)
(72) Inventor: GRACE, Kenneth, P., Woodland Park, Colorado 80863 (US); TRIFFO, Thomas, Colorado Springs, Colorado 80923 (US); CEZO, James, Colorado Springs, Colorado 80907 (US)
(74) Representative: de Haan, Poul Erik
(86) International application number: PCT/US2015/068161
(87) International publication number: WO 2016/109731

(56) References cited:
- EP-A2- 0 820 786
- WO-A1-91/10403
- JP-A- 2004 215 862
- JP-A- 2009 061 083
- US-A- 5 722 979
- US-A1- 2003 009 157
- US-A1- 2012 303 011
- US-A1- 2014 133 814
- US-A1- 2014 276 682
- US-B2- 8 790 386

## Description

### FIELD

The present disclosure relates generally to the use of medical devices for the treatment of vascular conditions. In particular, the present disclosure provides materials and methods for using laser-induced pressure waves to disrupt vascular blockages and to deliver therapeutic agents to the blockage area.

### BACKGROUND

Arterial disease is a common disease that affects millions of Americans. Coronary artery disease (CAD) most often results from a condition known as atherosclerosis, which generally manifests as the accumulation of a waxy substance on the inside of a subject's coronary arteries. This substance, called plaque, is made of cholesterol, fatty compounds, calcium, and a blood-clotting material called fibrin. Similarly, peripheral artery disease (PAD) often results from the accumulation of plaque on the inside of a subject's peripheral arteries, such as the arteries in a patient's arms, hands, legs and/or feet. As the plaque builds up, the artery narrows, or becomes stenotic, making it more difficult for blood to flow through the arteries. As the size of the stenosis increases and the blockage worsens, blood flow slows and upon the formation of a total occlusion, blood flow through the corresponding artery completely stops, which in turn may cause pain in the extremities and, in severe cases, gangrene, which may ultimately require amputation.

Balloon angioplasty and other transluminal medical treatments are well-known and have been proven efficacious in the treatment of stenotic lesions at the core of CAD and/or PAD, as long as the artery is only partially blocked and not totally blocked. In a typical angioplasty procedure to treat CAD, a catheter is inserted into the groin or arm of a subject and guided forward through the aorta and into the coronary arteries of the heart. The angioplasty catheter includes a balloon, which when placed within the partial occlusion, can be inflated, thereby dilating the occlusion and increasing the size of the diameter of the artery to provides more typical blood flow therethrough.

Over time, an occlusion, particularly a total occlusion, may calcify and/or becomes fibrous, thereby increasing the balloon's ability to dilate the occlusion. Certain types of catheters, such as electrically-induced shockwave balloon catheters, may be used to break the calcified tissue. An electrically-induced shockwave balloon catheter may include a liquid filled balloon and a one or more pairs of electrodes within the balloon. Upon creating a discharge across the electrodes, plasma is produced, which results in the formation of one or more cavitation bubbles. The cavitation bubbles created within the balloon cause the balloon to expand and contract. The expansion and contraction of the balloon creates a hydraulic force that transfers energy to the vascular occlusion and/or to the walls of the vessel in an amount sufficient to disrupt intraluminal as well as medial (within the tissue layer of the vascular wall) vascular obstructions (for example, calcium deposits). In addition to producing cavitation bubbles being upon the formation of plasma generated by the electrical reaction in the liquid, shockwaves are also produced. The shockwaves are transferred through the balloon and to the calcified occlusion, and the shockwaves break the calcified occlusion.

In the event a total stenotic occlusion forms, it may be difficult for the balloon to enter the stenosis. Additionally, if total occlusion calcifies and/or become fibrous, thereby increasing the hardness of occlusion, it may become even more difficult, if not impossible, to penetrate the occlusion and insert a balloon catheter. For example, the proximal and/or distal ends of the occlusion may become calcified to the point that "caps" or "calcified caps" are created, such that even an electrically-induced shockwave balloon catheter may be unable to penetrate the calcified total occlusion because the balloon must be within and adjacent the occlusion in order to operate. And because the balloon within an electrically-induced shockwave balloon catheter is typically proximal the distal end of the electrically-induced shockwave balloon catheter, it is unable to be inserted into or through the calcified cap of the total occlusion. In this context, reference is made to documents US2014/0133814 A1, US2003/0009157 A1, US2012/0303011 A1 and EP0820786 A2.

### SUMMARY

The invention is defined in the appended set of claims. What is needed is a device that is capable of penetrating a calcified and/or fibrous total occlusion, particularly a calcified cap(s), and destroying at least a portion of the occlusion as the device penetrates and traverses the total occlusion. What is also needed is a device that is capable of utilizing pressure waves to the vascular occlusion in order to disrupt the calcified and/or fibrous portions without applying a hydraulic force thereto. These and other needs are addressed by the various aspects, embodiments, and configurations of the present disclosure.

The present disclosure provides a catheter comprising an outer sheath having a proximal end and a distal end, wherein the distal end comprises a tip, an inner sheath may include at least one lumen, a proximal end and a distal end, wherein the inner sheath is disposed radially within the outer sheath, wherein the distal end of the inner sheath may be disposed proximate the tip, thereby creating a cavity among the outer sheath, inner sheath, , one or more optical fibers disposed within the inner sheath extending from a distal proximal portion of the inner sheath to the distal end of the inner sheath and into the cavity, wherein a proximal end of the one or more optical fibers is coupled to a laser generator, and at least one emitter coupled to the one or more optical fibers, wherein the at least one emitter is disposed within the cavity.

A catheter according to paragraph [0008], wherein the catheter further comprises one or more liquid medium ports disposed about the sheath.

A catheter according to any of paragraphs [0008]-[0009], wherein the outer sheath is capable of expanding and contracting in the axial direction.

A catheter according to any of paragraphs [0008]-[0010], wherein the outer sheath is constructed from the group consisting of a polymer, polymer with a coil or embedded with braid, polymer with an embedded braid and fluorinated ethylene propylene or lubricious flouropolymer liner, laser cut hypotube, tricoil or bicoil or any combination of the foregoing.

A catheter according to any of paragraphs [0008]-[0011], wherein the catheter further comprises a shield axially disposed within the cavity between the distal end of the inner sheath and the tip.

A catheter according to any of paragraphs [0008]-[0012], wherein the shield comprises a proximal end and a distal end, wherein the shield tapers from the proximal end to the distal end or the shield tapers from the distal end to the proximal end.

A catheter according to any of paragraphs [0008]-[0014], wherein the tip comprises a proximal end and a distal end, wherein the tip has a solid construction along its longitudinal axis from its proximal end to its distal end.

A catheter according to any of paragraphs [0008]-[0014], wherein the tip comprises an open construction at the distal end of the outer sheath.

A catheter according to any of paragraphs [0008]-[0015], wherein the tip comprises a proximal end and a distal end, wherein the tip has a hollow construction along its longitudinal axis from its proximal end to its distal end.

A catheter according to any of paragraphs [0008]-[0016], wherein the at least one emitter is configured to emit laser light energy at wavelengths of between about 300 nanometers to about 350 nanometers, at pulse durations between about 100 nanoseconds to about 150 nanoseconds, and at frequencies between about 1 pulse per second to about 250 pulses per second.

A catheter according to any of paragraphs [0008]-[0017], wherein the at least one emitter is configured to emit laser light energy at wavelengths of about 308 nanometers, at pulse durations between about 120 nanoseconds and about 140 nanoseconds, and at frequencies between about 1 pulse per second to about 80 pulses per second.

A catheter according to any of paragraphs [0008]-[0018], wherein total energy output for the at least one emitter is between about 1 to 100 mJ.

A catheter according to any of paragraphs [0008]-[0019], wherein the liquid medium is contrast medium or contrast solution.

A catheter according to any of paragraphs [0008]-[0020], wherein the liquid medium is any one of iodine-containing contrast medium or gadolinium contrast medium.

A catheter according to any of paragraphs [0008]-[0021], wherein the liquid medium is configured to exhibit high absorption of light energy emitted from the at least one emitter at wavelengths of between about 1 nanometer to about 1 millimeter, at pulse durations between about 1 nanosecond to about 1 second, and at frequencies between about 1 pulse per second to about 500 pulses per second.

A catheter according to any of paragraphs [0008]-[0022], wherein the at least one emitter is two or more concentric emitters.

A catheter according to any of paragraphs [0008]-[0023], wherein the at least one emitter is two or more single-fiber emitters.

A catheter according to any of paragraphs [0008]-[0024], wherein the at least one emitter is configured to translate within the outer sheath.

A catheter according to any of paragraphs [0008]-[0025], wherein the tip comprises a distal end and a flexible membrane at its distal end.

A catheter according to any of paragraphs [0008]-[0026], wherein the catheter further comprises one or more liquid medium ports disposed about the sheath.

A catheter according to any of paragraphs [0008]-[0027], wherein the emitters are configured to emit light energy (for example, laser light energy) at wavelengths of between about 300 nanometers to about 350 nanometers, at pulse durations between about 100 nanoseconds to about 150 nanoseconds, and at frequencies between about 1 pulse per second to about 250 pulses per second. In some cases, the emitters are configured to emit laser light energy at wavelengths of about 308 nanometers, at pulse durations between about 120 nanoseconds and about 140 nanoseconds, and at frequencies between about 25 pulses per second to about 80 pulses per second.

A catheter according to any of paragraphs [0008]-[0028], wherein the liquid medium is configured to absorb light energy, create a pressure wave and/or cavitation bubbles and produce additional resultant pressure waves to disrupt a vascular obstruction, modify the vascular surface to enhance drug absorption including but not limited to fracturing both intimal (or intraluminal) and medial calcium deposits, and/or to deliver a drug to an area around a vascular obstruction. Liquid medium can include contrast medium, including for example, iodine-containing contrast medium or gadolinium contrast medium, as well as contrast solutions comprising dye(s) and/or particle(s).

A catheter according to any of paragraphs [0008]-[0029], wherein the inner sheath further comprises a first guidewire lumen, and further comprising a sealable valve coupled to the outer sheath, the sealable valve having a second guidewire lumen and a seal, whereupon introducing a guidewire into the first guidewire lumen and the second guidewire lumen and introducing liquid medium to the cavity, the liquid medium actuates the seal within the valve and closes an opening between the valve and the guidewire.

A catheter according to any of paragraphs [0008]-[0030], wherein the sealable valve further comprises an exterior wall and a flange disposed radially therein, wherein a gap exists between the exterior wall and the flange.

A catheter according to any of paragraphs [0008]-[0031], wherein the sealable valve comprises a proximal portion and a distal portion, and wherein the flange is disposed toward the proximal portion of the sealable valve.

A catheter according to any of paragraphs [0008]-[0032], wherein the proximal portion of the sealable valve is tubular.

A catheter according to any of paragraphs [0008]-[0033], wherein the distal portion of the sealable valve is tapered radially inward from the exterior wall towards the second guidewire lumen.

A catheter according to any of paragraphs [0008]-[0034], wherein the sealable valve further comprises openings within the exterior wall extending toward the proximal portion.

A catheter according to any of paragraphs [0008]-[0035], wherein the flange is tapered radially inward towards the second guidewire lumen as the flange progresses from the distal portion toward the proximal portion.

The present disclosure also provides a method for treating an obstruction within the vasculature of a subject, the method comprising positioning a catheter within vasculature of a subject, the catheter comprising an outer sheath having a proximal end and a distal end, wherein the distal end comprises a tip, an inner sheath having at least one lumen, a proximal end and a distal end, wherein the inner sheath is disposed radially within the outer sheath, wherein the distal end of the inner sheath is disposed proximate the tip, thereby creating a cavity among the outer sheath, inner sheath and tip, a shield axially disposed within the cavity between the distal end of the inner sheath and the tip, one or more optical fibers disposed within the inner sheath extending from a distal portion of the inner sheath to the distal end of the inner sheath and into the cavity, wherein a proximal end of the one or more optical fibers is coupled to a laser generator, at least one emitter coupled to the one or more optical fibers, wherein the at least one emitter is disposed within the cavity, and one or more liquid medium ports coupled to the at least one lumen, positioning the tip adjacent an obstruction within the vasculature, introducing a liquid medium into the cavity through the one or more liquid medium ports, activating the at least one emitter within the cavity to transmit pulses of light energy into the liquid medium, wherein transmitting the pulses of light energy from the emitter into the liquid medium generates a plurality of propagating pressure waves that cause the tip to engage and disrupt at least a portion of the vascular obstruction.

A method for treating an obstruction within the vasculature of a subject according to any of paragraphs [0008]-[0037], wherein the outer sheath is a hypotube capable of expanding and contracting in the axial direction.

A method for treating an obstruction within the vasculature of a subject according to any of paragraphs [0008]-[0038], wherein the at least one emitter is configured to emit light energy at wavelengths of between about 300 nanometers to about 350 nanometers, at pulse durations between about 100 nanoseconds to about 150 nanoseconds, and at frequencies between about 1 pulse per second to about 250 pulses per second.

A method for treating an obstruction within the vasculature of a subject according to any of paragraphs [0008]-[0039], wherein the liquid medium is any one of iodine-containing contrast medium or gadolinium contrast medium.

The present disclosure provides a catheter system comprising: a sheath having a proximal end and a distal end and a lumen therein; a laser catheter comprising: a proximal end capable of coupling to the laser generator; a distal end; one or more optical fibers extending from a proximal portion of the laser catheter to the distal end of the laser; and at least one emitter coupled to the one or more optical fibers; wherein the catheter is disposed within the sheath, whereupon the distal end of the laser catheter being disposed proximate the distal end of the sheath, a cavity between the distal end of the laser catheter and the distal end of the sheath is created; a means for introducing a liquid medium into the cavity; a handle comprising: a base coupled to the proximal end of the sheath; and a drive mechanism translatably coupled to the base, the drive mechanism coupled to the laser catheter such that translation of the drive mechanism relative to the base causes translation of the laser catheter within the lumen of the sheath.

A catheter system according to any of paragraphs [0008]-[0041], wherein the drive mechanism comprises: a control element movably coupled to the base; and a coupling translatably coupled to the base and driven by the control element, the coupling coupled to the laser catheter such that movement of the control element relative to the base causes translation of the laser catheter within the lumen of the sheath.

A catheter system according to any of paragraphs [0008]-[0042], wherein the control element is rotatably coupled to the base, and rotation of the control element relative to the base causes translation of the laser catheter within the lumen of the sheath.

A catheter system according to any of paragraphs [0008]-[0043], wherein the control element includes a first threaded surface, and the drive mechanism further includes a shaft that is translatable within the base and coupled to the coupling, the shaft including a second threaded surface, and the second threaded surface coupling to the first threaded surface such that rotation of the control element relative to the base causes translation of the shaft within the base and translation of the laser catheter within the lumen of the sheath.

A catheter system according to any of paragraphs [0008]-[0044], wherein the handle further comprises a tube coupled to the base, the tube receiving the laser catheter, and wherein the shaft includes an inner lumen that translatably receives the tube as the shaft translates within the base.

A catheter system according to any of paragraphs [0008]-[0045], wherein the drive mechanism further comprises a seal coupled to the shaft, the seal translatably engaging the tube.

A catheter system according to any of paragraphs [0008]-[0046], wherein the tube is a hypotube.

A catheter system according to any of paragraphs [0008]-[0047], wherein the base includes a first key feature, the shaft includes a second key feature that couples to the first key feature to inhibit rotation of the shaft relative to the base.

A catheter system according to any of paragraphs [0008]-[0048], wherein the base includes an opening disposed within the control element, the second threaded surface extending through the opening to couple to the first threaded surface.

The present disclosure provides a handle for coupling to a sheath and a laser catheter, the handle comprising: a base configured to couple to a proximal end of the sheath; and a drive mechanism translatably coupled to the base, the drive mechanism configured to couple to the laser catheter such that translation of the drive mechanism relative to the base causes translation of the laser catheter within a lumen of the sheath.

A handle according to any of paragraphs [0008]-[0050], wherein the drive mechanism comprises: a control element movably coupled to the base; and a coupling translatably coupled to the base and driven by the control element, the coupling being configured to couple to the laser catheter such that movement of the control element relative to the base causes translation of the laser catheter within the lumen of the sheath.

A handle according to any of paragraphs [0008]-[0051], wherein the control element is rotatably coupled to the base, and rotation of the control element relative to the base causes translation of the laser catheter within the lumen of the sheath.

A handle according to any of paragraphs [0008]-[0052], wherein the control element includes a first threaded surface, and the drive mechanism further includes a shaft that is translatable within the base and coupled to the coupling, the shaft including a second threaded surface, and the second threaded surface coupling to the first threaded surface such that rotation of the control element relative to the base causes translation of the shaft within the base and translation of the laser catheter within the lumen of the sheath.

A handle according to any of paragraphs [0008]-[0053], wherein the handle further comprises a tube coupled to the base, the tube receiving the laser catheter, and wherein the shaft includes an passageway that translatably receives the tube as the shaft translates within the base.

A handle according to any of paragraphs [0008]-[0054], wherein the drive mechanism further comprises a seal coupled to the shaft, the seal translatably engaging the tube.

A handle according to any of paragraphs [0008]-[0055], wherein the tube is a hypotube.

A handle according to any of paragraphs [0008]-[0056], wherein the base includes a first key feature, the shaft includes a second key feature that couples to the first key feature to inhibit rotation of the shaft relative to the base.

A handle according to any of paragraphs [0008]-[0057], wherein the base includes an opening disposed within the control element, the second threaded surface extending through the opening to couple to the first threaded surface.

The present disclosure provides a catheter comprising: an outer sheath having a proximal end, a distal end and a porous attenuating member disposed adjacent the distal end, wherein the porous attenuating member comprises a plurality of openings; an inner sheath having a proximal end and a distal end, wherein the inner sheath is disposed radially within the outer sheath; one or more optical fibers disposed within the inner sheath extending from a proximal portion of the inner sheath to the distal end of the inner sheath, wherein a proximal end of the one or more optical fibers is coupled to a laser generator; and at least one emitter coupled to the one or more optical fibers, wherein the at least one emitter is disposed radially within the outer sheath such that the at least one emitter is disposed radially within the porous attenuating member.

A catheter according to any of paragraphs [0008]-[0059], wherein the porous attenuating member is constructed to form a semi-rigid biocompatible structure.

A catheter according to any of paragraphs [0008]-[0060], wherein the porous attenuating member is constructed to form a rigid biocompatible structure.

A catheter according to any of paragraphs [0008]-[0061], wherein the outer sheath comprises a non-porous biocompatible layer.

A catheter according to any of paragraphs [0008]-[0062], wherein the porous attenuating member is integrally disposed within the solid biocompatible layer.

A catheter according to any of paragraphs [0008]-[0063], wherein the porous attenuating member is disposed on the exterior of the solid biocompatible layer.

A catheter according to any of paragraphs [0008]-[0064], wherein the porous attenuating member is disposed on the interior of the solid biocompatible layer.

A catheter according to any of paragraphs [0008]-[0065], wherein the plurality of openings comprise at least one of the following shapes: circle; oval; triangle; square; rectangle; polygon; diamond; pentagon; hexagon; heptagon; octagon; nonagon; and decagon.

A catheter according to any of paragraphs [0008]-[0066], wherein the at least one emitter is configured to emit laser light energy at wavelengths of between about 300 nanometers to about 350 nanometers, at pulse durations between about 100 nanoseconds to about 150 nanoseconds, and at frequencies between about 1 pulse per second to about 250 pulses per second.

A catheter according to any of paragraphs [0008]-[0067], wherein the at least one emitter is configured to emit laser light energy at wavelengths of about 308 nanometers, at pulse durations between about 120 nanoseconds and about 140 nanoseconds, and at frequencies between about 25 pulses per second to about 80 pulses per second.

A catheter according to any of paragraphs [0008]-[0068], wherein total energy output for the at least one emitter is between about 1 to about 100 millijoules.

A catheter according to any of paragraphs [0008]-[0069], wherein the liquid medium is contrast medium or contrast solution.

A catheter according to any of paragraphs [0008]-[0070], wherein the liquid medium is any one of iodine-containing contrast medium or gadolinium contrast medium.

A catheter according to any of paragraphs [0008]-[0071], wherein the liquid medium is configured to exhibit high absorption of light energy emitted from the at least one emitter at wavelengths of between about 1 nanometer to about 1 millimeter, at pulse durations between about 1 nanosecond to about 1 second, and at frequencies between about 1 pulse per second to about 500 pulses per second.

A catheter according to any of paragraphs [0008]-[0072], wherein the at least one emitter is two or more concentric emitters.

A catheter according to any of paragraphs [0008]-[0073], wherein the at least one emitter is two or more single-fiber emitters.

A catheter according to any of paragraphs [0008]-[0074], wherein the at least one emitter is configured to translate within the outer sheath.

A catheter according to any of paragraphs [0008]-[0075], wherein the distal end of the outer sheath comprises an open configuration.

A catheter according to any of paragraphs [0008]-[0076], wherein the distal end of the outer sheath comprises a closed configuration.

The present disclosure provides a method for treating an obstruction within vasculature of a subject, the method comprising: introducing a catheter within vasculature of a subject, the catheter comprising: an outer sheath having a proximal end, a distal end and a porous attenuating member disposed adjacent the distal end, wherein the porous attenuating member comprises a plurality of openings; an inner sheath having a proximal end and a distal end, wherein the inner sheath is disposed radially within the outer sheath; one or more optical fibers disposed within the inner sheath extending from a proximal portion of the inner sheath to the distal end of the inner sheath, wherein a proximal end of the one or more optical fibers is coupled to a laser generator; and at least one emitter coupled to the one or more optical fibers, wherein the at least one emitter is disposed radially within the outer sheath; one or more liquid medium ports coupled to the at least one lumen; positioning the outer sheath at a location such that the porous attenuating member is adjacent an obstruction within the vasculature; positioning the at least one emitter the such that the at least one emitter is disposed radially within the porous attenuating member; introducing a liquid medium into the outer sheath; activating the at least one emitter to transmit pulses of light energy into the liquid medium; wherein transmitting the pulses of light energy from the emitter into the liquid medium generates a plurality of propagating pressure waves that pass through the outer sheath and the openings in the porous attenuating member and disrupt at least a portion of the obstruction.

The present disclosure provides a kit comprising: an outer sheath having a proximal end, a distal end and a porous attenuating member disposed adjacent the distal end, wherein the porous attenuating member comprises a plurality of openings; and a laser catheter configured to be disposed within the porous attenuating member, the laser catheter comprising: an inner sheath having a proximal end and a distal end, wherein the inner sheath is configured to be disposed radially within the outer sheath; one or more optical fibers disposed within the inner sheath extending from a proximal portion of the inner sheath to the distal end of the inner sheath, wherein a proximal end of the one or more optical fibers is configured to be coupled to a laser generator; and at least one emitter coupled to the one or more optical fibers, wherein the at least one emitter is configured to be disposed radially within the outer sheath such that the at least one emitter is disposed radially within the porous attenuating member.

The present disclosure provides a method for treating an obstruction within vasculature of a subject, the method comprising: introducing an outer sheath within vasculature of a subject, wherein the outer sheath comprises a proximal end, a distal end and a porous attenuating member disposed adjacent the distal end, wherein the porous attenuating member comprises a plurality of openings; introducing a laser catheter within the vasculature of the subject, wherein the laser catheter is configured to be disposed within the porous attenuating member, wherein the laser catheter comprises an inner sheath having a proximal end and a distal end, wherein the inner sheath is configured to be disposed radially within the outer sheath; one or more optical fibers disposed within the inner sheath extending from a proximal portion of the inner sheath to the distal end of the inner sheath, wherein a proximal end of the one or more optical fibers is configured to be coupled to a laser generator; and at least one emitter coupled to the one or more optical fibers, wherein the at least one emitter is disposed radially within the outer sheath; one or more liquid medium ports coupled to the at least one lumen; positioning the outer sheath at a location such that the porous attenuating member is adjacent a portion of an obstruction within the vasculature; positioning the at least one emitter the such that the at least one emitter is disposed radially within the porous attenuating member; introducing a liquid medium into the outer sheath; activating the at least one emitter to transmit pulses of light energy into the liquid medium; wherein transmitting the pulses of light energy from the emitter into the liquid medium generates a plurality of propagating pressure waves that pass through the outer sheath and the openings in the porous attenuating member and disrupt at least a portion of the obstruction.

A method according to any of paragraphs [0008]-[0080], further comprising the step of re-positioning the outer sheath such that the porous attenuating member is adjacent another portion of the obstruction.

A method according to any of paragraphs [0008]-[0081], further comprising the step of re-positioning the laser catheter within outer sheath.

A method according to any of paragraphs [0008]-[0082], wherein the within the laser catheter is re-positioned within the porous attenuating member.

A method according to any of paragraphs [0008]-[0083], further comprising the step of re-positioning the laser catheter within outer sheath.

A method according to any of paragraphs [0008]-[0084], wherein the within the laser catheter is re-positioned within the porous attenuating member.

A method according to any of paragraphs [0008]-[0085], further comprising the steps of removing the laser catheter from the vasculature and removing the outer sheath from the vasculature.

A method according to any of paragraphs [0008]-[0086], further comprising the step of inserting a drug-coated balloon into the vasculature such that the drug-coated balloon is disposed adjacent a remaining portion of the occlusion.

A method according to any of paragraphs [0008]-[0087], further comprising the step of inflating the drug-coated balloon and applying a drug disposed on the drug-coated balloon to the remaining portion of the occlusion.

The present disclosure provides a catheter comprising: a sheath having a guidewire lumen, a proximal end, and a distal end; a plurality of optical fibers circumferentially arranged around or adjacent to the guidewire lumen, wherein at least a portion of the plurality of optical fibers comprise a distal end, wherein each distal end comprises an emitter to emit laser light; and means for directing laser light emitted from the emitter towards the guidewire lumen or a guidewire within the guidewire lumen.

A catheter according to any of paragraphs [0008]-[0089], wherein the means for directing laser light emitted from the emitter towards the guidewire lumen or a guidewire within the guidewire lumen comprises an outer band coupled to the distal end of the sheath, wherein the outer band comprises a distal end, and the emitter is disposed proximate the distal end of the outer band.

A catheter according to any of paragraphs [0008]-[0090], wherein the emitter is directed at the guidewire lumen or a guidewire within the guidewire lumen.

A catheter according to any of paragraphs [0008]-[0091], wherein the means for directing laser light emitted from the emitter towards the guidewire lumen or a guidewire comprises a cap coupled to the distal end of the sheath.

A catheter according to any of paragraphs [0008]-[0092], wherein the cap comprises an interior side and an exterior side, wherein the interior side is tapered to direct laser light emitted from the emitter towards the guidewire lumen or a guidewire within the guidewire lumen.

A catheter according to any of paragraphs [0008]-[0093], wherein the emitter is disposed proximate the interior side of the cap.

A catheter according to any of paragraphs [0008]-[0087], wherein the sheath is an inner sheath, and further comprising an outer sheath translatably receiving the inner sheath.

A catheter according to any of paragraphs [0008]-[0095], wherein the outer sheath comprises a sleeve, and wherein the means for directing laser light emitted from the emitter towards the guidewire lumen or a guidewire within the guidewire lumen comprises an attenuating member of the outer sheath coupled to the sleeve.

A catheter according to any of paragraphs [0008]-[0096], wherein the attenuating member comprises an inner surface, an outer surface, and a plurality of openings extending from the inner surface to the outer surface.

A catheter according to any of paragraphs [0008]-[0097], wherein the plurality of openings comprise at least one of the following shapes: circle; oval; triangle; square; rectangle; polygon; diamond; pentagon; hexagon; heptagon; octagon; nonagon; and decagon.

The present disclosure provides a method for treating an obstruction within vasculature of a subject, the method comprising: positioning a catheter within the vasculature of the subject, the catheter comprising: a sheath having a guidewire lumen, a proximal end, and a distal end; a plurality of optical fibers circumferentially arranged around or adjacent to the guidewire lumen, wherein at least a portion of the plurality of optical fibers comprise a distal end, wherein each distal end comprises an emitter to emit laser light; means for directing laser light emitted from the emitter towards the guidewire lumen or a guidewire within the guidewire lumen; positioning the distal end of the sheath adjacent the obstruction within the vasculature; delivering a liquid medium to the distal end of the sheath; activating the emitter to transmit pulses of light energy through the liquid medium; wherein transmitting the pulses of light energy from the emitter into the liquid medium generates a plurality of propagating pressure waves that disrupt at least a portion of the obstruction; and wherein the means for directing laser light emitted from the emitter towards the guidewire lumen or a guidewire within the guidewire lumen induces vibrations within the guidewire.

A method according to any of paragraphs [0008]-[0099], wherein the means for directing laser light emitted from the emitter towards the guidewire lumen or a guidewire within the guidewire lumen comprises an outer band coupled to the distal end of the sheath, wherein the outer band comprises a distal end, and the emitter is disposed proximate the distal end of the outer band.

A method according to any of paragraphs [0008]-[0100], wherein the means for directing laser light emitted from the emitter towards the guidewire lumen or a guidewire within the guidewire lumen comprises an outer band coupled to the distal end of the sheath, wherein the outer band comprises a distal end, and the emitter is disposed proximate the distal end of the outer band.

A method according to any of paragraphs [0008]-[0101], wherein the emitter is directed at the guidewire lumen or a guidewire in the guidewire lumen.

A method according to any of paragraphs [0008]-[0102], wherein the means for directing laser light emitted from the emitter towards the guidewire lumen or a guidewire comprises a cap coupled to the distal end of the sheath.

A method according to any of paragraphs [0008]-[0103], wherein the cap comprises an interior side and an exterior side, wherein the interior side is tapered to direct laser light emitted from the emitter towards the guidewire lumen or a guidewire within the guidewire lumen.

A method according to any of paragraphs [0008]-[0104], wherein the emitter is disposed proximate the interior side of the cap.

A method according to any of paragraphs [0008]-[0105], wherein the sheath is an inner sheath, and wherein the catheter further comprises an outer sheath translatably receiving the inner sheath.

A method according to any of paragraphs [0008]-[0106], wherein the outer sheath comprises a sleeve, and wherein the means for directing laser light emitted from the emitter towards the guidewire lumen or a guidewire within the guidewire lumen comprises an attenuating member of the outer sheath coupled to the sleeve.

A method according to any of paragraphs [0008]-[0107], wherein the attenuating member comprises an inner surface, an outer surface, and a plurality of openings extending from the inner surface to the outer surface, and wherein transmitting the pulses of light energy from the emitter into the liquid medium generates a plurality of propagating pressure waves that pass through the plurality of openings.

A method according to any of paragraphs [0008]-[0108], wherein the plurality of openings comprise at least one of the following shapes: circle; oval; triangle; square; rectangle; polygon; diamond; pentagon; hexagon; heptagon; octagon; nonagon; and decagon.

The present disclosure provides a method for treating an obstruction within vasculature of a subject, the method comprising: positioning a catheter within the vasculature of the subject, the catheter comprising: a sheath having a guidewire lumen, a proximal end, and a distal end; a plurality of optical fibers circumferentially arranged around or adjacent to the guidewire lumen, wherein at least a portion of the plurality of optical fibers comprise a distal end, wherein each distal end comprises an emitter to emit laser light; positioning the distal end of the sheath adjacent the obstruction within the vasculature; delivering a liquid medium to the distal end of the sheath; activating the emitter to transmit pulses of light energy through the liquid medium; wherein transmitting the pulses of light energy from the emitter into the liquid medium generates a plurality of propagating pressure waves that disrupt at least a portion of the obstruction; and wherein the plurality of propagating pressure waves induce vibrations within the guidewire.

The present disclosure provides a method for treating an obstruction within vasculature of a subject, the method comprising: positioning a catheter within vasculature of a subject, the catheter comprising: an outer sheath having a proximal end and a distal end; a tip coupled to the distal end of the outer sheath; an inner sheath having a first guidewire lumen, a proximal end, and a distal end, wherein the inner sheath is disposed radially within the outer sheath, wherein the distal end of the inner sheath is disposed proximate the tip, thereby creating a cavity among the outer sheath, inner sheath and tip; a sealable valve coupled to the outer sheath, the sealable valve having a second guidewire lumen and a seal; one or more optical fibers disposed within the inner sheath extending from the proximal end of the inner sheath to the distal end of the inner sheath and into the cavity, wherein a proximal end of the one or more optical fibers is coupled to a laser generator; at least one emitter coupled to the one or more optical fibers, wherein the at least one emitter is disposed within the cavity; positioning the tip adjacent an obstruction within the vasculature by advancing the catheter along a guidewire, wherein the guidewire is received in the first guidewire lumen and the second guidewire lumen; introducing a liquid medium into the cavity, wherein the liquid medium actuates the seal within the valve and closes an opening between the valve and the guidewire; and; activating the at least one emitter within the cavity to transmit pulses of light energy into the liquid medium; wherein transmitting the pulses of light energy from the emitter into the liquid medium generates a plurality of propagating shock waves that cause the tip to engage and disrupt at least a portion of the vascular obstruction.

A method according to any of paragraphs [0008]-[0111], wherein the sealable valve further comprises an exterior wall and a flange disposed radially therein, wherein a gap exists between the exterior wall and the flange, and wherein introducing the liquid medium into the cavity includes introducing the liquid medium to the gap to actuate the seal within the valve and close the opening between the valve and the guidewire.

A method according to any of paragraphs [0008]-[0112], wherein the sealable valve further comprises openings within the exterior wall extending into the gap, and wherein introducing the liquid medium to the gap includes introducing the liquid medium to the gap via the openings.

A method according to any of paragraphs [0008]-[0113], wherein the flange is tapered radially inward towards the second guidewire lumen as the flange progresses from a distal portion of the valve toward a proximal portion of the valve.

The present disclosure provides a catheter comprising a sheath having a proximal end and a distal end; a tip coupled to and forming a cavity at the distal end of the sheath; one or more optical fibers carried by the sheath and extending from the proximal end toward the distal end and into the cavity, wherein a proximal end of the one or more optical fibers is configured to be coupled to a laser generator; at least one emitter coupled to the one or more optical fibers, wherein the at least one emitter is disposed within the cavity; and a light absorbing material coupled to at least one of the sheath and the tip and disposed such that light emitted from the at least one emitter intersects with the light absorbing material.

A catheter according to any of paragraphs [0008]-[0115], wherein the light absorbing material is disposed within the cavity.

A catheter according to any of paragraphs [0008]-[0116], wherein the light absorbing material is disposed outside of the cavity.

A catheter according to any of paragraphs [0008]-[0117], wherein the light absorbing material is applied as a coating to a support structure located within the cavity.

The present disclosure provides a method for treating an obstruction within vasculature of a subject, the method comprising positioning a catheter within vasculature of a subject, the catheter comprising a sheath having a proximal end and a distal end; a tip coupled to and forming a cavity at the distal end of the sheath; one or more optical fibers carried by the sheath and extending from the proximal end toward the distal end and into the cavity, wherein a proximal end of the one or more optical fibers is coupled to a laser generator; at least one emitter coupled to the one or more optical fibers, wherein the at least one emitter is disposed within the cavity; a light absorbing material coupled to at least one of the sheath and the tip; positioning the tip adjacent an obstruction within the vasculature by advancing the catheter within the vasculature; and activating the at least one emitter to transmit a pulse of light energy such that the light energy intersects with at least a portion of the light absorbing material.

A method according to any of paragraphs [0008]-[0119], further comprising delivering a liquid medium to the cavity.

A method according to any of paragraphs [0008]-[0120], wherein the tip further comprises a flexible membrane carrying the light absorbing material, and activating the at least one emitter to transmit the pulse of light energy such that the light energy intersects with the at least a portion of the light absorbing material causes deflection of the flexible membrane.

The present disclosure provides a method for treating an obstruction within vasculature of a subject, the method comprising positioning a catheter within the vasculature of the subject, the catheter comprising a sheath having a proximal end and a distal end; a plurality of optical fibers carried by the sheath, wherein at least a portion of the plurality of optical fibers comprise a distal end, wherein each distal end comprises an emitter to emit laser light; positioning the distal end of the sheath adjacent the obstruction within the vasculature; delivering a gas-saturated liquid medium to the distal end of the sheath; activating the emitter to transmit pulses of light energy through the gas-saturated liquid medium; wherein transmitting the pulses of light energy from the emitter into the gas-saturated liquid medium generates a plurality of propagating pressure waves that disrupt at least a portion of the obstruction.

A method according to any of paragraphs [0008]-[0122], wherein the gas-saturated liquid medium is any one of iodine-containing contrast medium or gadolinium contrast medium.

A method according to any of paragraphs [0008]-[0123], wherein the gas-saturated liquid medium comprises a super saturated liquid medium.

The present disclosure provides a method for treating an obstruction within vasculature of a subject, the method comprising: positioning a catheter within the vasculature of the subject, the catheter comprising: an outer sheath having a proximal end, a distal end, and a lumen therein; an inner sheath comprising: a proximal end capable of coupling to a laser generator; a distal end opposite the proximal end; one or more optical fibers extending from the proximal end of the inner sheath to the distal end of the inner sheath; and at least one emitter coupled to the one or more optical fibers; positioning the distal end of the outer sheath adjacent the obstruction within the vasculature; positioning the distal end of the inner sheath proximally relative to the distal end of the outer sheath to create a cavity between the distal end of the inner sheath and the distal end of the outer sheath; delivering a liquid medium to the cavity; and activating the emitter to transmit pulses of light energy through the liquid medium; wherein transmitting the pulses of light energy from the emitter into the liquid medium generates a plurality of propagating pressure waves that disrupt at least a portion of the obstruction.

A method according to any of paragraphs [0008]-[0125], further comprising: repositioning the inner sheath within outer sheath after activating the emitter; and subsequently activating the emitter to transmit pulses of light energy through the liquid medium; wherein transmitting the pulses of light energy from the emitter into the liquid medium generates another plurality of propagating pressure waves that disrupt at least a portion of the obstruction.

A method according to any of paragraphs [0008]-[0126], further comprising traversing the entire occlusion with the inner sheath and the outer sheath after activating the emitter.

A method according to any of paragraphs [0008]-[0127], wherein the inner sheath further comprises a guidewire lumen, wherein positioning the catheter within the vasculature of the subject comprises receiving a guidewire in the guidewire lumen and advancing the inner sheath and the outer sheath along the guidewire, and further comprising traversing the occlusion with the guidewire.

A method according to any of paragraphs [0008]-[0128], further comprising traversing the occlusion with the inner sheath and without the outer sheath after activating the emitter.

A method according to any of paragraphs [0008]-[0129], further comprising: inserting a drug-coated balloon into the vasculature, after activating the emitter, such that the drug-coated balloon is disposed adjacent a remaining portion of the occlusion; and inflating the drug-coated balloon and applying a drug disposed on the drug-coated balloon to the remaining portion of the occlusion.

According to the present disclosure, after penetrating the calcified and/or fibrous total occlusion and destroying at least a portion of the occlusion with the laser-induced shock wave emitting catheter sheath, the method may also include delivering via a balloon catheter, one or more therapeutic agents, which is/are disposed on the balloon, wherein the therapeutic agents may comprise one or more oxidation-insensitive drugs in a polymer-free drug preparation, including one or more of taxanes, thalidomide, statins, corticoids, and lipophilic derivatives of corticoids. The therapeutic agents may also include one or more lipophilic antioxidants, such as nordihydroguaiaretic acid, resveratrol and propyl gallate in a polymer-free preparation. For example, U.S. Application Ser. No. 13/628,608, which is a continuation of International Application No. PCT/EP2010/066754, filed November 3, 2010, discloses a scoring or cutting balloon catheter providing improved adherence of therapeutic agents to the balloon catheter using a combination of an oxidation-insensitive drug and a lipophilic antioxidant.

Additionally, U.S. Application Ser. No. 13/707,401, filed December 6, 2012, and issued on October 21, 2014, which is a divisional application of U.S. Application Ser. No. 11/411,635, filed April 26, 2006, and which claims priority to U.S. Provisional Application Ser. No. 60/680,450, filed May 11, 2005, discloses scoring elements of a balloon catheter coated with a polymer matrix to deliver hydrophobic and lipophilic drugs to regions within a thrombus or plaque.

Additionally, U.S. Application Ser. No. 13/310,320, filed December 2, 2011, and issued October 22, 2013, which is a divisional application of U.S. Application Ser. No. 12/712,134, filed February 24, 2010, and issued March 6, 2012, and U.S. Application Ser. No. 12/726,101, filed March 17, 2010, and issued February 14, 2012, which is a continuation-in-part of U.S. Application Ser. No. 12/712,134, filed February 24, 2010, and issued March 6, 2012, which is a continuation-in-part of U.S. Application Ser. No. 12/558,420, filed September 11, 2009, which is a continuation-in-part of U.S. Application Ser. No. 12/210,344, filed September 15, 2008, and issued September 4, 2012, and U.S. Application Ser. No. 14/149,862, filed January 8, 2014, which is a continuation of U.S. Application Ser. No. 13/560,538, filed June 27, 2012, and issued March 18, 2014, which is a divisional application of U.S. Application Ser. No. 12/210,344, filed September 15, 2008, and issued September 4, 2012, disclose methods and devices for local delivery of water-soluble and water-insoluble therapeutic agents to the surface of normal and diseased body lumens.

Additionally, U.S. Application Ser. No. 13/926,515, filed June 25, 2013, which claims priority to U.S. Provisional Application Ser. No. 61/665,758, filed June 28, 2012, disclose methods and devices for coating a medical device that includes a therapeutic agent dispersed in a polymer or oligomer matrix.

The present disclosure also provides a catheter system comprising a sheath having a proximal end and a distal end and a lumen therein, a laser catheter comprising, a proximal end, a distal end capable of coupling to a laser generator, one or more optical fibers extending from a proximal portion of the laser catheter to the distal end of the laser, and at least one emitter coupled to the one or more optical fibers, wherein the catheter is configured to be disposed within the sheath, whereupon the distal end of the laser catheter being disposed proximate the distal end of the sheath, a cavity between the distal end of the laser catheter and the distal end of the sheath is created, and a means for introducing a liquid medium into the cavity.

A catheter system according to any of paragraphs [0008]-[0135], wherein the laser catheter is configured to translate within the sheath.

A catheter system according to any of paragraphs [0008]-[0136], wherein the liquid medium is contrast medium or contrast solution.

As used herein, "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C," and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (for example, X₁ and X₂) as well as a combination of elements selected from two or more classes (for example, Y₁ and Zₒ).

It is to be noted that the term "a" or "an" entity refers to one or more of that entity. As such, the terms "a" (or "an"), "one or more" and "at least one" can be used interchangeably herein. It is also to be noted that the terms "comprising," "including," and "having" can be used interchangeably.

The term "catheter" as used herein generally refers to a tube that can be inserted into a body cavity, duct, lumen, or vessel, such as the vasculature system. In most uses, a catheter is a relatively thin, flexible tube ("soft" catheter), though in some uses, it may be a larger, solid-less flexible-but possibly still flexible-catheter ("hard" catheter).

The term "balloon catheter" as used herein generally refers to the various types of angioplasty catheters which carry a balloon for performing angioplasty. Balloon catheters may also be of a wide variety of inner structure, such as different lumen design, of which there are at least three basic types: triple lumen, dual lumen and co-axial lumen. All varieties of internal structure and design variation are meant to be included by use of the term "balloon catheter" herein.

The terms "coupler" or "fiber optic coupler" refers to the optical fiber device with one or more input fibers and one or several output fibers. Fiber couplers are commonly special optical fiber devices with one or more input fibers for distributing optical signals into two or more output fibers. Optical energy is passively split into multiple output signals (fibers), each containing light with properties identical to the original except for reduced amplitude. Fiber couplers have input and output configurations defined as M x N. M is the number of input ports (one or more). N is the number of output ports and is always equal to or greater than M. Fibers can be thermally tapered and fused so that their cores come into intimate contact. This can also be done with polarization-maintaining fibers, leading to polarization-maintaining couplers (PM couplers) or splitters. Some couplers use side-polished fibers, providing access to the fiber core. Couplers can also be made from bulk optics, for example in the form of micro lenses and beam splitters, which can be coupled to fibers ("fiber pig-tailed").

The term "emitter" as used herein refers to an end portion of a fiber or an optical component that emits light from a distal end of device, such as a catheter, towards a desired target or region, which typically comprises tissue. As described herein, an emitter or emitters can be used to emit light of any wavelength, insofar as the light emitted is coupled with a suitable absorptive liquid such that pressure waves and/or cavitation bubbles are generated and additional pressure waves are produced from the cavitation bubbles. An emitter or emitters can emit light, including but not limited to, laser light, white light, visible light, infrared light, and ultraviolet light.

A "laser emitter" as used herein refers to an end portion of a fiber or an optical component that emits laser light from a distal end of the catheter towards a desired target, which is typically tissue.

The term "laser-induced pressure wave" as used herein is a pressure wave caused by an explosion due to the interaction of laser light and a contrast medium. The laser-induced pressure wave may be produced in air or liquid, such as saline that includes a contrast medium.

The term "means" as used herein shall be given its broadest possible interpretation. Accordingly, a claim incorporating the term "means" shall cover all structures, materials, or acts set forth herein, the structures, materials or acts shall include all those described in the summary, brief description of the drawings, detailed description, abstract, and claims themselves.

The term an "optical fiber" (or laser active fibre) as used herein refers to a flexible, transparent fiber made of an optically transmissive material, such as glass (silica) or plastic, which functions as a waveguide, or "light pipe", to transmit light between the two ends of the fiber.

The term "porous attenuating member" as used herein shall mean a rigid member or semi-rigid member having openings therein. Examples of a rigid member and a semi-rigid member include a member constructed of coils, braids, laser-cut tubing, reinforced polymer extrusions, patterned plastics, metals and ceramics. Specific materials used to construct such rigid member and a semi-rigid member may include nitinol, stainless steel, titanium, silver, aluminum, cobalt, chromium, pebax, silicone, urethane, polyethylene and derivatives, nylons, polytetrafluoroethylene and derivatives, polyethylene terephthalate, polypropylene, poly(ether ether ketone), hydroxyapatite, alumina, tricalcium phosphate, silicates or other biocompatible metals, ceramics or polymers. Possible configurations for the porous attenuating member include, but are not limited to, spiral cuts, interrupted spiral cut, honeycomb, lattice structures as found commonly in vascular stents, slots, offset slots, helices, slits that are either longitudinal, radial, circumferential, or a combination thereof, openings that are shaped cutouts.

The term "pressure wave" as used herein includes both a shock wave and a sound wave, wherein the shock wave is a pressure wave that moves above the velocity of sound, and the sound wave is a pressure wave that moves at or below the speed of sound.

The term "rigid structure" as used herein shall mean a structure that is able to bend or otherwise conform to the shape of the vasculature as it passes therethrough but is substantially unable to expand and/or contract in the radial direction upon a pressure wave passing therethrough.

The term "semi-rigid structure" as used herein shall mean a structure that is partly rigid with an additional degree of flexibility as it passes through the vasculature but is substantially unable to expand and/or contract in the radial direction upon a pressure wave passing therethrough.

The term "shock wave" as used herein shall mean a region of abrupt change of pressure moving as a wave front above the velocity of sound.

The term "sound wave" as used herein is pressure wave of audible or inaudible sound. That is, a sound wave is a pressure wave that moves at or below the speed of sound. An "acoustic wave" may also be referred to as a sound wave.

The term "therapeutic agent" as used herein generally refers to any known or hereafter discovered pharmacologically active agent that provides therapy to a subject through the alleviation of one or more of the subject's physiological symptoms. A therapeutic agent may be a compound that occurs in nature, a chemically modified naturally occurring compound, or a compound that is chemically synthesized. The agent will typically be chosen from the generally recognized classes of pharmacologically active agents, including, but not necessarily limited to, the following: analgesic agents; anesthetic agents; antiarthritic agents; respiratory drugs, including antiasthmatic agents; anticancer agents, including antineoplastic drugs; anticholinergics; anticonvulsants; antidepressants; antidiabetic agents; antidiarrheals; antihelminthics; antihistamines; antihyperlipidemic agents; antihypertensive agents; anti-infective agents such as antibiotics and antiviral agents; antiinflammatory agents; antimigraine preparations; antinauseants; antiparkinsonism drugs; antipruritics; antipsychotics; antipyretics; antispasmodics; antitubercular agents; antiulcer agents; antiviral agents; anxiolytics; appetite suppressants; attention deficit disorder (ADD) and attention deficit hyperactivity disorder (ADHD) drugs; cardiovascular preparations including calcium channel blockers, CNS agents; beta-blockers and antiarrhythmic agents; central nervous system stimulants; cough and cold preparations, including decongestants; diuretics; genetic materials; herbal remedies; hormonolytics; hypnotics; hypoglycemic agents; immunosuppressive agents; leukotriene inhibitors; mitotic inhibitors; restenosis inhibitors; muscle relaxants; narcotic antagonists; nicotine; nutritional agents, such as vitamins, essential amino acids and fatty acids; ophthalmic drugs such as antiglaucoma agents; parasympatholytics; psychostimulants; sedatives; steroids; sympathomimetics; tranquilizers; and vasodilators including general coronary, peripheral and cerebral.

The terms "vasculature" and "vascular" as used herein refer to any part of the circulatory system of a subject, including peripheral and non-peripheral arteries and veins. Vascular material found within the vasculature can be comprised of both biological material (for example, nucleic acids, amino acids, carbohydrates, polysaccharides, lipids and the like) and non-biological material (for example, fat deposits, fibrous tissue, calcium deposits, remnants of dead cells, cellular debris and the like).

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 illustrates an exemplary ablation system, including a laser generator and a laser induced pressure wave emitting catheter sheath;
FIG. 2A is a schematic view of a distal portion of the laser induced pressure wave emitting catheter sheath of FIG. 1, according to one embodiment of the present disclosure;
FIG. 2B is an elevation view of an embodiment of the distal portion of the laser induced pressure wave emitting catheter, according to one embodiment of the present disclosure;
FIG. 2C is a cross-sectional view (through plane C in FIG. 2B) of the distal portion of the laser induced pressure wave emitting catheter;

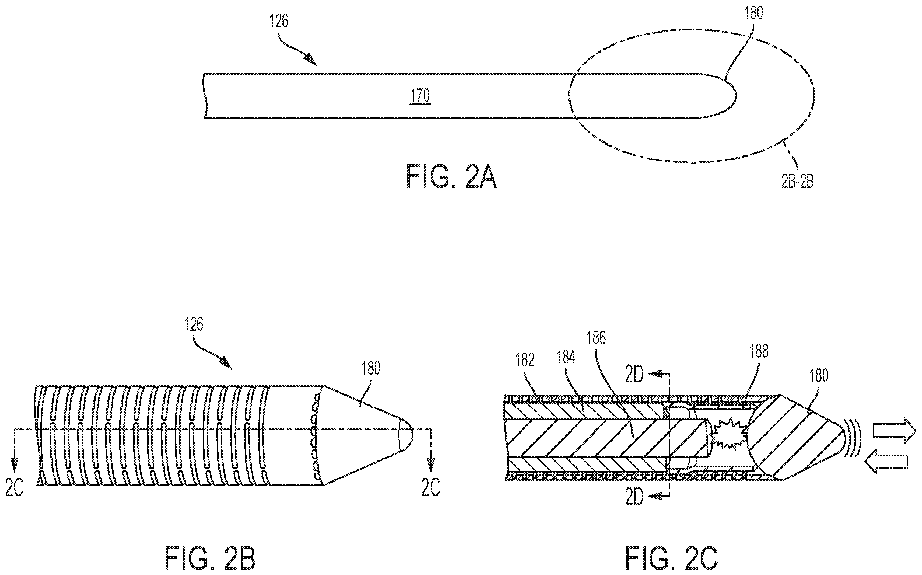

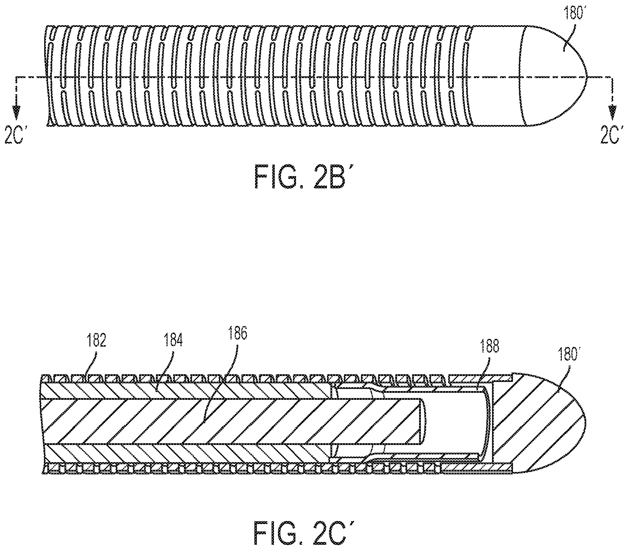

FIG. 2B' is an elevation view of an embodiment of the distal portion of the laser induced pressure wave emitting catheter, according to one embodiment of the present disclosure;
FIG. 2C' is a cross-sectional view (through plane C in FIG. 2B') of the distal portion of the laser induced pressure wave emitting catheter, according to one embodiment of the present disclosure;

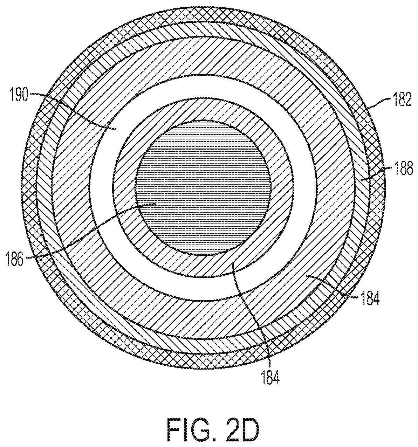

FIG. 2D is a cross-sectional view (through plane D in FIG. 2C) of the distal portion of the laser induced pressure wave emitting catheter, according to one embodiment of the present disclosure;

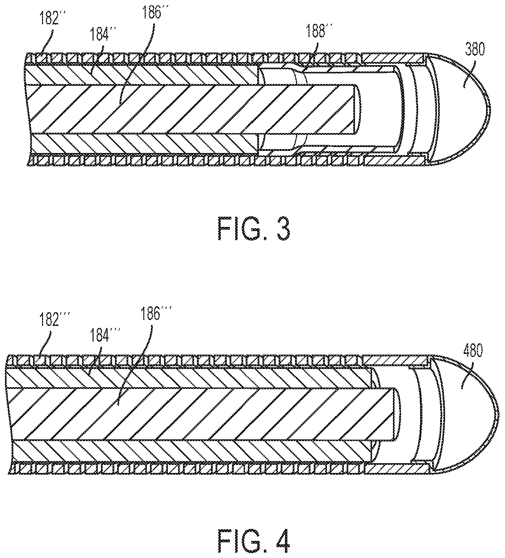

FIG. 3 is a cross-sectional view of the distal portion of the laser induced pressure wave emitting catheter, according to one embodiment of the present disclosure;
FIG. 4 is a cross-sectional view of the distal portion of the laser induced pressure wave emitting catheter, according to one embodiment of the present disclosure;

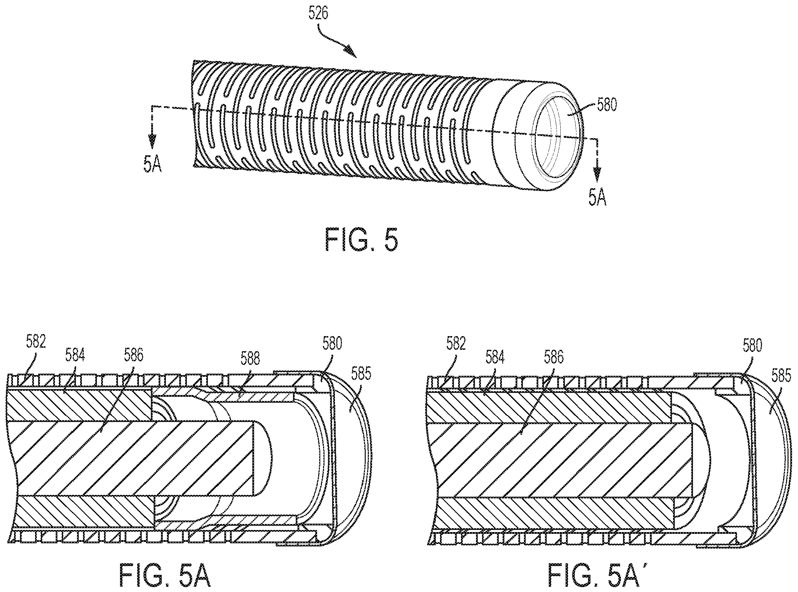

FIG. 5 is a perspective view of an embodiment of the distal portion of the laser induced pressure wave emitting catheter, according to one embodiment of the present disclosure;
FIG. 5A is a cross-sectional view (through plane A in FIG. 5) of the distal portion of the laser induced pressure wave emitting catheter, according to one embodiment of the present disclosure;
FIG. 5A' is a cross-sectional view (through plane A in FIG. 5) of the distal portion of the laser induced pressure wave emitting catheter, according to one embodiment of the present disclosure;

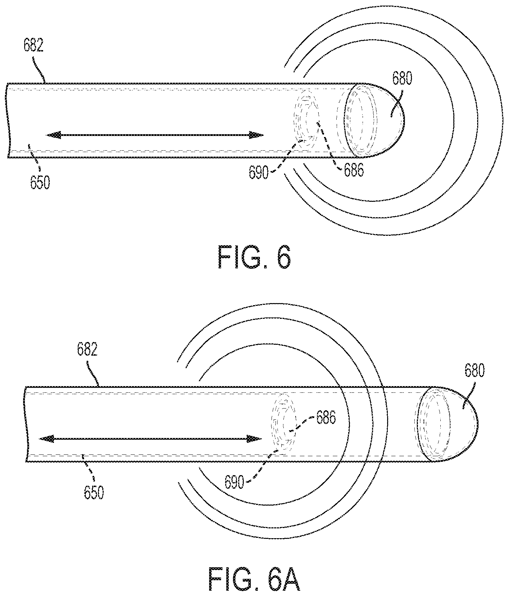

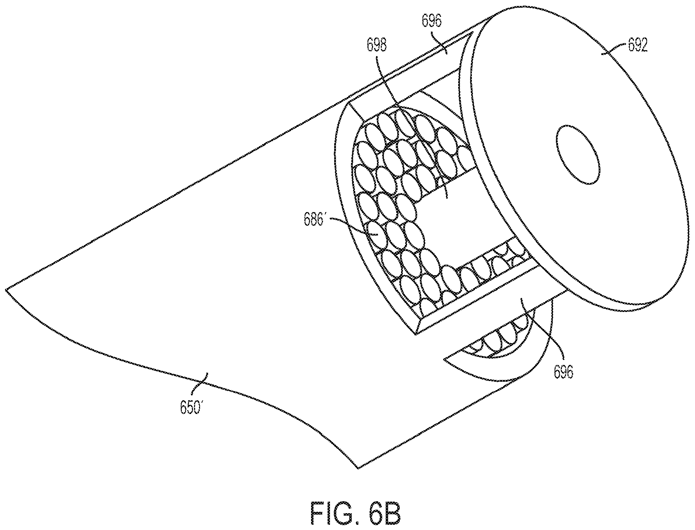

FIG. 6 is a partially transparent elevation view of the distal portion of a laser catheter disposed at one location within the distal portion of a catheter sheath;
FIG. 6A is a partially transparent elevation view of the distal portion of the laser catheter disposed at an alternative location within the distal portion of the catheter sheath, in comparison to the position of the laser catheter in FIG. 6;
FIG. 6B is an enlarged partial perspective view of the distal portion of an alternative laser catheter including a deflector coupled to the distal end of the laser catheter;

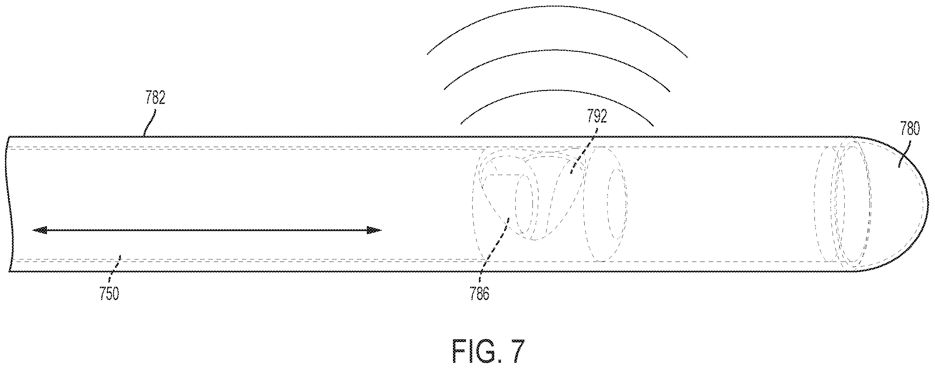

FIG. 7 is a perspective view of a laser catheter including a shield or deflector as the distal end thereof, according to one embodiment of the present disclosure, wherein the laser catheter is disposed at one location within the distal portion of a catheter sheath;

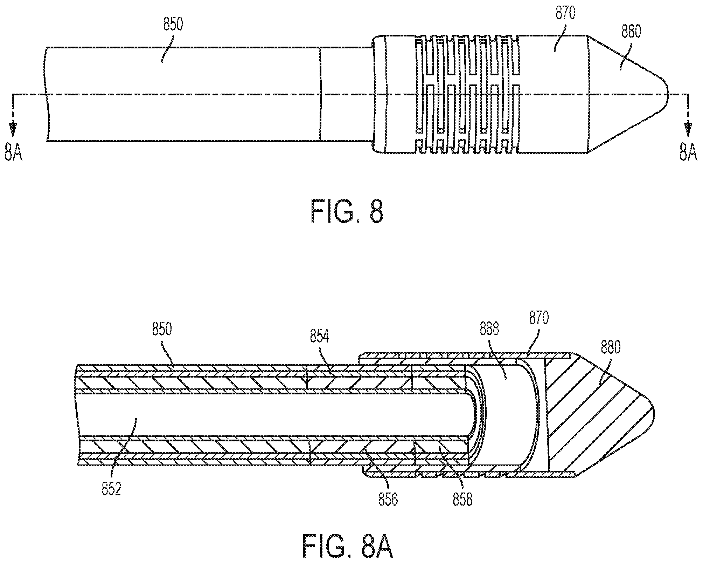

FIG. 8 is an elevation view of a removable tip for an ablation catheter, according to one embodiment of the present disclosure;
FIG. 8A is a cross-sectional view (through plane A in FIG. 8) of a removable tip for an ablation catheter;

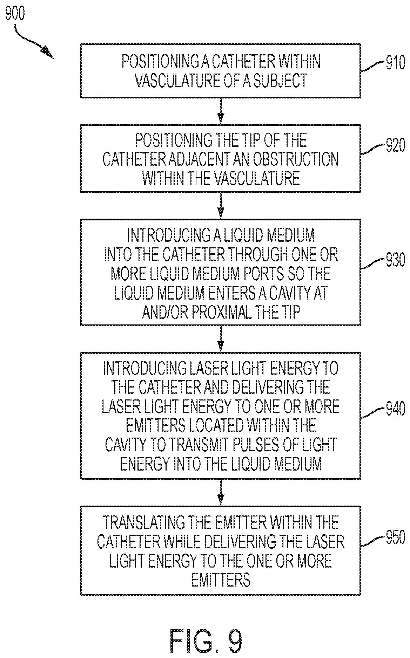

FIG. 9 is a representative flow diagram of a method of treating a subject using a catheter, according to one embodiment of the present disclosure;

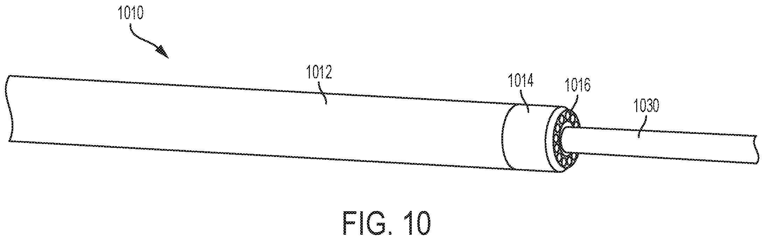

FIG. 10 is a perspective view of a laser catheter and a guidewire, according to one embodiment of the present disclosure;

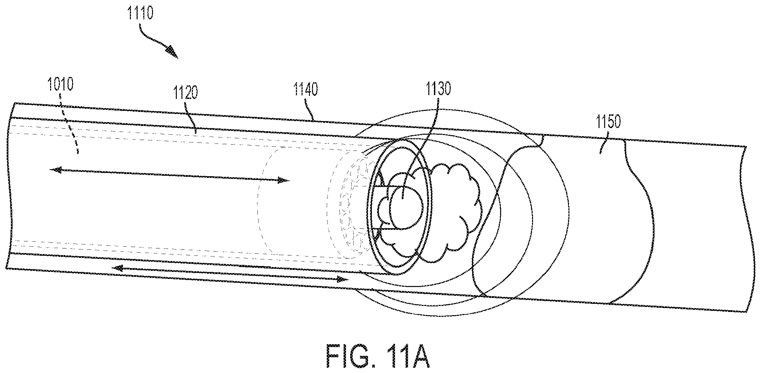

FIG. 11A is a perspective view of a kit within the vasculature of a patient, wherein the kit includes a laser catheter radially disposed within a sheath and over a guidewire, according to one embodiment of the present disclosure, wherein the kit and guidewire are located proximally of an occlusion;

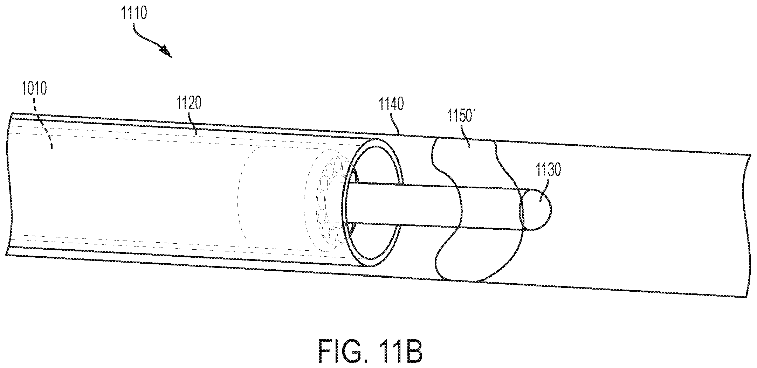

FIG. 11B is a perspective view of a kit within the vasculature of a patient, wherein the kit includes a laser catheter radially disposed within a sheath and over a guidewire, according to one embodiment of the present disclosure, wherein the kit is located proximally of an occlusion, and the guidewire has penetrated the occlusion;

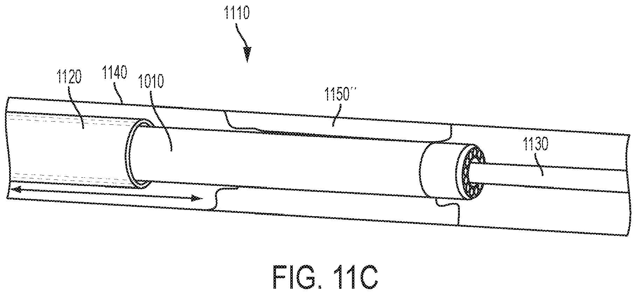

FIG. 11C is a perspective view of a kit within the vasculature of a patient, wherein the kit includes a laser catheter radially disposed within a sheath and over a guidewire, according to one embodiment of the present disclosure, wherein the sheath is located proximally of an occlusion, and the laser catheter and guidewire have penetrated the occlusion;

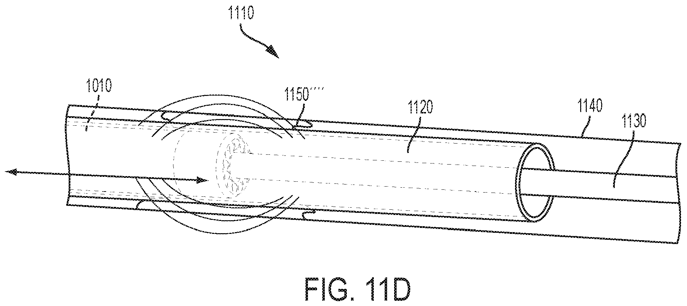

FIG. 11D is a perspective view of a kit within the vasculature of a patient, wherein the kit includes a laser catheter radially disposed within a sheath and over a guidewire, according to one embodiment of the present disclosure, wherein the kit and guidewire have penetrated the occlusion;

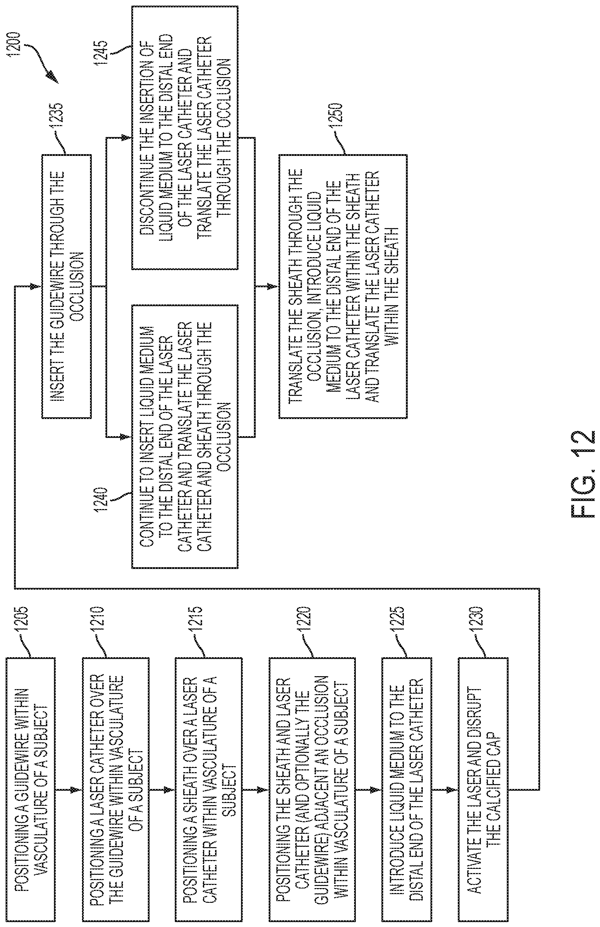

FIG. 12 is a representative flow diagram of a method of treating a subject using a laser catheter and sheath, according to one embodiment of the present disclosure;

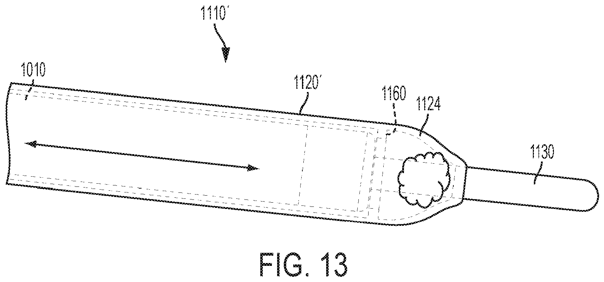

FIG. 13 is an elevation view of a kit that includes a laser catheter radially disposed within a sheath and over a guidewire, according to one embodiment of the present disclosure;

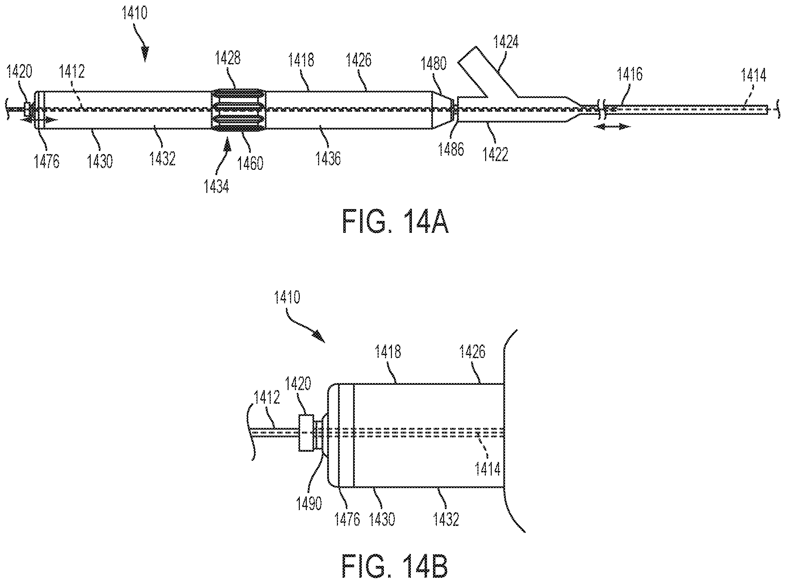

FIG. 14A is an elevation view of a kit that includes a laser catheter radially disposed within a handle and a sheath and over a guidewire, according to one embodiment of the present disclosure;
FIG. 14B is a detail elevation view of the laser catheter and the handle of FIG. 14A at a proximal end of the handle;

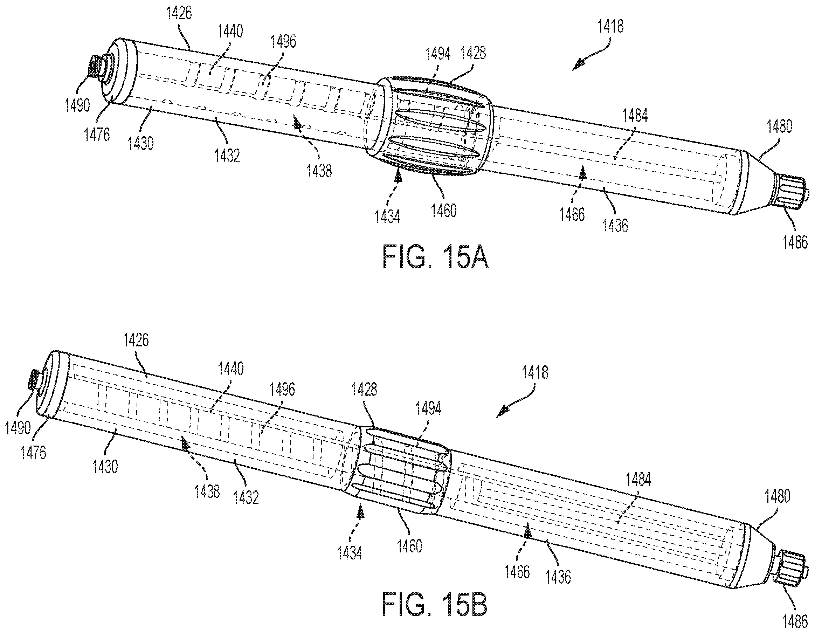

FIG. 15A is a perspective view of the handle of FIG. 14A, wherein several external components are partially transparent to illustrate internal components, and a shaft of the handle is shown in a proximal position;
FIG. 15B is another perspective view of the handle of FIG. 14A, wherein several external components are partially transparent to illustrate internal components, and the shaft is shown in the proximal position;

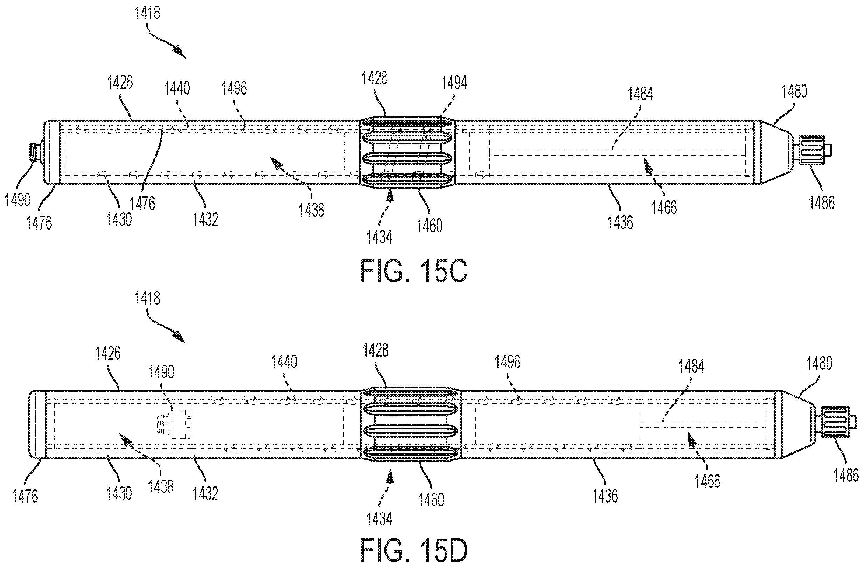

FIG. 15C is an elevation view of the handle of FIG. 14A, wherein several external components are partially transparent to illustrate internal components, and the shaft is shown in the proximal position;
FIG. 15D is an elevation view of the handle of FIG. 14A, wherein several external components are partially transparent to illustrate internal components, and the shaft is shown in an intermediate position;

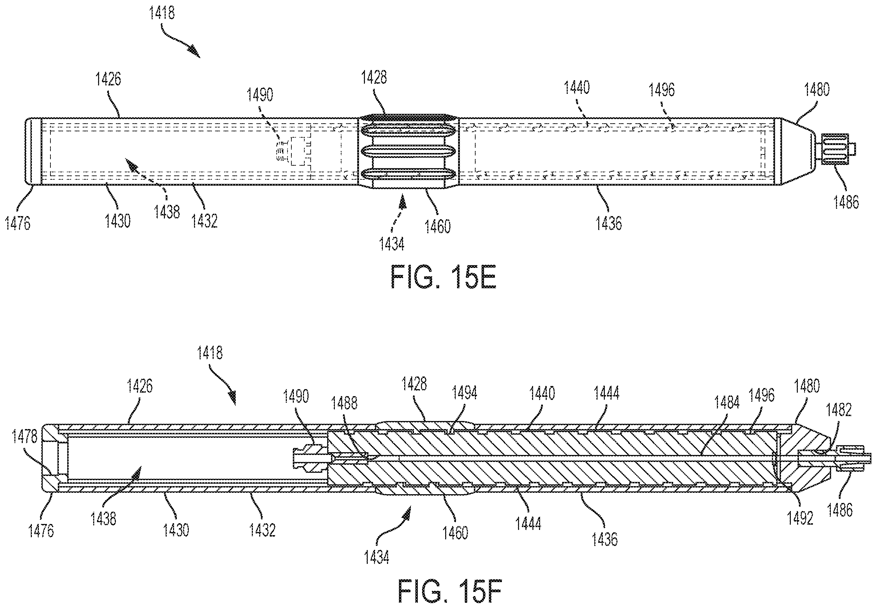

FIG. 15E is an elevation view of the handle of FIG. 14A, wherein several external components are partially transparent to illustrate internal components, and the shaft is shown in a distal position;
FIG. 15F is a cross-sectional view of the handle of FIG. 14A, wherein the shaft is shown in the proximal position;

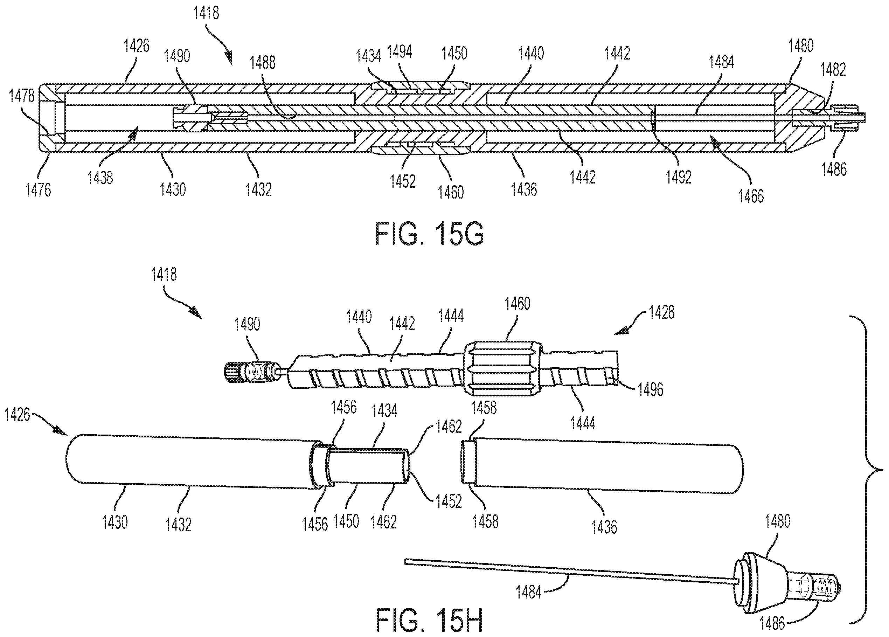

FIG. 15G is a cross-sectional view of the handle of FIG. 14A, wherein the shaft is shown in an intermediate position;
FIG. 15H is an exploded view of the handle of FIG. 14A;

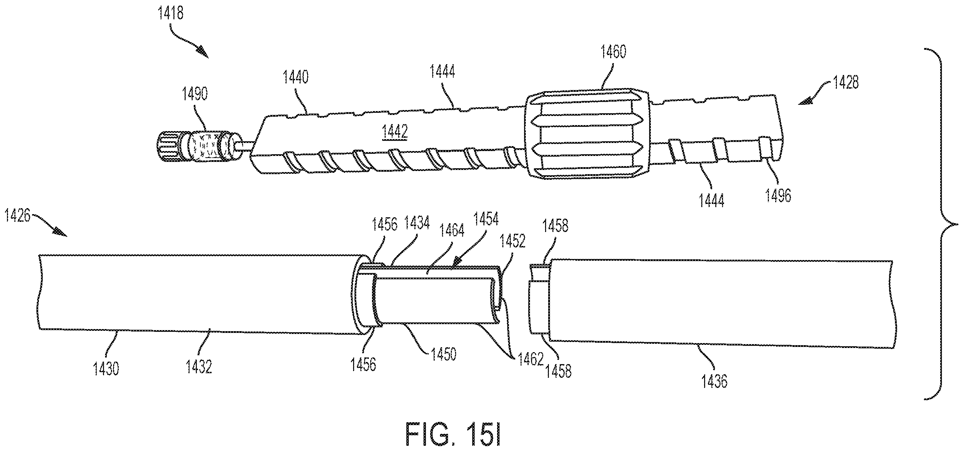

FIG. 15I is a detail exploded view of the handle of FIG. 14A;

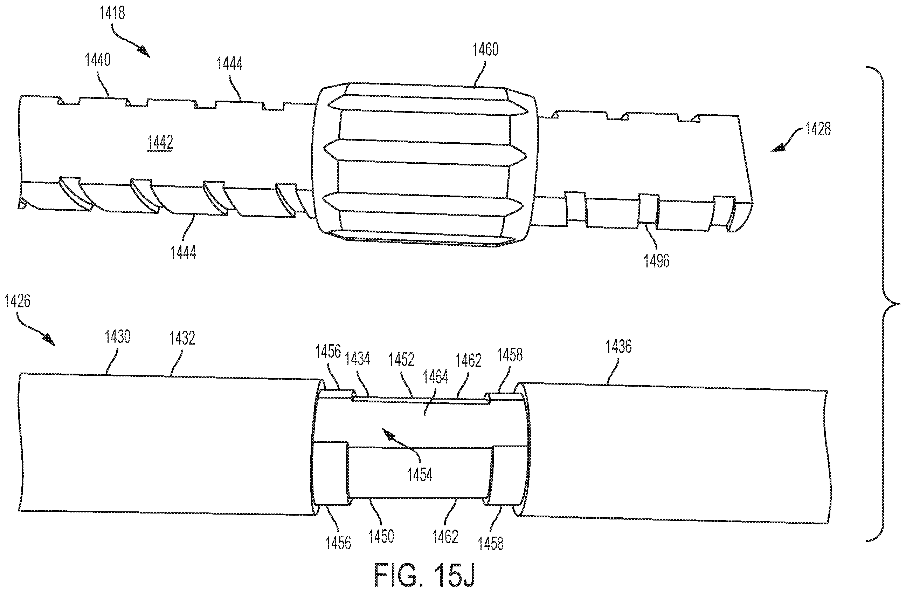

FIG. 15J is another detail exploded view of the handle of FIG. 14A;

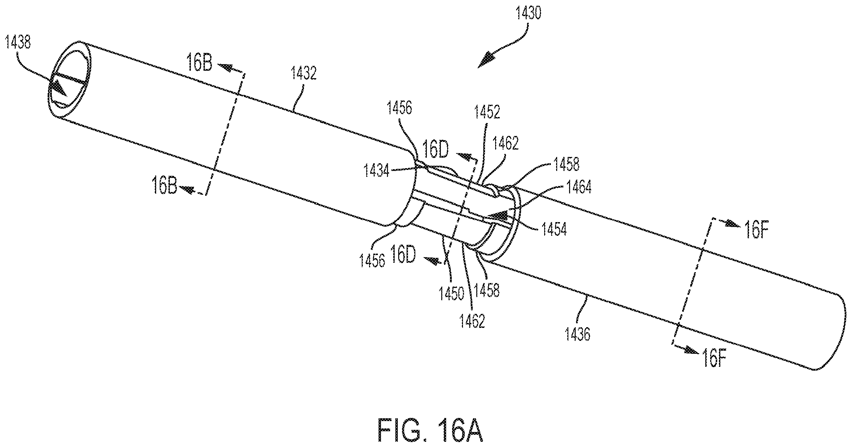

FIG. 16A is a perspective view of a frame of the handle of FIG. 14A;

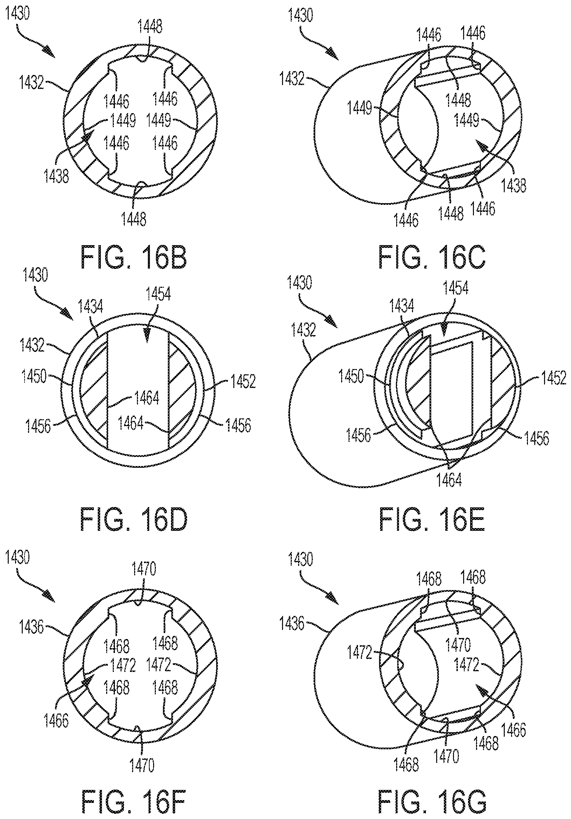

FIG. 16B is an elevation cross-sectional view of the frame along line 16B-16B of FIG. 16A;
FIG. 16C is a perspective cross-sectional view of the frame along line 16B-16B of FIG. 16A;
FIG. 16D is an elevation cross-sectional view of the frame along line 16D-16D of FIG. 16A;
FIG. 16E is a perspective cross-sectional view of the frame along line 16D-16D of FIG. 16A;
FIG. 16F is an elevation cross-sectional view of the frame along line 16F-16F of FIG. 16A;
FIG. 16G is a perspective cross-sectional view of the frame along line 16F-16F of FIG. 16A; and

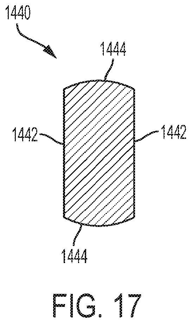

FIG. 17 is an elevation cross-sectional view of the shaft of the handle of FIG. 14A.

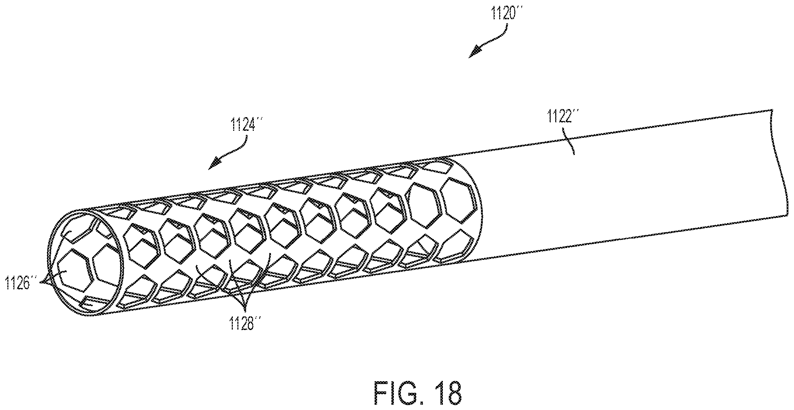

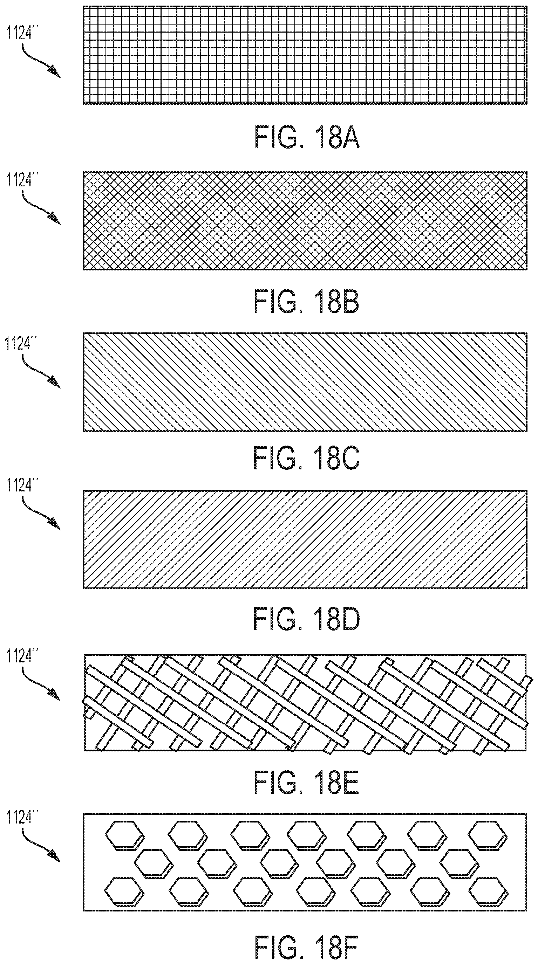

FIG. 18 is a perspective view of an outer sheath comprising a porous attenuating member, according to one embodiment of the present disclosure;
FIG. 18A is a side elevation view of a porous attenuating member comprising a plurality of square-shaped openings, according to one embodiment of the present disclosure;
FIG. 18B is a side elevation view of a porous attenuating member comprising a plurality of diamond-shaped openings, according to one embodiment of the present disclosure;
FIG. 18C is a side elevation view of a porous attenuating member comprising a plurality of openings formed by a helical structure wound in a particular direction, according to one embodiment of the present disclosure;
FIG. 18D is a side elevation view of a porous attenuating member comprising a plurality of openings formed by a helical structure wound in a particular direction, according to one embodiment of the present disclosure;
FIG. 18E is a side elevation view of a porous attenuating member comprising a plurality of openings formed by a helical wound ribbons, according to one embodiment of the present disclosure;
FIG. 18F is a side elevation view of a porous attenuating member comprising a plurality of hexagon-shaped openings, according to one embodiment of the present disclosure; and

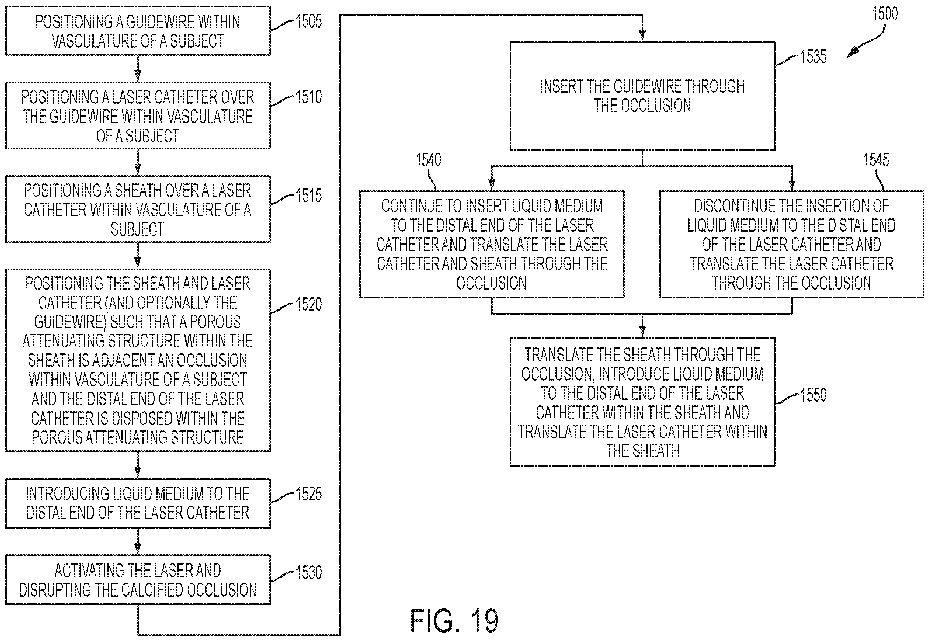

FIG. 19 is a representative flow diagram of a method of treating a subject using a laser catheter and sheath, according to one embodiment of the present disclosure.

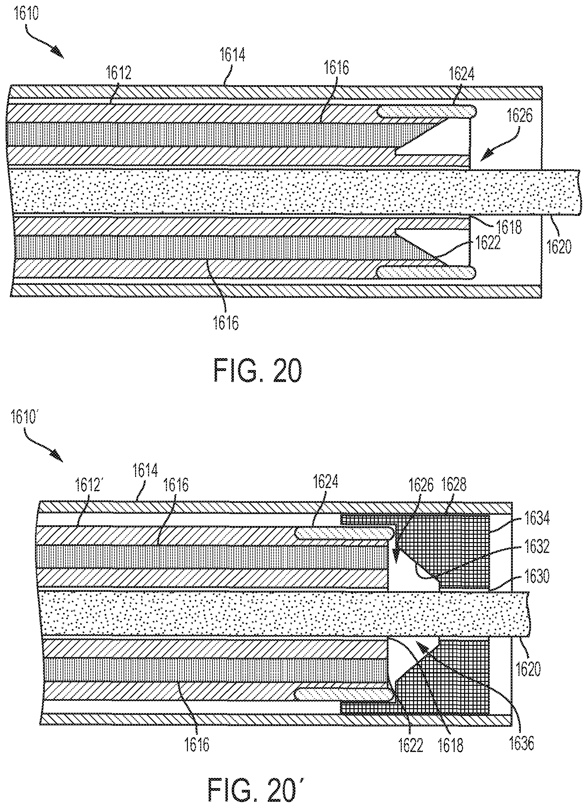

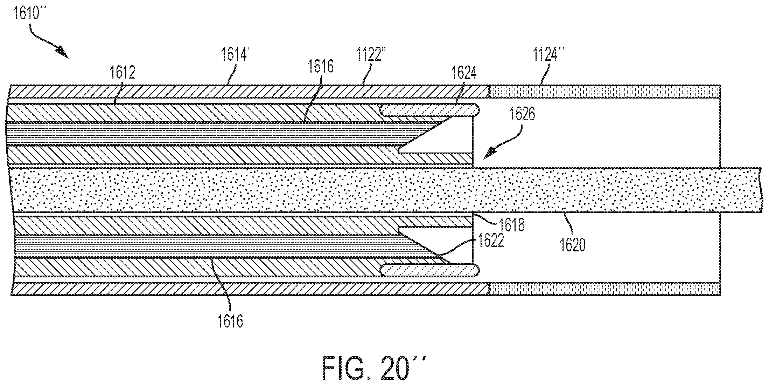

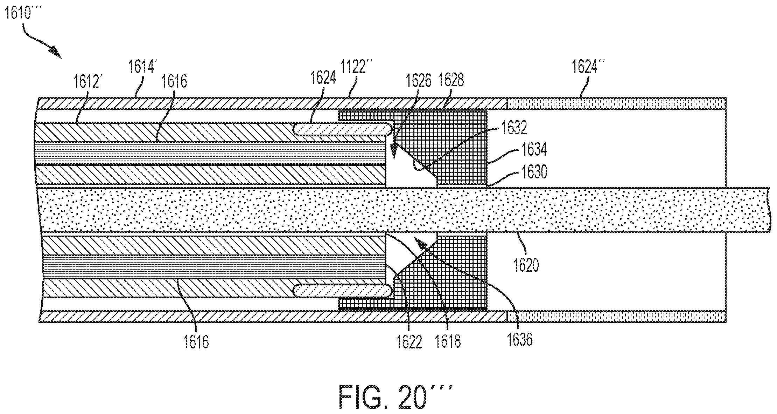

FIG. 20 is a representative cross-sectional view of the distal end of a catheter, according to one embodiment of the present disclosure.
FIG. 20' is a representative cross-sectional view of the distal end of the catheter illustrated in FIG. 20, according to an alternate embodiment of the present disclosure.
FIG. 20" is a representative cross-sectional view of the distal end of the catheter illustrated in FIG. 20, according to another alternate embodiment of the present disclosure.
FIG. 20'" is a representative cross-sectional view of the distal end of the catheter illustrated in FIG. 20, according to yet another alternate embodiment of the present disclosure.

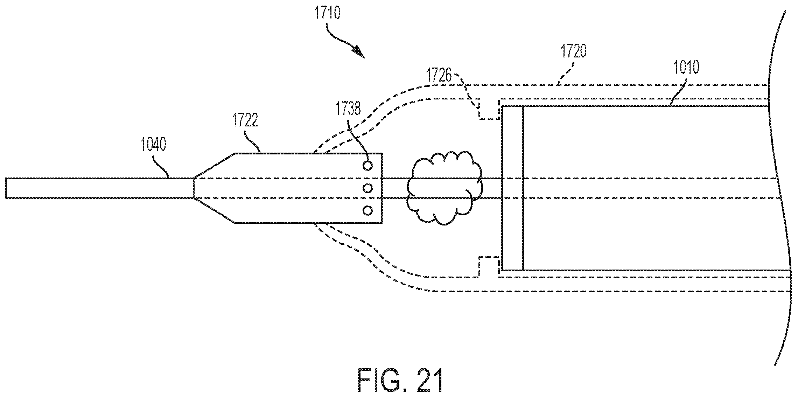

FIG. 21 is a representative side view of a catheter system including a laser catheter and a sheath having a sealable valve, according to an embodiment of the present disclosure.

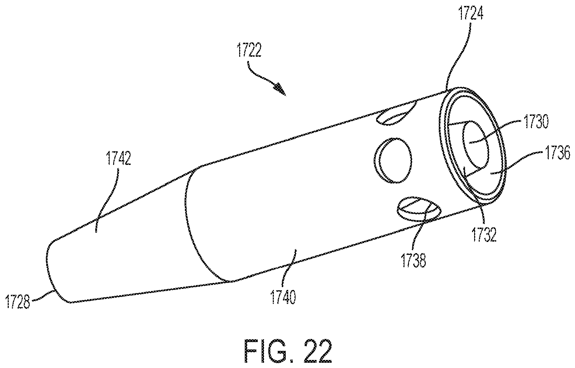

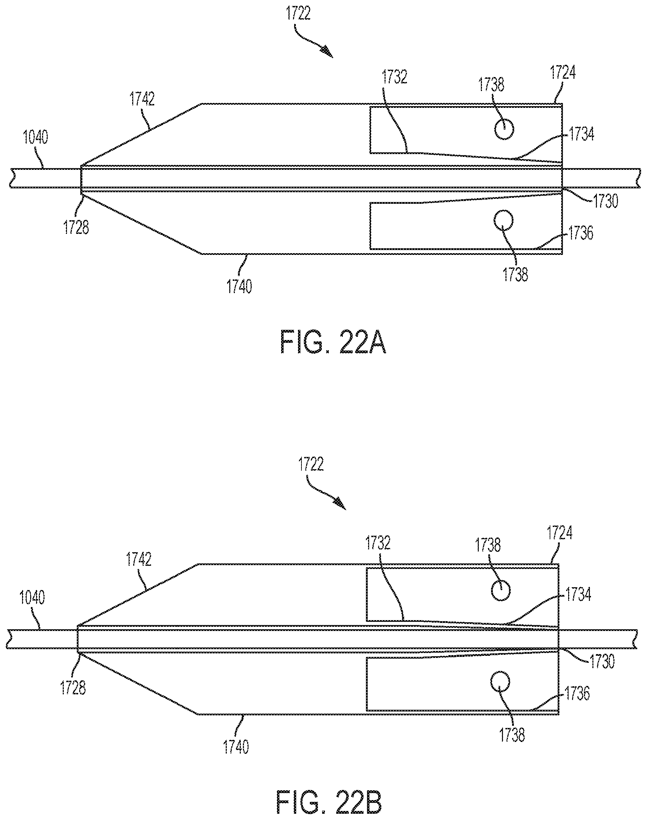

FIG. 22 is an enlarged representative perspective view of the sealable valve of the catheter system depicted in FIG. 21.
FIG. 22A is an enlarged representative cross-sectional side view of the sealable valve of the catheter system depicted in FIG. 21 in an unsealed configuration with respect to a guidewire.
FIG. 22B is an enlarged representative cross-sectional side view of the sealable valve of the catheter system depicted in FIG. 21 in a sealed configuration with respect to a guidewire.

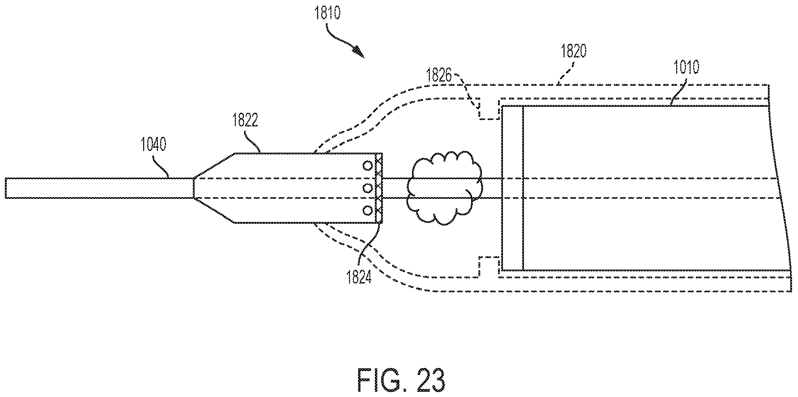

FIG. 23 is a representative side view of a catheter system including a laser catheter, a sheath having a sealable valve, and a light absorbing material support structure, according to an embodiment of the present disclosure.

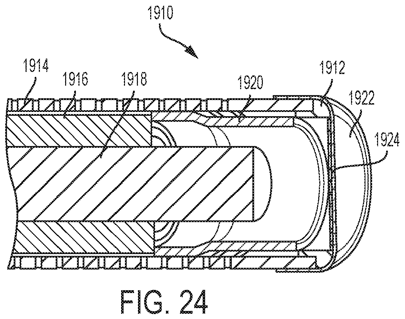

FIG. 24 is a cross-sectional view of a distal portion of a catheter system including a light absorbing material support structure, according to one embodiment of the present disclosure.

### DETAILED DESCRIPTION

The present disclosure relates generally to the use of medical devices for the treatment of vascular conditions. In particular, the present disclosure provides materials and methods for using laser-induced pressure waves to disrupt vascular blockages and to deliver therapeutic agents to the blockage area.

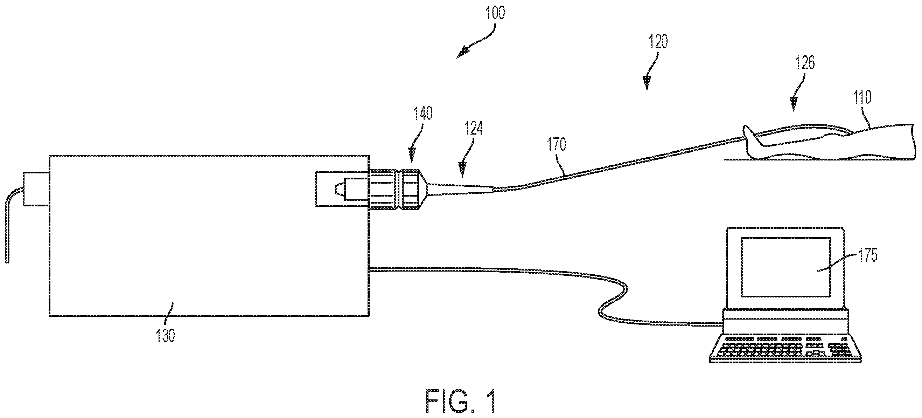

Referring to FIG. 1, there is depicted an exemplary ablation system 100 of the present disclosure. Ablation system 100 includes a laser apparatus 130 coupled to a laser controller 175. Controller 175 includes one or more computing devices programmed to control laser 130. Controller 175 may be internal or external to laser apparatus 130, such as a laser generator. Laser apparatus 130 may include an excimer laser or another suitable laser. In some embodiments, laser 130 produces light in the ultraviolet frequency range. In one embodiment, laser 130 produces optical energy in pulses.

Laser 130 is connected with the proximal end of a laser energy delivery system 120, illustratively a laser catheter 170 via coupler 140. Laser catheter 170 includes one or more transport members which receive laser energy from laser 130 and transports the received laser energy from a first, proximal end 124 of laser energy catheter 170 towards a second, distal end 126 of laser catheter 170. The distal end of catheter 170 may be inserted into a vessel or tissue of a human body 110. In some embodiments, system 100 employs a plurality of light guides as the transport members, such as optical fibers, that guide laser light from laser 130 through catheter 170 toward a target area in human body 110.

Exemplary laser catheter devices or assemblies may include laser catheters and/or laser sheaths. Examples of laser catheters or laser sheath are sold by The Spectranetics Corporation under the tradenames ELCA™ and Turbo Elite™ (each of which is used for coronary intervention or peripheral intervention, respectively, such as recanalizing occluded arteries, changing lesion morphology, and facilitating stent placement) and SLSII™ and GlideLight™ (which is used for surgically implanted lead removal). The working (distal) end of a laser catheter typically has a plurality of laser emitters that emit energy and ablate the targeted tissue. The opposite (proximal) end of a laser catheter typically has a fiber optic coupler 140 and an optional strain-relief member 124. The fiber optic coupler 140 connects to a laser system or generator 130. One such example of a laser system is the CVX-300 Excimer Laser System, which is also sold by the Spectranetics Corporation.

The laser controller 175 of FIG. 1 includes a non-transitory computer-readable medium (for example, memory 204) that includes instructions that, when executed, cause one or more processors 200 to control laser 130 and/or other components of ablation system 100. Controller 175 includes one or more input devices 206 to receive input from an operator. Exemplary input devices include keys, buttons, touch screens, dials, switches, mouse, and trackballs which providing user control of laser 130. Controller 175 further includes one or more output devices 208 to provide feedback or information to an operator. Exemplary output devices include a display, lights, audio devices which provide user feedback or information.

A laser source 210 of laser 130 is operatively coupled to laser controller 175. Laser source 210 is operative to generate a laser signal or beam and provide the laser signal through a fiber optic bundle 214 of catheter 170 to the human. Fiber optic bundle 214 serves as delivery devices for delivering the laser signal to the target area of the human body 110.

FIG. 1 depicts the catheter 170 entering the leg, preferably through the femoral artery, of the human body. As discussed above, it may be desirable to treat either CAD or PAD. After entering the femoral artery, it the catheter 170 is intended to treat CAD, the catheter 170 will be directed through the patient's vasculature system and to the coronary arteries. Alternatively, if the catheter 170 is intended to treat PAD, the catheter 170 will be directed through the patient's vasculature system and to the peripheral arteries, such as the vasculature below the knee, particularly the vasculature in the patient's legs and/or feet. Unlike balloon catheters, the catheter 170 of the present disclosure is able to more easily navigate and enter smaller sized vasculature because the overall diameter of the sheath is smaller in comparison to balloon catheters, thereby allowing the catheter 170 of the present disclosure more easily treat PAD. That is, the increased size of a balloon of an electrically-induced shockwave balloon catheter and/or a typical dilation balloon catheter (in comparison to the catheter 170 of the present disclosure) may prevent or increase the difficulty of the balloon-type catheter from entering, penetrating and/or treating the peripheral vasculature, such the vasculature below the knee in the legs and/or feet.

Referring to FIGS. 2A, 2B, 2C and 2D, the catheter 170 of the present disclosure may include an outer sheath 182, an inner sheath 184, one or more optical fibers 186, and a tip 180. The outer sheath 182, inner sheath 184, and one or more optical fibers 186 generally span the length of the catheter 170, and each have a proximal end and a distal end. The inner sheath 184 is disposed concentrically and/or radially within the outer sheath 182, and the one or more optical fibers 186 are disposed concentrically and/or radially within the inner sheath 184.

As depicted in FIG. 2C, at the distal end 126 of the catheter 170, the distal end of the outer sheath 182 is directly coupled to the tip 180. The inner sheath 184 and the one or more optical fibers 186 are not directly coupled to the tip 180. Rather, the inner sheath 184 and the one or more optical fibers 186 are disposed proximate the tip 180, thereby forming a cavity among the outer sheath 182, the inner sheath 184, one or more optical fibers 186, and the tip 180.

The inner sheath 184, which is constructed of a biocompatible polymer has one or more lumens 190, which are used to deliver a liquid medium to the cavity, thereby partially or completely filling the cavity with the liquid medium. The liquid medium is introduced to the catheter 170 through one or more liquid medium ports (not shown) in fluid communication with the one or more lumens 190 within the inner sheath 184 and disposed about the outer sheath 182. The liquid medium ports may also serve as a means for removing the liquid medium from the catheter 170. The liquid medium is configured to absorb light energy, produce pressure waves and create cavitation bubbles that produce additional resultant pressure waves that are transmitted to the tip 180 and/or the outer sheath 182 to disrupt a vascular obstruction. Liquid medium can include contrast medium, including for example, iodine-containing contrast medium or gadolinium contrast medium, as well as contrast solutions comprising dye(s) and/or particle(s). Additionally, any liquid medium can be used, as long as the liquid medium is coupled with a light source, such as emitters coupled to the one or more optical fibers, which emits light at a suitable wavelength such that the liquid absorbs the light, produces pressure waves and creates cavitation bubbles the produce additional resultant pressure waves. In some cases, the liquid medium can be contrast medium (for example, iodine-containing contrast medium or gadolinium contrast medium) and/or the liquid medium can be a contrast solution comprising a biocompatible fluid (for example, saline) in which a contrast dye(s) or particle(s) have been mixed at various concentrations.

As mentioned above, one or more optical fibers 186 are disposed within the inner sheath 184 extending from a proximal portion of the inner sheath 184 to the distal end of the inner sheath 184 and into the cavity. The proximal end of the one or more optical fibers is coupled to the laser generator 130. The distal end(s) of the one or more optical fibers 186 are proximate, at, or distal the distal end of the inner sheath 184. Again, one or more emitters are disposed at the distal end of the one or more optical fibers 186. The emitter(s) are in direct contact with the liquid medium, such that when laser light energy is emitted from the emitter(s), the liquid medium absorbs the emitted light, which in turn produces pressure waves and generates cavitation bubbles that produce additional pressure waves.

To treat a subject having a vascular occlusion, the tip 180 of the catheter 170 is positioned adjacent to the vascular occlusion. When the laser system 130 is activated, light energy travels through one or more optical fibers until the light energy is released from the emitter. As the liquid medium absorbs the light energy, pressure waves are produced. Additionally, the liquid medium rapidly expands and contracts, creating vapor bubbles that propagate pressure waves through the liquid medium. The energy produced by the pressure waves is captured within the cavity and converted to mechanical energy via moving the tip 180 and/or transferred to the vascular occlusion through the tip 180. The transfer of the energy produced by the pressure waves to the vascular occlusion is sufficient to disrupt vascular obstructions, particularly the calcified and/or fibrous (for example, calcium deposits) portions of a total occlusion. It is desirable for the mechanical energy created at the tip 180 by the pressure waves to be transferred to the occlusion. Accordingly, when the energy produced by the pressure waves is captured within the cavity, it is desirable for the forces generated by the pressure waves to propagate longitudinally, including in a forward (that is, parallel with the vessel) direction, thereby increasing the tip's ability to disrupt, destroy and/or penetrate the vascular obstructions. That is, as the pressure waves are produced, the tip 180 of the catheter rapidly moves (translates) forwards and backwards towards and away from, respectively, the occlusion. Pressure waves produced in this manner can also be used to increase vessel compliance prior to performing another procedure, such as a traditional balloon angioplasty or drug eluting balloon treatment.

In order to facilitate the direction in which the forces that are produced by the pressure waves translate into the movement of the tip 180 in a forward/backward longitudinal direction, the outer sheath 182 is not only flexible, but the outer sheath 182 also has the ability to expand and contract in a longitudinal direction. One example of such an outer sheath 182 includes a slotted or laser-cut hypotube constructed of a biocompatible material, such as stainless steel, or a biocompatible polymer. The hypotube has spring-like characteristics, which allow it to expand and contract in a longitudinal direction. Specifically, the slotted or laser cut pattern in the hypotube allows it to expand and contract. Another example of the outer sheath 182 may include of one or more spirally wound wires, thereby creating a coiled sheath, which also has the ability to expand and contract in a longitudinal direction.

In order to further facilitate the movement of the tip 180 in a longitudinal direction, it may be desirable to include a shield 188 disposed axially between the distal end of the inner sheath 184 and the proximal end of the tip 180, and disposed radially between the one or more optical fibers 186 and the outer sheath 182. As illustrated in FIG. 2C, the shield 188, which is depicted as a generally cylindrical tube, may increase the pressure waves' resistance in the radial direction, thereby reducing the ability of the pressure waves to travel radially towards the outer sheath 182 and may concentrate the energy produced by the pressure waves in the longitudinal direction. The configuration of the cylindrically-shaped shield 188 may allow for a reduced resistance in the longitudinal direction, in comparison to the radial direction, thereby potentially increasing the energy produced by the pressure waves in the longitudinal direction and increasing the tip's ability to translate in a forward/backward direction. The cylindrically-shaped shield 188 may also be configured such that its diameter is greater at its proximal end in comparison to its distal end, thereby potentially concentrating the pressure waves towards the center of the tip 180. Or the cylindrically-shaped shield 188 may be configured such that its diameter is less its proximal end in comparison to its distal end, thereby potentially concentrating the pressure waves towards the center of the tip 180.

The tip 180 illustrated in FIGS. 2B & 2C has a closed configuration. However, the present disclosure contemplates that the tip can also have an open configuration. Additionally, the tip 180 illustrated in FIGS. 2B & 2C is a separate component from the distal end 126 of laser catheter 170 and is coupled to the distal end 126. However, the present disclosure contemplates that the tip can also be integral with the distal end 126 of the laser catheter 170. The tip 180 illustrated in FIGS. 2B & 2C is generally tapered from a larger diameter to a smaller diameter as the tip progresses distally. Additionally, the shape of the tip 180 shown in FIGS. 2B & 2C is generally conical. Furthermore, the tip 180 has a completely solid configuration, but it may be also be partially solid. It is desirable for the tip to be constructed of a biocompatible material, such as stainless steel or a biopolymer.

Similar to the tip 180 illustrated in FIGS. 2B & 2C, the tip 180' in FIGS. 2B' & 2C' has a completely solid configuration, but the tip 180' in FIGS. 2B' & 2C' may alternatively have a partially solid configuration. Unlike the tip 180 illustrated in FIGS. 2B & 2C, the tip 180' in FIGS. 2B' & 2C' may have a generally convex, spherical shape. Although the present disclosure only depicts a generally conically-shaped tip 180 in FIGS. 2B & 2C and a generally spherically-shaped tip 180' in FIGS. 2B' & 2C', the tip may have alternative shapes, such as a flat shape, concave shape, triangular shape, a pyramid shape, chisel, etc.

Referring to FIG. 3, there is depicted an alternative embodiment of the catheter of the present disclosure. Similar to the tip 180' in FIGS. 2B' & 2C', the tip 380 in FIG. 3 has a generally spherical shaped. However, unlike the tip 180' in FIGS. 2B' & 2C', which has a solid configuration, the tip 380 in FIG. 3 has a hollow or shell-type configuration. Also, the tip 280 in this embodiment, as well as the other embodiments, may be press fit and/or welded to the outer sheath 182".

The embodiments of the catheter depicted in FIGS. 2B & 2C and FIGS. 2B' & 2C' and FIG. 3 include a shield 188. However, it may not be necessary to include a shield within the catheter. For example, FIG. 4 illustrates an embodiment of the catheter 170 in which the shield is omitted from the distal tip of the catheter. As depicted in this figure, in the event that a shield is omitted, it may be desirable for the outer sheath 182'" to include a solid portion between the distal end of the inner sheath 184'" and the proximal end of the tip 480 in order to create a non-porous cavity.

Referring to FIGS. 5, 5A and 5A', the distal end 526 of the catheter may include a tip 580 that comprises a non-metallic component in lieu of a metallic (for example, stainless steel) solid or hollow construction. Referring to FIG. 5A, the catheter includes an outer sheath 582, an inner sheath 584 disposed concentrically and/or radially within the outer sheath 582, and one or more optical fibers 586 disposed concentrically and/or radially within the inner sheath 584. The distal end of the outer sheath 582 is directly coupled (via a press fit and/or a weld) to the tip 580. The inner sheath 584 and the one or more optical fibers 586 are not directly coupled to the tip 580. Rather, the inner sheath 584 and the one or more optical fibers 586 are disposed proximate the tip 580, thereby forming a cavity among the outer sheath 582, the inner sheath 584, one or more optical fibers 586, and the tip 580.

Continuing to refer to FIG. 5A, the catheter may include a shield 588 disposed axially between the distal end of the inner sheath 584 and the proximal end of the tip 580, and disposed radially between the one or more optical fibers 586 and the outer sheath 582. As illustrated in FIG. 5A, the shield 588, which is depicted as a generally cylindrical tube, increases the pressure waves' resistance in the radial direction, thereby reducing the ability of the pressure waves to travel radially towards the outer sheath 582. The configuration of the cylindrically-shaped shield 588 allows for a reduced resistance in the longitudinal direction, in comparison to the radial direction, thereby increasing the tip's ability to translate in a forward/backward direction. The cylindrically-shaped shield 588 may also be configured such that its diameter is greater (or less) at its proximal end in comparison to its distal end, thereby potentially tapering in either the proximal or distal direction and concentrating the pressure waves towards the center of the tip 580. The shield 588 may also serve to create a sealed cavity at the distal end of the catheter, thereby preventing the leakage of the liquid medium through the outer sheath 582 because a portion of the shield overlaps with a portion of the outer sheath 582 that may be porous. Referring to FIG. 5A', if the shield is omitted, it may be desirable for the outer sheath 582 to include a solid or non-porous sheath portion between the distal end of the inner sheath 584 and the proximal end of the tip 580 in order to create a non-porous cavity.

The inner sheath also includes one or more lumens for passage of liquid medium into the cavity. The distal end(s) of the one or more optical fibers 586 are proximate, at, or distal the distal end of the inner sheath 584. Again, one or more emitters are disposed at the distal end of the one or more optical fibers 586. The emitter(s) are in direct contact with the liquid medium, such that when laser light energy is emitted from the emitter(s), the liquid medium absorbs the emitted light, which in turn produces pressure waves and generates pressure waves and/or cavitation bubbles that produce additional pressure waves.

As depicted in FIGS. 5A and 5A', the tip 580 has a circular construction, thereby creating a collar for the distal end of the outer sheath 582. The tip 580 also includes a flexible membrane 585 at its distal end. For example, the membrane 585 may be constructed of Mylar and be adhesively bonded to the distal end of the tip 580 in an orientation perpendicular to the longitudinal axis. In addition the membrane may be compliant in order to form against and engage the shape of the calcified cap, total occlusion or lesion.

To treat a subject having a vascular occlusion, the distal end of the catheter, particularly the tip 580 is positioned adjacent to the vascular obstruction with the membrane adjacent the vascular occlusion. The liquid medium is delivered to the cavity from the one more lumens within the inner sheath 584 through one or more liquid medium ports or between the outer sheath and the inner sheath or laser catheter. When the laser system 130 is activated, light energy travels through one or more optical fibers until the light energy is released from the emitter(s) at the end of the one or more optical fibers. As the liquid medium absorbs the light energy, the liquid medium rapidly expands and contracts, creating a pressure wave and/or a vapor bubbles that propagate pressure waves through the liquid medium. The energy produced by the pressure waves is captured within the closed system provided by the cavity and transferred to the vascular occlusion through the flexible membrane 585 of the tip 580. The transfer of the energy produced by the pressure waves to the vascular occlusion is sufficient to disrupt calcium deposits and/or fibrous tissue within the vascular occlusion. The forces generated by the pressure waves can propagate longitudinally in forward (that is, parallel to the vessel). Pressure waves produced in this manner can also be used to increase vessel compliance prior to performing another procedure, such as a traditional balloon angioplasty.

The embodiments of the catheters discussed hereinabove with respect to FIGS. 1-5 are integral catheters, such that the optical fiber(s) are integrated within the design of a single catheter. The present disclosure, however, also encompasses a two-piece catheter system or kit. Referring to FIGS. 6 and 6A, the catheter system may include a laser catheter 650 and a tubular sheath 682 having a lumen therein and/or therethrough and configured to surround the laser catheter 650. Depending upon whether the tubular sheath 682 has an open or closed distal end, the tubular sheath 682 may be coupled to a tip 680. A traditional laser catheter 650, including one or more optical fibers 686, can be inserted within the lumen, thereby allowing a clinician to translate the laser catheter 650 within sheath 682 along the longitudinal axis of the sheath in a forward (distal) and backwards (proximal) direction. For the purposes of this disclosure, the laser catheter 650 may include one or more one more lumens 690 to deliver the liquid medium from the one or more liquid medium ports. When the laser system 130 is activated, light energy travels through one or more optical fibers until the light energy is released from the emitter(s) at the end of the one or more optical fibers. As the liquid medium absorbs the light energy, the liquid medium rapidly expands and contracts, thereby creating a pressure wave and/or vapor bubbles that propagate pressure waves through the liquid medium. The energy produced by the pressure waves is captured within the closed system provided by the cavity and transferred to the vascular occlusion through the walls of the sheath 682 and/or the tip 680. The transfer of the energy produced by the pressure waves to the vascular occlusion is sufficient to disrupt calcium deposits and/or fibrous tissue within the vascular occlusion. Depending upon the location of the emitter(s) within lumen of the sheath 682, a smaller or larger cavity is created among the distal end of the laser catheter 650, the tip 680, and the sheath 682. It may desirable for the distal end of the laser catheter 650 to be disposed proximal of the tip 680 or translated inside of the outer sheath to create a cavity with which the forces generated by the pressure waves can propagate radially from multiple axial positions along the longitudinal axis of the outer sheath.

Depending upon the location of the emitter(s) within lumen of the sheath 682, the forces generated by the pressure waves may propagate radially, including in forward (that is, parallel to the vessel), upward (that is, perpendicular to the vessel), and backward (that is, proximally) directions. As depicted in FIG. 6, when the laser catheter 650 is disposed proximate the tip 680, the pressure waves may propagate radially from the sheath 650 and forward (that is, parallel to the vessel) from the tip. As depicted in FIG. 6A, upon the laser catheter 650 translating proximally along the longitudinal axis of the lumen within the sheath 682 in in comparison to the position of the laser catheter 650 in FIG. 6, the pressure waves may propagate in a direction radially from the sheath 650, forward (that is, parallel to the vessel), upward (that is, perpendicular to the vessel), and/or backward (that is, proximally). Accordingly, after the tip 680 of the sheath 682 destroys the calcified and/or fibrous tissue within the vascular occlusion, the sheath 682 may penetrate and cross the occlusion, and the laser catheter 650 can slide in proximal and/or distal direction to destroy additional portions of the occlusion.

Referring to FIG. 6B, the laser catheter 650' may also include a deflector 692 attached to its distal end via at least one, and possibly a plurality of, support member(s) 696, 698. The purpose of the deflector is to direct the pressure waves generated by the interaction between the liquid medium and the emitters from one or more optical fibers 686' within the liquid medium in a particular direction. In this figure, the deflector 692 is positioned along the longitudinal axis of the laser catheter 650', but the shape of the deflector 692 is oriented in a radial direction that is perpendicular to longitudinal axis of the laser catheter 650'. Accordingly, as the pressure waves are produced from the interaction between the liquid medium and the laser light energy emitted from the emitters, the deflector 692 may direct the pressure waves in a radial direction, such as 360 degrees about the circumference of the laser catheter 650' and/or sheath 682. The deflector 692 may be constructed of a non-metallic material or a metallic material, such as stainless steel. The deflector 692 may also have a solid or porous construction. Regardless of the construction of the deflector 692, the deflector 692 shall direct the pressure waves in a particular direction that is in an advantageous direction, such as toward the vascular occlusion, which may be on the side of the sheath 682.

Various shapes and configurations of deflectors are envisioned. For example, referring to FIG. 7, the laser catheter 750 may include a deflector 792 disposed distally from the one or more optical fibers 786 of the laser catheter 750, may be configured to direct the pressure waves in a radial direction less than 360 degrees (for example, 5 degrees, 10 degrees, 15 degrees, ... , 30 degrees, ... , 45 degrees, ... , 60 degrees, ... , 75 degrees, ... , 90 degrees, ... , 105 degrees, ... , 120 degrees, ... , 135 degrees, ... , 150 degrees, ... , 165 degrees, ... , 180 degrees, etc.) about the circumference of the laser catheter 750 and/or sheath 782. For example, the deflector 792 may have a solid construction with an opening facing a particular direction. And the shape and size of the opening within the deflector may dictate the direction that the pressure waves may travel. As depicted in FIG. 7, the laser catheter 750 may translate axially within the sheath 782 along the longitudinal axis of the laser catheter 750 and/or sheath 782. The present disclosure also contemplates that the laser catheter 750 may rotate within the sheath 782 about the longitudinal axis of the laser catheter 750 and/or sheath 782, thereby directing not only directing the pressure waves along a longitudinal direction but also about a radial direction.

The transfer of the energy produced by the creating a pressure wave to the vascular obstruction and/or to the walls of the vessel is sufficient to disrupt intraluminal as well as intimal and/or medial (within the tissue layer of the vascular wall) vascular obstructions (for example, calcium deposits). The forces generated by the pressure waves can propagate radially, including in forward (that is, parallel to the vessel), upward (that is, perpendicular to the vessel), and backward (that is, proximally) directions. Pressure waves produced in this manner can also be used to increase vessel compliance prior to performing another procedure, such as a traditional balloon angioplasty, drug-eluting balloon angioplasty and/or stent placement. That is, the pressure wave disruption of the intraluminal layer and/or medial layer and the vascular obstructions, can improve the vasculature's ability to absorb drugs, particularly when such drugs are applied with a drug eluting balloon.

Referring again to FIGS. 6 and 6A, the catheter system includes the laser catheter 650 disposed within the sheath 682 and proximate the tip 680, thereby allowing the pressure waves to propagate radially from the sheath 650 and forward (that is, parallel to the vessel) from the tip. In order to ensure that the laser catheter 650 is proximate the tip 680 to create a cavity between the distal end of the laser catheter 650, the sheath 682 and the tip 680, the laser catheter 650 and/or the sheath 682 may include stops and/or matingly engageable springs and/or recesses to maintain the laser catheter 650 at the desirable distance proximate the tip 680. The stops and/or matingly engageable springs and/or recesses shall also be configured to allow the clinician to easily disengage the laser catheter 650 from the sheath 682 so the laser catheter 650 may translate within the sheath 682.

Referring to FIGS. 8 and 8A, if it is not desirable to slide the laser catheter within the sheath, then the laser system may include laser catheter 850 that is engageable with and removable from a cap 870. The cap 870 may include a relatively short sheath attached to a tip 880 and an optional shield 888. Similar to the embodiment in FIGS. 6 and 6A, the embodiment in FIGS. 8 and 8A may include matingly engageable springs and/or recesses that are configured to allow the clinician to easily engage and disengage the laser catheter 850 from the cap 870.

The laser catheter 850 may include one or more layers of optical fibers 854 arranged circumferentially around or adjacent to an optional inner lumen, which may be used to insert a guidewire and/or the liquid medium. Or the laser catheter 850 may include one or more additional lumens to serve independent purposes. Again, the proximal end of the laser catheter is coupled to a laser generator. The one or more layers of optical fibers 854 are housed in a flexible tubular catheter and terminate at the distal emitter at the distal tip 856 of the laser catheter 850. The liquid medium travels through the lumen 852 until being introduced from one or more liquid medium ports (not shown) into the cavity. The emitter is in direct contact with the liquid medium such that when laser light energy is emitted from the proximal emitter, the liquid medium absorbs the emitted light, which in turn generates a pressure wave and/or cavitation bubbles that produce additional pressure waves, thereby converting the pressure waves to mechanical energy via moving the tip 880 and/or transferring the pressure to the vascular occlusion through the tip 880.

Referring to the flow chart in FIG. 9, the present disclosure includes a method 900 for treating a subject with a vascular obstruction using embodiments of the catheter assemblies and systems described herein. Although it is not illustrated in FIG. 9, it may be desirable to treat at least a portion of the vascular occlusion in the vessel of the subject prior to performing the method set forth in FIG. 9. Such treatment may include, for example, mechanically cutting and/or ablating (via application of laser light energy, microwave energy, radiofrequency energy, or the like) at least a portion of the vascular occlusion. The method 900 in FIG. 9 begins at block 910 by positioning an embodiment of a catheter assembly or system described herein within the vasculature of a subject. At block 920, the distal tip of the catheter assembly or system is positioned adjacent the obstruction within the vasculature (for example, by advancing the distal tip through the vasculature). At block 930, a liquid medium, such as any of the liquid mediums described herein, is introduced to cavity at and/or proximal the distal tip of the catheter assembly or system (for example, introduced via a space between an outer sheath and a laser catheter carried within the sheath). At block 940, laser light energy is delivered to one or more emitters (for example, optical fibers) located within the cavity to transmit pulses of light energy into the liquid medium. As described herein, transmission of pulses of light energy into the liquid medium creates a pressure wave and/or cavitation bubbles and additional resultant pressure waves to disrupt the vascular obstruction. At block 950, the catheter (and the emitters) may be translated relative to the sheath while laser light energy is delivered to one or more emitters and the liquid medium. The catheter (and the emitters) or the entire catheter assembly can be repositioned within the vasculature. The method 900 also includes ending the procedure when the desired therapeutic outcome is obtained, or repeating any of blocks 910 through 960 as may be necessary to treat a subject having a vascular obstruction. Furthermore, although it is not illustrated in FIG. 9, after performing the method 900 it may be desirable to use a drug eluting (coated) balloon (DEB or DCB) catheter to deliver drugs to the remnants of the vascular occlusion. Disrupting the vascular occlusion with the pressure waves prior to utilizing a DEB may increase the effectiveness of the drugs being applied to the vascular occlusion because to the pressure waves disrupt the intraluminal as well as medial (within the tissue layer of the vascular wall) vascular obstructions (for example, calcium deposits), thereby creating a pathway for the drug to enter the intraluminal and medial portions of the vasculature and/or vascular occlusion.

Referring to FIG. 10, there is depicted a laser catheter 1010 and a guidewire 1020 extending through the lumen of the catheter 1010. The laser catheter 1010 includes one or more layers of a plurality of optical fibers 1016 surrounding a lumen extending therethrough, and a sheath 1012 surrounds the layer(s) of optical fibers 1016. The distal end of the laser catheter 1010 may include a metal band 1014, which includes the strength of the distal end and provides a radiopaque marker. Examples of laser catheters 1010 or laser sheaths are sold by The Spectranetics Corporation under the tradenames ELCA™ and Turbo Elite™ (each of which is used for coronary intervention or catheterization such as recanalizing occluded arteries, changing lesion morphology, and facilitating stent placement) and SLSII™ and GlideLight™ (which is used for surgically implanted lead removal). Again, as illustrated in FIG. 10, the working (distal) end of a laser catheter typically has a plurality of laser emitters that emit energy and ablate the targeted tissue. The opposite (proximal) end of a laser catheter, which is not shown, typically has a fiber optic coupler that connects to a laser system or generator. One such example of a laser system is the CVX-300 Excimer Laser System, which is also sold by The Spectranetics Corporation.

Again, the present disclosure envisions a two-piece catheter system or kit. Referring to FIG. 11A, the system 1110 may include a laser catheter 1010 radially disposed within a sheath 1140. The system may optionally include a guidewire 1150 disposed within a lumen of the laser catheter 1010. As discussed above, a liquid medium is introduced into the sheath 1120 distal to the laser catheter 1010, particularly distal to the optical fibers/emitters of the laser catheter 1010 such that when the laser is activated, the liquid absorbs the light and creates pressure waves and/or cavitation bubbles and additional resultant pressure waves. Although the liquid is not shown in this figure, the liquid may be introduced through a lumen in the laser catheter 1010, a lumen in the sheath 1120 and/or the lumen or space between the laser catheter 1010 and the sheath 1120. Regardless of which of these locations is used, one or more liquid medium ports located at or toward the proximal end of the catheter system will be also be used.

Referring to FIG. 12, there is depicted a representative flow diagram of a method 1200 of removing an occlusion using a laser catheter 1010 to ablate a portion of the occlusion, and/or using the laser catheter 1010 in conjunction with the sheath 1120 to create pressure waves in the presence of a liquid medium and disrupt a portion of the occlusion. The method 1200 may include the step 1205 of positioning a guidewire 1130 within the vasculature 1140 of a subject, the step 1210 of positioning a laser catheter 1010 over the guidewire 1130 within the vasculature 1215, the step of positioning a sheath 1120 over the laser catheter 1010 within the vasculature and the step 1220 of positioning the sheath 1120 and laser catheter 1010 (and optionally the guidewire 1130) adjacent an occlusion 1150 within the vasculature 1140 of a subject. Referring again to FIG. 11A, positioning the sheath 1120 and laser catheter 1010 adjacent the occlusion 1150 creates a cavity for the liquid medium to collect distally of the laser catheter 1010, particularly distally of the emitters/optical fibers of the laser catheter 1010.

FIG. 11A depicts the distal end of the laser catheter 1010 proximal of the distal end of the sheath 1120. However, it is envisioned that the distal end of the laser catheter 1010 may be disposed at or distally of the distal end of the sheath 1120, as long as there is liquid medium between the emitters/optical fibers of the laser catheter 1010 and the occlusion 1150. The axial locations of the laser catheter 1010 and the sheath 1120 may be adjusted be translating either or both components with respect to one another. In order to visualize the respective locations of the laser catheter 1010 and the sheath 1120 under fluoroscopy, the laser catheter 1010 and the sheath 1120 may include radiopaque markers at any corresponding locations along their lengths.

Continuing to refer to FIGS. 11A, once the sheath 1120 and laser catheter 1010 are disposed adjacent the occlusion 1150, the liquid medium may be introduced to the distal end of the laser catheter as set forth in step 1225 of FIG. 12. Continuing to refer to FIG. 12, step 1230 includes activating the laser to create pressure waves in the presence of the liquid medium and disrupting a portion of the occlusion, particularly the calcified cap of the occlusion. The laser catheter 1010 and sheath 1120 may be used to traverse the entire occlusion 1150, as set forth in step 1240 of FIG. 12 (and optionally step 1235 of FIG. 12), or only disrupt a portion of the occlusion 1150. If the laser catheter 1010 and sheath 1120 are only used to disrupt a portion of the occlusion 1150, then the guidewire 1130 may penetrate and traverse the occlusion 1150. For example, FIG. 11B depicts the guidewire 1130 penetrating and traversing the occlusion 1150.

Referring to FIG. 11C, assuming that the laser catheter 1010 and sheath 1120 are only used to disrupt a portion of the occlusion 1150', the laser catheter 1120 may be used to traverse the occlusion 1150 without the sheath 1120. Referring to step 1245 of FIG. 12, the insertion of the liquid medium may be discontinued and the laser catheter 1010 may be used to ablate occlusion as the laser catheter 1150 passes over the guidewire 1130 through the occlusion 1150' while the sheath 1120 remains proximal of the occlusion.

Once the entire occlusion has been traversed by the laser catheter 1010, the opening created by the laser catheter 1010 should be large enough to translate the sheath 1120 distally and through the occlusion. At this point, both the distal end of the sheath 1120 and the distal end of the laser catheter 1010 should be distally of the occlusion. At this point, referring to FIG. 11D, the laser catheter 1010 is able to translate proximally while the sheath 1120 remains stationary within the occlusion. Upon introducing the liquid medium into the sheath 1120 in front of the laser catheter 1010, the laser may be activated, thereby creating pressure waves in the presence of the liquid medium. At least a portion of the pressure waves are directed radially, and as the laser catheter 1010 translates proximally within the sheath 1120, the pressure waves transmit through the sheath 1120 and/or the sheath 1120 itself expands and contracts, thereby disrupting the remainder of the occlusion 1150'''.

To ensure that the majority of the remainder of the occlusion 1150''' is disrupted, and if desired, disrupt the intraluminal layer and/or medial layer and the vascular obstructions, the laser catheter 1010 may be repeatedly translated distally and proximally within the sheath 1120. As discussed above, disruption of the intraluminal layer and/or medial layer and the vascular obstructions, can improve the vasculature's ability to absorb drugs, particularly when such drugs are applied with a drug eluting balloon. Also, it is contemplated that prior to, during and/or after any step in the process outlined in FIG. 12, the laser catheter 1010 may be used individually to ablate a portion of the occlusion, or the laser catheter 1010 may be used in conjunction with the sheath 1120.

FIGS. 11A-11D illustrate the catheter system as having a sheath 1120 with an open distal end or tip 1124. Referring to FIG. 13, the sheath 1120' may have a tip 1124 that is fully or partially closed. For example, if it is desirable to have a guidewire 1130 pass through the laser catheter 1010 and the sheath 1120', the tip 1124 will only be partially closed, but if it is not necessary to utilize a guidewire, then the tip 1124 may be fully closed.

Similar to FIGS. 11A-11D, the laser catheter 1010 may translate distally and/or proximally within the sheath 1120'. In order to ensure that a cavity remains between the distal end of the laser catheter 1010 and the proximal end of the tip of the sheath 1120', the sheath 1120' may include one or more internal stops 1160. The shape of the tip 1124 may be configured similar to the tips 180 illustrated and described with respect to FIGS. 2-6 such that the catheter system 1110', including the laser catheter 1010 tip 1124, is configured such that the energy produced by the pressure waves is captured within the cavity and the forces generated by the pressure waves propagate longitudinally, including in a forward (that is, parallel with the vessel) direction, thereby increasing the tip's ability to disrupt, destroy and/or penetrate the vascular obstructions.

Referring to FIGS. 14A and 14B, a laser catheter system 1410 generally includes a laser catheter 1412, a guidewire 1414, a sheath 1416, and a handle 1418 that translatably couples the laser catheter 1412 to the sheath 1416. The laser catheter 1412, the guidewire 1414, and the sheath 1416 may be similar to, for example, the components of the two-piece catheter systems or kits described herein. As a specific example, the laser catheter 1412, the guidewire 1414, and the sheath 1416 may be similar to the components described above in connection with FIGS. 11A-11D. The laser catheter 1412 is disposed within a lumen of the sheath 1416 and the handle 1418, and the laser catheter 1412 includes a proximal coupling 1420 for coupling to the handle 1418. The guidewire 1414 is disposed within a lumen of the laser catheter 1412. The sheath 1416 includes a proximal coupling 1422 for coupling to the handle 1418.

A liquid medium is introduced into the sheath 1416 distal to the laser catheter 1412, particularly distal to the optical fibers/emitters of the laser catheter 1412 such that when the laser is activated, the liquid absorbs the light and creates pressure waves and/or cavitation bubbles and resultant pressure waves. The liquid is introduced via the lumen or space between the laser catheter 1412 and the sheath 1416, which in turn receives the liquid from a proximal port 1424 coupled to the sheath 1416.

Referring now to FIGS. 14A, 14B, 15A-15G, the handle 1418 generally includes a base 1426 that couples to the sheath 1416 and a drive mechanism 1428 that couples to the laser catheter 1412. As described in further detail below, a portion of the drive mechanism 1428 is translatably coupled to the base 1426 to facilitate translating the laser catheter 1412 within the lumen of the sheath 1416 (for example, to the various positions shown in FIGS. 11A-11D). The drive mechanism 1428 may be translated to a proximal position relative to the base 1426 (see FIGS. 15A-15C), a distal position relative to the base 1426 (see FIGS. 15E and 15F), and an infinite number of intermediate positions therebetween (see FIGS. 15D and 15G). As a result, the laser catheter 1412 may be translated to corresponding positions relative to the sheath 1416.

Referring now to FIGS. 14A-17, the base 1426 includes an elongated, hollow frame 1430 that movably couples to the drive mechanism 1428. The frame 1430 includes a proximal portion 1432, an intermediate portion 1434, and a distal portion 1436. The proximal portion 1432 defines a proximal passageway 1438 for translatably receiving a shaft 1440 of the drive mechanism 1428 therein. Referring specifically to FIGS. 16B, 16C, and 17, the proximal passageway 1438 may include a first key feature that, by coupling to a second key feature of the shaft 1440, inhibits rotation of the shaft 1440 relative to the frame 1430. For example, the second key feature of the shaft 1440 may be a non-circular cross-sectional area, and the first key feature of the proximal passageway 1438 may be a cross-sectional area that is approximately identical (that is, permitting sufficient clearance to permit relative longitudinal translation, but inhibit relative rotation and transverse translation) to the cross-sectional area of the shaft 1440, or a cross-sectional area that is approximately identical to a portion of the cross-sectional area of the shaft 1440. As a more specific example and as shown in FIGS. 16B, 16C, and 17, the shaft 1440 includes rectangle-like cross-sectional shape, with two opposing flat side surfaces 1442 and two opposing arcuate side surfaces 1444. The proximal passageway 1438 includes a cross-sectional area that is approximately identical to a portion of the cross-sectional area of the shaft 1440. Specifically, the proximal passageway 1438 is defined by four opposing flat side surfaces 1446 and two opposing arcuate side surfaces 1448. The flat side surfaces 1446 and the arcuate side surfaces 1448 engage the flat side surfaces 1442 and the arcuate side surfaces 1444 of the shaft 1440, respectively, to permit relative longitudinal translation, but inhibit relative rotation and transverse translation of the shaft 1440 relative to the frame 1430. In the present example, the proximal passageway 1438 is also defined by two additional opposing arcuate side surfaces 1449 that extend between the flat side surfaces 1446. The arcuate side surfaces 1449 are disposed apart from the shaft 1440 to reduce sliding friction between the shaft 1440 and the frame 1430.

Referring specifically to FIGS. 16A, 16D, and 16E, the intermediate portion 1434 of the frame 1430 includes a first bearing portion 1450, a second bearing portion 1452, and an opening 1454 extending therebetween and aligned with the proximal passageway 1438. Each of the first and second bearing portions 1450, 1452 includes first and second bearing surfaces 1456, 1458. The first and second bearing surfaces 1456, 1458 rotatably support a control element 1460 of the drive mechanism 1428. Each of the first and second bearing portions 1450, 1452 also includes a clearance surface 1462 between the bearing surfaces 1456, 1458. The clearance surface 1462 is also disposed radially inwardly relative to the bearing surfaces 1456, 1458. The clearance surface 1462, together with the opening 1454, facilitates driving engagement of the control element 1460 with the shaft 1440, as described in further detail below. Within the opening 1454, each of the first and second bearing portions 1450, 1452 includes a guide surface 1464. The guide surface 1464s translatably couple to the shaft 1440 and inhibit the shaft 1440 from rotating within the frame 1430.

Referring briefly to FIGS. 15H-15J, to facilitate assembly of the base 1426, each clearance surface 1462 may be monolithically coupled with the first bearing surface 1456, 1458. After positioning the shaft 1440 within the frame 1430 and the control element 1460 over the first bearing surface 1456, 1458 and the clearance surface 1462, each clearance surface 1462 may couple to the second bearing surface 1456, 1458 via, for example, press fit, one or more adhesives, snap connectors (not shown), or the like.

Referring to FIGS. 16A, 16F, and 16G, the distal portion 1436 of the frame 1430 may be similar to the proximal portion 1432 of the frame 1430. That is, the distal portion 1436 defines a distal passageway 1466 aligned with the opening 1454 for translatably receiving the shaft 1440. Referring specifically to FIGS. 16F, 16G, and 17 and in a similar manner to the proximal passageway 1438, the distal passageway 1466 may include a first key feature that, by coupling to the second key feature of the shaft 1440, inhibits rotation of the shaft 1440 relative to the frame 1430. For example, the second key feature of the shaft 1440 may be a non-circular cross-sectional area, and the first key feature of the distal passageway 1466 may be a cross-sectional area that is approximately identical to the cross-sectional area of the shaft 1440, or a cross-sectional area that is approximately identical to a portion of the cross-sectional area of the shaft 1440. In accordance with the specific example described above and as shown in FIGS. 16F, 16G, and 17, the distal passageway 1466 includes a cross-sectional area that is approximately identical to a portion of the cross-sectional area of the shaft 1440. Specifically, the distal passageway 1466 is defined by four opposing flat side surfaces 1468 and two opposing arcuate side surfaces 1470. The flat side surfaces 1468 and the arcuate side surfaces 1470 engage the flat side surfaces 1442 and the arcuate side surfaces 1444 of the shaft 1440, respectively, to permit relative longitudinal translation, but inhibit relative rotation and transverse translation of the shaft 1440 relative to the frame 1430. In the present example, the distal passageway 1466 is also defined by two additional opposing arcuate side surfaces 1472 that extend between the flat side surfaces 1468. The arcuate side surfaces 1472 are disposed apart from the shaft 1440 to reduce sliding friction between the shaft 1440 and the frame 1430.

Referring again to FIGS. 14A-16G, at its proximal end, the frame 1430 couples to a proximal cover 1476 (for example, via press fit, one or more adhesives, or the like). The proximal cover 1476 includes a proximal aperture 1478 (see FIGS. 15F and 15G) for permitting the laser catheter 1412 to extend into the frame 1430. At its distal end, the frame 1430 couples to a distal cover 1480 (for example, via press fit, one or more adhesives, or the like). The distal cover 1480 includes a distal aperture 1482 (see FIGS. 15F and 15G) for permitting the laser catheter 1412 to extend out of the frame 1430 and into the sheath 1416. The distal aperture 1482 press-fittingly receives a tube 1484 (for example, a hypotube 1484) that extends into the shaft 1440 and receives the laser catheter 1412. The distal aperture 1482 also press-fittingly receives a distal coupling 1486 that detachably and sealingly couples to the proximal coupling 1422 of the sheath 1416.

Referring now to FIGS. 14A and 15A-15J, the drive mechanism 1428 generally includes the shaft 1440 and the control element 1460. Referring specifically to FIGS. 15F-15J, the shaft 1440 includes a shaft 1440 passageway 1488 for permitting the laser catheter 1412 to extend through the shaft 1440 and for receiving the tube 1484. The shaft 1440 passageway 1488 press-fittingly receives a proximal coupling 1490 that detachably and sealingly couples to the proximal coupling 1420 of the laser catheter 1412. As such, movement of the control element 1460 relative to the base 1426 causes the shaft 1440 to translate within the base 1426, the laser catheter 1412 thereby translates within the lumen of the sheath 1416.

The shaft 1440 passageway 1488 also receives a seal 1492, for example, an O-ring, that translatably engages the outer surface of the tube 1484. As such, the seal 1492 inhibits the liquid in the shaft 1440 passageway 1488 (received from the sheath 1416 via the distal coupling 1486 and the hypotube 1484) from exiting the shaft 1440 by flowing between the shaft 1440 and the tube 1484.

As described briefly above, the control element 1460 is rotatably supported by the frame 1430. The control element 1460 includes a first engagement feature that couples to a second engagement feature of the shaft 1440 such that rotation of the control element 1460 relative to the base 1426 causes translation of the shaft 1440 relative the base 1426 (and translation of the laser catheter 1412 within the lumen of the sheath 1416). For example and as shown in the Figures, the first engagement feature may be a first threaded surface 1494 within the control element 1460, and the second engagement feature may be a second threaded surface 1496 formed on the arcuate side surfaces 1444 of the shaft 1440. Stated differently, the shaft 1440 may include a second, interrupted threaded surface that extends from the opening 1454 in the frame 1430 to engage the first threaded surface 1494 of the control element 1460. In any case, rotation of the control element 1460 and the first threaded surface 1494, together with the shaft 1440 being rotatably fixed within the frame 1430, causes translation of the second threaded surface 1496 and the shaft 1440 relative to the frame 1430 (and translation of the laser catheter 1412 within the lumen of the sheath 1416).

Pressure waves generally have different characteristics in comparison to ultrasound. Ultrasound typically consists of periodic oscillations with limited bandwidth. Pressure waves are single, mainly positive pressure pulses that are followed by comparatively small tensile wave components. Ultrasound applies an alternating high frequency load to tissue, with a frequency range of several megahertz, and can thus lead to heating, tissue tears and cavitation at high amplitudes. The effect of pressure waves in comparison, however, largely involves radially directed energy, as described above, enabling the treatment of deep tissue as well as adjacent tissue with enhanced sensitivity.

The ability of the catheter of the present disclosure to generate pressure waves for treating a vascular obstruction in a subject involves the suitable coupling of the light system and the liquid medium. Any wavelength of light can be used, including but not limited to, laser light, visible light, ultraviolet light and infrared light, as long as the light being emitted is coupled with a liquid medium capable of absorbing the light and producing pressure waves. Additionally, any liquid medium can be used, as long as the liquid medium is coupled with a light source that emits light at a suitable wavelength such that the liquid absorbs the light and creates pressure waves and/or cavitation bubbles. In some cases, the liquid medium can be contrast medium (for example, iodine-containing contrast medium or gadolinium contrast medium) and/or the liquid medium can be a contrast solution comprising a biocompatible fluid (for example, saline) in which a contrast dye(s) or particle(s) have been mixed at various concentrations.

The degree of force generated by the pressure waves depends in part on the degree of absorption of the light energy by the liquid medium as well as total energy deposited by the light source. Generally, the greater the absorption of the light energy by the liquid medium 160, the greater the force generated by the pressure waves. Also, the greater the amount of the light energy delivered to the liquid medium 160, the greater the force generated by the pressure waves. For example, an excimer laser typically emits laser light at a wavelength of about 308 nanometers at pulse durations between about 120 nanoseconds and about 140 nanoseconds, at frequencies between about 25 pulses per second to about 80 pulses per second, and with a total energy output between about 1 to about 100 millijoules. In some cases, however, total energy output of a laser light system can range from greater than 0 to about 300 mJ. When emitted within contrast medium, such as iodine-containing contrast medium or gadolinium contrast medium, there will be a very high degree of absorption by the contrast medium, thus creating pressure waves with sufficient force to treat a vascular obstruction in a subject.

Light energy can be emitted at any suitable wavelength capable of generating cavitation bubbles and producing corresponding pressure waves. Light energy can be emitted between about 1 nanometer and about 1 millimeter. In some cases, light can be emitted from about 10 nanometers to about 5000 nanometers. In some cases, light can be emitted from about 100 nanometers to about 1000 nanometers. In some cases, light can be emitted from about 250 nanometers to about 750 nanometers. In some cases, light can be emitted from about 300 nanometers to about 600 nanometers. In still other cases, light can be emitted from about 300 nanometers to about 350 nanometers.

Light energy can be emitted at any suitable pulse duration capable of generating cavitation bubbles and producing corresponding pressure waves. In some cases, light can be emitted at pulse durations between about 1 nanosecond to about 1 second. In some cases, light can be emitted at pulse durations between about 10 nanoseconds to about 500 nanoseconds. In some cases, light can be emitted at pulse durations between about 100 nanoseconds to about 150 nanoseconds. In still other cases, light can be emitted at pulse durations between about 120 nanoseconds and about 140 nanoseconds.

Light energy can be emitted at any suitable pulse repetition frequency (PRF), or pulses per second, capable of generating cavitation bubbles and producing resultant pressure waves that propagate through the surrounding vasculature. In some cases, light can be pulsed at a frequency of between about 1 pulse to about 500 pulses per second. In some cases, light can be pulsed at a frequency of between about 10 pulses to about 250 pulses per second. In some cases, light can be pulsed at a frequency of between about 10 pulses to about 150 pulses per second. In some cases, light can be pulsed at a frequency of between about 10 pulses to about 100 pulses per second. In other cases, light can be pulsed at a frequency of between about 50 pulses to about 150 pulses per second. In other cases, light can be pulsed at a frequency of between about 50 pulses to about 100 pulses per second. In still other cases, light can be pulsed at a frequency of between about 25 pulses to about 80 pulses per second.

The total number of pulses administered during a particular treatment period depends on a variety of factors, including patient characteristics, the type of condition being treated, and the specific characteristics of the vascular obstruction, as one of ordinary skill in the art would readily appreciate based on the present disclosure. In some cases, the total number of pulses administered during a treatment period can range from a single pulse to any number of pulses generated in a 10 second treatment period, a 15 second treatment period, a 20 second treatment period, a 25 second treatment period, a 30 second treatment period, up to a 1 minute treatment period. Treatment periods can be repeated depending on the extent of the vascular obstruction remaining after initial treatment.

The degree of force generated by the pressure waves can be modulated by using lasers that produce laser light energy at different wavelengths and at different pulse durations, as would be appreciated by one of ordinary skill in the art based on the present disclosure. For example, different degrees of force may be required to break apart a vascular obstruction, as compared to the degree of force required to deliver a therapeutic agent to vascular tissue. In some embodiments, a laser having a holmium source, referred a Holmium laser, can emit laser light energy at a wavelength of about 2,100 nanometers (nm) and can be coupled with various light absorbing materials, including an aqueous or saline-based medium, to treat a vascular obstruction in a subject.

Several other additional sources of laser light energy can be paired with corresponding light absorbing materials to generate pressure waves to treat a vascular obstruction. For example, YAG crystal lasers can produce wavelengths of infrared light, which is highly absorptive in aqueous solutions. Aqueous solutions can be used as light absorbing material or medium to generate pressure waves. Aqueous solutions include, but are not limited to, saline, dextrose, radio-opaque contrast, lactated ringer's, and electrolyte solutions. In some cases, YAG wavelengths can be doubled to generate visible spectrum light of 532 nm wavelength. Materials or medium capable of absorbing light of this wavelength include, but are not limited to, gold nanospheres, nitrite solutions, potassium permanganate solutions, copper salts, aluminum solutions, aluminon, ammonia salts, and dyes such as hemotoxylin and propidium iodide. Light absorbing materials such as these can be part of a solution, such as an aqueous solution as described above, and/or they can be applied as coatings on various surfaces within a device.

In some embodiments, a Holmium YAG laser can emit laser light energy at a wavelength of about 2,120 nm and can be coupled with various light absorbing materials, including an aqueous or saline-based medium, to treat a vascular obstruction in a subject. In some embodiments, a thulium laser, such as a Thulium YAG laser, can emit laser light energy at a wavelength of about 2,013 nm and can be coupled with various light absorbing materials, including an aqueous or saline-based medium, to treat a vascular obstruction in a subject. In some embodiments, a thulium laser, such as a Thulium Fiber laser, can emit laser light energy at a wavelength of about 1,908 nm and can be coupled with various light absorbing materials, including an aqueous or saline-based medium, to treat a vascular obstruction in a subject. In some embodiments, an Nd-YAG laser can emit laser light energy at a wavelength of about 1,064 nm and can be coupled with various light absorbing materials to treat a vascular obstruction in a subject. In some embodiments, a doubled YAG laser laser can emit laser light energy at a wavelength of about 532 nm and can be coupled with various light absorbing materials to treat a vascular obstruction in a subject. In some embodiments, an alternative band YAG laser can emit laser light energy at a wavelength of about 1,319 nm and can be coupled with various light absorbing materials to treat a vascular obstruction in a subject. In still other embodiments, an Er-YAG laser can emit laser light energy at a wavelength of about 2,940 nm and can be coupled with various light absorbing materials to treat a vascular obstruction in a subject.

Carbon dioxide (CO₂) lasers can emit infrared light that is highly absorptive in aqueous solutions. CO₂ lasers are common surgical lasers and are highly absorptive in tissues due to their high water content. Light absorbing materials that can be coupled with CO₂ lasers that emit infrared light, such as light emitted at a 10.6 micron wavelength, to generate pressure waves include, but are not limited to, aqueous solutions such as saline, dextrose, radio-opaque contrast, lactated ringer's, and electrolyte solutions.

Nitrogen lasers can be used to produce low frequency, high energy laser pulses. Nitrogen lasers can emit light in the UV spectrum can emit laser light energy at a wavelength of about 337 nm and can be coupled with various light absorbing materials to generate pressure waves, including but not limited to, radio-opaque contrast as well as metals and oxides such as aluminum, silver, gold, copper, nickel, cerium, zinc, titanium, and dyes such as hydroxycoumarin and aminocoumarin.

Other medically useful lasers that can be used to generate a pressure wave to treat a vascular obstruction include Ti-Sapphire lasers, which can emit laser light energy at wavelengths of about 800 nm; Ruby lasers, which can emit laser light energy at wavelengths of about 694 nm; and Alexandrite lasers, which can emit laser light energy at about 755 nm. These medical lasers emit laser light energy in the near infrared light spectrum, and can be used for pressure wave generation. Light absorbing material or medium that can be coupled with these laser include, but are not limited to, dyes and colorants which could be used in solution, suspension, or coating on another material or surface within a device. Various materials capable of absorbing laser light energy in these wavelengths include aqueous copper, copper salts, and cupric sulfate, and materials such as fluorophores that are used in fluorescent microscopy (for example, methylene blue).

Dye lasers can also be used to generate pressure waves to treat a vascular occlusion. In some cases, dye lasers can be tuned to output a specific wavelength of light in the visible spectrum, which can allow for the optimization of the laser for a certain light absorbing material, as an alternative or in addition to, using a material which is highly absorptive of a specific wavelength of light. In this way, the light absorbing material can be any of the previously mentioned materials, as well as dyes, colorants, and visible light chromophores.

For certain applications, it may be desirable to increase the amount and/or the size of cavitation bubbles produced along with a pressure wave that is generated by emitting laser light energy into a corresponding light absorbing liquid medium. For example, when entering smaller diameter sized blood vessels, the size of the catheter may be limited. In some cases, the force that cavitation bubbles exert on tissue (for example, a vascular occlusion) may be proportional to the size of the individual cavitation bubbles created, as the bubbles expand and contract after laser light energy is emitted into liquid medium and a pressure wave is generated. That is, the strength of the initial pressure wave and/or the size of the cavitation bubble may be limited with the use of a non-gas saturated liquid medium. One manner by which the size of individual cavitation bubbles can be increased (for example, to impart greater amount of force on a particular tissue) is to saturate the liquid medium with gaseous substances so that the gas within the liquid medium exhibits a higher vapor pressure as compared to that of the liquid medium without such gas. Suitable gaseous substances that may be used to create gas-saturated liquid medium include, but are not limited to, ambient air, carbon dioxide, iodine gas, oxygen, nitrogen, compressed air, nitrous oxide, and combinations of these.

The higher vapor pressure of the gaseous substance added to the liquid medium will cause the gaseous substance to return to a gaseous state faster (under smaller pressure fluctuations) than the liquid medium. In other words, less pressure is required to cause the saturated gaseous substances to come out of solution, resulting in the creation of larger cavitation bubbles, and concomitantly, a greater amount of force. In some cases, the use of gas-saturated liquid medium allows for the use of laser light energy at decreased intensities, or decreased pulses or pulse durations, without any accompanying decrease in the overall force generated by the cavitation bubbles (as each cavitation bubble is larger). This can enhance both the safety and efficacy of the procedure being performed.

The gaseous substances can be imparted to the liquid medium through various means, including under pressure, through mechanical agitation, and/or by bubbling the gas into the liquid medium. In some cases, gas-saturated liquid medium can be prepared prior to a procedure and then delivered to the distal end of a catheter prior to performing the procedure. Additionally or alternatively, gaseous substances can be delivered into that liquid medium that is already present in the catheter.

The gases and/or gaseous substances may be dissolved and quantified by the amount of gases present in a 1 kg of the liquid medium. The maximum amount of gas that will dissolve in the liquid medium is dependent on the solubility of the particular gas in that liquid medium, the pressure, and the temperature as described by Henry's law of gas solubility. For example, carbon dioxide may be dissolved into water at a concentration of 1.25 g/kg of water or less at 30 degrees C under atmospheric pressure. And upon dissolving carbon dioxide into water or saline, an overall concentration between 0.25-3.5 g/kgH₂O is produced. The concentrations of other dissolved gases in a kilogram of liquid medium ranges from 1mg-1g/kg for iodine, 5-80mg/kg for oxygen, 5-40mg/kg for nitrogen, 5-500mg/kg for room air, and 0. 1-4g/kg for nitrous oxide.

The gases and/or gaseous substances may be dissolved in quantities above the theoretical limit, which is known as super saturation. The theoretical limit is described by Henry's law as mentioned previously. By dissolving the gases under increased pressure or decreased temperature and then returning it to normal atmospheric conditions, it is possible to dissolve a larger quantity of gas then is possible at atmospheric conditions. For example, 2.5g of carbon dioxide may be dissolved into 30 degrees C water under 2 atm of pressure, and then returned to atmospheric pressure. For any dissolved gas, the saturation percentage is defined by the concentration of gas over the theoretical maximum concentration. For any of the previously mentioned gases in a supersaturated solution, the saturation percentage can range from 100-300 percent.

The use of a gas saturated liquid medium or super saturated liquid medium may also increase the initial pressure wave caused by the interaction of the laser light and the liquid medium. That is, the gas saturated liquid medium or super saturated liquid medium may contain larger potential energy, which when activated by the laser light, may create a larger initial pressure wave in comparison to a pressure wave created by the interaction of laser light and a non-gas saturated liquid medium.

Additionally or alternatively, methods of the present disclosure also include activating at least one proximal laser emitter enclosed within the sheath assembly to send pulses of laser light energy through the liquid medium and propagating pressure waves to assist in stent deployment. Pressure waves generated from cavitation bubbles can assist in seating or expanding the stent to its full diameter as part of a medical procedure.

As discussed above, activating one or more emitters and transmitting pulses of light energy into the liquid medium produces cavitation bubbles. Upon emitting light from an emitter, such as a laser catheter, within a sheath that contain a liquid medium, the cavitation bubbles may be produced within the interior of the sheath and/or exterior to the sheath. Assuming that the cavitation bubbles are created on the interior of the sheath, it may be desirable to limit some or all of the potential expansion of the relevant portion of the sheath caused by the cavitation bubbles. That is, it may be desirable to reduce or prevent the sheath's ability to expand and contract upon creation of the cavitation bubbles therein so as to reduce or prevent the sheath from applying a hydraulic force or pressure to the vascular occlusion and/or to the walls of the vessel. Also, assuming that the cavitation bubbles are created on exterior of the sheath within the vessel wall, it may be desirable to reduce or prevent the formation of such cavitation bubbles so as to reduce or prevent the cavitation bubbles themselves from applying a hydraulic force or pressure to the vascular occlusion and/or to the walls of the vessel.

Referring to FIG. 18, there is depicted a perspective view of biocompatible sheath 1120" that can be used in conjunction with a laser catheter or any of the earlier embodiments to perform a method of treating a subject, such as removing or treating a vascular occlusion. The sheath 1120" may include a sleeve or jacket 1122" and a porous attenuating member 1124". FIG. 18 illustrates the porous attenuating member 1124" as being exposed and coupled to the distal end of the sleeve 1122" via an adhesive. The porous attenuating member 1124", however, can alternatively be integrally disposed within the sleeve 1122", disposed on the exterior of the sleeve 1122" and/or disposed on the interior of the sleeve 1122". Additionally, if the porous attenuating member 1124" is coupled to the distal end of the sleeve 1122" or the porous attenuating member 1124" is integrally disposed within the sleeve 1122" or disposed on the interior of the sleeve 1122", the entire porous attenuating member 1124" may be covered (unexposed) by the sleeve 1122", the entire porous attenuating member 1124" may be exposed, or a portion (for example, distal portion) of the porous attenuating member 1124" may be exposed and another portion (for example, proximal portion) may be covered.

FIG. 18 also illustrates the porous attenuating member 1124" as being disposed at the distal end of the sheath 1120". The porous attenuating member 1124", however, may alternatively and/or additionally as be disposed at the proximal end of the sheath 1120", the central portion of the sheath 1120", any location or multiple locations between the proximal end and distal end of the sheath 1120", or in the entire length or substantially the entire length of the sheath 1120".

The porous attenuating member 1124" has two purposes. One purpose is to reinforce the sleeve 1122" and/or the sheath 1120", and the other purpose is to reduce or prevent the formation of cavitation bubbles exterior of the attenuating member 1124", the sleeve 1122" and/or the sheath 1120". Regarding the reinforcing the sleeve 1122", coupling the porous attenuating member 1124" with the sleeve 1122" may reduce or prevent the sheath's ability to expand and contract upon creation of the cavitation bubbles therein so as to reduce or prevent the sleeve 1122" from applying a hydraulic force or pressure to the vascular occlusion and/or to the walls of the vessel. Both the attenuating member 1124" and the sleeve 1122" are constructed of biocompatible materials. Coupling the porous attenuating member 1124" with the sleeve 1122" forms a rigid or semi-rigid structure within the sheath 1120" such that it applies a small hydraulic force or it does not apply a hydraulic force to the vascular occlusion and/or to the walls of the vessel upon formation of cavitation bubbles therein. It may be desirable that the majority or only force(s) applied to the vascular occlusion and/or to the walls of the vessel are a result of the pressure waves that pass through the 1120", thereby allowing more precise control over the pressure waves.

Regarding the other purpose of the attenuating member 1124", which is to reduce or prevent the formation of cavitation bubbles exterior of the attenuating member 1124", the sleeve 1122" and/or the sheath 1120" and continuing to refer to FIG. 18, the openings 1126" within the attenuating member 1124" may prevent the formation of large sized cavitation bubbles on the sheath 1120". The openings 1126" not only allow the pressure waves to pass therethrough, but the quantity and size of the openings 1126", particularly with respect to the remainder of the structural mass (or portions thereof 1128") of the sleeve 1122", may also limit the size of the cavitation bubbles that can form on the exterior of the sheath 1120". The relationship between the open area and the closed area (or the ratio of the open area to the overall area) within the attenuating member 1124" should be such that a sufficient amount of the pressure waves pass through the attenuating member 1124". And the size of the openings 1126" should allow the pressure waves to pass therethrough, while also limiting the size of the cavitation bubbles that can form on the exterior of the sheath 1120". Accordingly, it may be desirable for the ratio of the open area to the overall area of the attenuating member 1124" to be between 1 percent-99 percent, including any increment therebetween such as 2 percent, 3 percent, 4 percent, 5 percent, 6 percent, 7 percent, 8 percent, 9 percent, 10 percent, ..., 15 percent ...20 percent, ..., 25 percent, ...,30 percent, ..., 35 percent, ..., 40 percent, ..., 45 percent, .. ., 50 percent, ..., 55 percent, ..., 60 percent, ..., 65 percent, ..., 70 percent, ..., 75 percent, ..., 80 percent, ..., 85 percent, ..., 90 percent, 91 percent, 92 percent, 93 percent, 94 percent, 95 percent, 96 percent, 97 percent, and 98 percent. It may also be desirable for the ratio of the open area to the overall area of the attenuating member 1124" to be within a particular range such as between 5 percent to 95 percent, 10 percent to 90 percent, 15 percent to 85 percent, 20 percent to 80 percent, 25 percent to 75 percent, 30 percent to 70 percent, 35 percent to 65 percent, 40 percent to 60 percent, and 45 percent to 55 percent. Additionally, for any of the above listed ratios it may be desirable for each opening to have a particular size, such as between 50 microns to 1000 microns (1 millimeter), including any increment therebetween such as 100 microns, ..., 125 microns, .., 150 microns, 175 microns, ..., 200 microns, ..., 225 microns, ..., 250 microns, ..., 300 microns, ..., 325 microns, ..., 350 microns, ..., 400 microns, ..., 425 microns, ..., 450 microns, ..., 475 microns, ..., 500 microns, ..., 525 microns, ..., 550 microns, ..., 575 microns, ..., 600 microns, ..., 625 microns, .., 650 microns, ..., 675 microns, ..., 700 microns, ..., 725 microns, ..., 750 microns, ..., 775 microns, ..., 800 microns, ..., 825 microns, ..., 850 microns, ..., 875 microns, ... and 950 microns. It may also be desirable for the size openings within the attenuating member 1124" to be within a particular range such as between 100 to 900 microns, 150 to 850 microns, 200 to 800 microns, 250 to 750 microns, 300 to 700 microns, 350 to 650 microns, 400 to 600 microns, and 450 to 550 microns.

The openings 1126" in the attenuating member 1124" depicted in FIG. 18 are shown as hexagons, which are disposed around the circumference of the attenuating member 1124", as well as along its length. Although the openings 1126" in the attenuating member 1124" are illustrated as hexagons, the openings may have an alternate shape, such as a circle, oval, triangle, square, rectangle, polygon, diamond, pentagon, heptagon, octagon, nonagon, and decagon. For example, FIG. 18A illustrates a side view of a porous attenuating member 1124" comprising a plurality of square-shaped openings; FIG. 18B is a side view of a porous attenuating member 1124" comprising a plurality of diamond-shaped openings, FIG. 18C is a side view of a porous attenuating member 1124" comprising a plurality of openings formed by a helical structure wound in a particular direction (for example, clockwise or left to right) while FIG. 18D is a side view of a porous attenuating member 1124" comprising a plurality of openings formed by a helical structure wound in an alternate direction (for example, counter-clockwise or right to left). Additionally, the two helically formed porous attenuating members 1124" may be combined to form the porous attenuating member 1124" depicted in FIG. 18E. The porous attenuating member 1124" depicted in FIG. 18E is similar to the porous attenuating member 1124" depicted in FIG. 18B, but the porous attenuating member 1124" depicted in FIG. 18B is braided and the porous attenuating member 1124" depicted in FIG. 18E is wound or formed by one or two hypotubes. Additionally, the structural mass (or portions thereof) of the porous attenuating member 1124" depicted in FIG. 18E is larger than the structural mass (or portions thereof 1128") of the porous attenuating member 1124" depicted in FIG. 18B because braided materials are generally smaller in size. Referring to FIG. 18F, the structural mass (or portions thereof) of the attenuating member 1124" are substantial in comparison to the size of the hexagonal openings.

Referring to FIG. 19, there is depicted a representative flow diagram of a method 1500 of treating a subject using a laser catheter 1010 (depicted in FIG. 10) and the sheath 1120" (depicted in FIG. 18), and/or using the laser catheter 1010 in conjunction with the sheath 1120 to ablate an occlusion and/or create pressure waves in the presence of a liquid medium and disrupt a portion of the occlusion as depicted in FIGS. 11A-11D. The method 1500 may include the step 1505 of positioning a guidewire 1130 within the vasculature 1140 of a subject, the step 1510 of positioning a laser catheter 1010 over the guidewire 1130 within the vasculature 1515, the step of positioning a sheath 1120 over the laser catheter 1010 within the vasculature and the step 1520 of positioning the sheath 1120 and laser catheter 1010 (and optionally the guidewire 1130) adjacent an occlusion 1150 within the vasculature 1140 of a subject. Referring again to FIG. 11A, positioning the sheath 1120 and laser catheter 1010 adjacent the occlusion 1150 creates a cavity for the liquid medium to collect distally of the laser catheter 1010, particularly distally of the emitters/optical fibers of the laser catheter 1010.

FIG. 11A depicts the distal end of the laser catheter 1010 proximal of the distal end of the sheath 1120. However, it is envisioned that the distal end of the laser catheter 1010 may be disposed at or distally of the distal end of the sheath 1120, as long as there is liquid medium between the emitters/optical fibers of the laser catheter 1010 and the occlusion 1150. The axial locations of the laser catheter 1010 and the sheath 1120 may be adjusted be translating either or both components with respect to one another. In order to visualize the respective locations of the laser catheter 1010 and the sheath 1120 under fluoroscopy, the laser catheter 1010 and the sheath 1120 may include radiopaque markers at any corresponding locations along their lengths.

Continuing to refer to FIGS. 11A, once the sheath 1120 and laser catheter 1010 are disposed adjacent the occlusion 1150, the liquid medium may be introduced to the distal end of the laser catheter as set forth in step 1525 of FIG. 19. Continuing to refer to FIG. 19, step 1530 includes activating the laser to create pressure waves in the presence of the liquid medium and disrupting a portion of the occlusion, particularly the calcified cap of the occlusion. The laser catheter 1010 and sheath 1120 may be used to traverse the entire occlusion 1150, as set forth in step 1540 of FIG. 19 (and optionally step 1535 of FIG. 19), or only disrupt a portion of the occlusion 1150. If the laser catheter 1010 and sheath 1120 are only used to disrupt a portion of the occlusion 1150, then the guidewire 1130 may penetrate and traverse the occlusion 1150. For example, FIG. 11B depicts the guidewire 1130 penetrating and traversing the occlusion 1150.

Referring to FIG. 11C, assuming that the laser catheter 1010 and sheath 1120 are only used to disrupt a portion of the occlusion 1150', the laser catheter 1120 may be used to traverse the occlusion 1150 without the sheath 1120. Referring to step 1545 of FIG. 19, the insertion of the liquid medium may be discontinued and the laser catheter 1010 may be used to ablate occlusion as the laser catheter 1150 passes over the guidewire 1130 through the occlusion 1150' while the sheath 1120 remains proximal of the occlusion.

Referring to step 1550 of FIG. 19 and once the entire occlusion has been traversed by the laser catheter 1010, the opening created by the laser catheter 1010 should be large enough to translate the sheath 1120 distally and through the occlusion. At this point, both the distal end of the sheath 1120 and the distal end of the laser catheter 1010 should be distally of the occlusion. At this point, referring to FIG. 11D, the laser catheter 1010 is able to translate proximally while the sheath 1120 remains stationary within the occlusion. Upon introducing the liquid medium into the sheath 1120 in front of the laser catheter 1010, the laser may be activated, thereby creating pressure waves in the presence of the liquid medium. At least a portion of the pressure waves are directed radially, and as the laser catheter 1010 translate proximally within the sheath 1120, the pressure waves transmit through the sheath 1120 and/or the sheath 1120 itself expands and contracts, thereby disrupting the remainder of the occlusion 1150'''.

To ensure that the majority of the remainder of the occlusion 1150'" is disrupted, and if desired, disrupt the intraluminal layer and/or medial layer and the vascular obstructions, the laser catheter 1010 may be repeatedly translated distally and proximally within the sheath 1120. As discussed above, disruption of the intraluminal layer and/or medial layer and the vascular obstructions, can improve the vasculature's ability to absorb drugs, particularly when such drugs are applied with a drug eluting balloon. Also, it is contemplated that prior to, during and/or after any step in the process outlined in FIG. 19, the laser catheter 1010 may be used individually to ablate a portion of the occlusion, or the laser catheter 1010 may be used in conjunction with the sheath 1120.

As discussed above, transmitting pulses of light energy from an emitter into a liquid medium creates pressure waves and/or cavitation bubbles and additional resultant pressure waves that disrupt at least a portion of a vascular obstruction. The catheter may include a guidewire lumen through which a guidewire can pass and cross the occlusion. It may also be desirable to excite and vibrate the guidewire to increase the guidewire's ability to pierce and cross the occlusion. Accordingly, the present disclosure also contemplates directing the laser light energy emitted by the emitter into the liquid medium in a direction which causes the liquid medium to propagate pressure waves toward the guidewire lumen and/or guidewire such that the pressure waves excite and vibrate the guidewire.

Referring to FIG. 20, there is depicted a cross-sectional view of the distal end of system 1610 including a laser catheter 1612 radially disposed within a sheath 1614. As shown, the distal end of the catheter 1612 includes one or more layers of optical fibers 1616 arranged circumferentially around an inner guidewire lumen 1618 that receives a guidewire 1620. The inner layer of optical fibers 1616 extends to the distal tip 1626 of the catheter 1612 and terminates at the distal emitter 1622 within the catheter 1612. The liquid medium may be introduced distal to the catheter 1612 through a lumen in the catheter 1612 (for example, the guidewire lumen 1618), a lumen in the sheath 1614 (not shown), and/or the lumen or space between the laser catheter 1612 and the sheath 1614.

Continuing to refer to FIG. 20, in addition to having a plurality of optical fibers 1616 and a guidewire lumen 1618, the catheter 1612 may also include an outer band 1624 that surrounds the distal tip 1626, thereby increasing the strength and rigidity of the distal tip 1626. As mentioned above, the present disclosure contemplates directing the laser light energy emitted by the emitter 1622 into the liquid medium in a direction which causes the liquid medium to propagate pressure waves toward the guidewire lumen 1618 and/or the guidewire 1620 such that the pressure waves excite and vibrate the guidewire 1620. A means for directing laser light emitted from the emitter 1622 towards the guidewire lumen 1618 or the guidewire 1620 includes disposing the emitter 1622 proximate the distal tip 1626 of the catheter 1612 and/or proximate the distal end of the outer band 1624 such that the emitter 1622 is recessed from the distal tip 1626 of the catheter 1612 and/or proximate the distal end of the outer band 1624 along the longitudinal axis of the catheter 1612. By recessing the emitter 1622 from the distal tip 1626 of the catheter 1612 and/or proximate the distal end of the outer band 1624, the pressure waves may be directed toward the guidewire lumen 1618 and/or the guidewire 1620.

An additional means for directing laser light emitted from the emitter 1622 towards the guidewire lumen 1618 and/or the guidewire 1620 includes directing the emitter 1622 toward the guidewire lumen 1618 or the guidewire 1620. For example, as discussed above, the term "emitter" as used herein may refer to an end portion of a fiber or an optical component that emits light from a distal end thereof. The emitter 1622 is directed towards the guidewire lumen 1618 and/or the guidewire 1620 because the optical fiber is tapered in a manner that the light emitted therefrom is directed radially inward towards the guidewire lumen 1618 and/or the guidewire 1620. As illustrated in FIG. 20, the guidewire lumen 1618 and/or guidewire 1620 may extend longitudinally distal of the emitter 1622. Accordingly, as the laser light is emitted from the emitter 1622, the light interacts with the liquid medium, and the liquid medium absorbs the light energy, thereby creating pressure waves and/or cavitation bubbles and additional resultant pressure waves that cause the guidewire lumen 1618 and/or guidewire 1620 to excite and/or vibrate.

Referring to FIG. 20', there is depicted an alternate embodiment of the present disclosure, particularly an alternate embodiment of a means for directing laser light emitted from the emitter 1622 towards the guidewire lumen 1618 and/or the guidewire 1620. Similar to the embodiment discussed above with respect to FIG. 20, the system 1610' in FIG. 20' includes a catheter 1612' having a plurality of optical fibers 1616, a guidewire lumen 1618, and an outer band 1624 that surrounds the distal tip 1626. This embodiment also includes a cap 1628 having a guidewire lumen 1630 extending therethrough.

The cap 1628 can be either removably coupled to the catheter 1612', particularly removably coupled to the outer band 1624, or the cap 1628 can be permanently affixed to the catheter 1612', particularly permanently affixed to the outer band 1624. The cap 1628 includes a proximal (for example, interior) side 1632 and a distal (for example, exterior) side 1634. The interior side 1632 is tapered such that a cavity 1636 forms between the distal end of the catheter 1612' and the interior side 1632 of the cap 1628, thereby allowing the liquid medium to enter and collect within the cavity 1636. Although FIG. 20' is depicted as having a catheter 1612' with a flush distal end and a tapered, recessed cap 1628 to create the cavity 1636 between the catheter 1612' and the cap 1628 for the liquid medium to collect, the present disclosure also contemplates having a catheter with a recessed distal end, as depicted in FIG. 20, that could be used in conjunction with a cap 1628 having a flush or recessed interior side 1632 to create a cavity for the liquid medium to collect. Accordingly, as the laser light is emitted from the emitter 1622, the light interacts with the liquid medium within the cavity, and the liquid medium absorbs the light energy, thereby creating pressure waves and/or cavitation bubbles and additional resultant pressure waves that cause the guidewire lumen 1618 and/or guidewire 1620 to excite and/or vibrate.

The sheath 1614 may be, for example, any of the sheaths described herein. In some embodiments, the sheath 1614 may be the sheath 1140 shown in FIGS. 11A-11D, and the catheter 1612 or 1612' may be translatably carried therein. Referring now to FIGS. 20" and 20"', systems 1610" and 1610''' include laser catheters 1612 and 1612', respectively. The systems 1610" and 1610''' also include a sheath 1614' that translatably carries the catheters 1612 and 1612', respectively. The sheath 1614' may be the sheath 1120" shown in FIGS. 18-18E. That is, the sheath 1614' includes a sleeve or jacket 1122" and a porous attenuating member 1124", which may be any of the attenuating members described herein.

The porous attenuating member 1124" has multiple purposes, as follows: (1) reinforcing the sleeve 1122" and/or the sheath 1614'; (2) reducing or preventing the formation of cavitation bubbles exterior of the attenuating member 1124", the sleeve 1122" and/or the sheath 1614'; (3) redirecting at least a portion of the pressure waves toward the guidewire lumen 1618 and/or guidewire 1620 to excite and/or vibrate the guidewire 1620. Accordingly, the attenuating member 1124" acts as (1) a means for reinforcing the sleeve 1122" and/or the sheath 1614'; (2) a means for reducing or preventing the formation of cavitation bubbles exterior of the attenuating member 1124", the sleeve 1122" and/or the sheath 1614'; (3) a means for redirecting at least a portion of the pressure waves toward the guidewire lumen 1618 and/or guidewire 1620 to excite and/or vibrate the guidewire 1620.

Further details regarding the first and second purposes of the attenuating member 1124" are described in connection with FIG. 18. Regarding the ability of the attenuating member 1124" to redirect at least a portion of the pressure waves toward the guidewire lumen 1618 and/or guidewire 1620 to excite and/or vibrate the guidewire 1620, the pressure waves or portion of the pressure wave(s) that do not pass through the attenuating member 1124" may be redirected by the attenuating member 1124" toward the guidewire lumen 1618 and/or guidewire 1620 to excite and/or vibrate the guidewire 1620. The sizes of the openings 1126" of the attenuating member 1124" (see FIG. 18) may be selected to control the amount of pressure waves that are reflected toward the guidewire lumen 1618 and/or guidewire 1620.

Also, similar to the discussion included above with respect to FIG. 18 the porous attenuating member 1124" depicted in FIGS. 20" and 20'" is shown at the distal end of the sheath 1614'. The porous attenuating member 1124", however, may alternatively and/or additionally as be disposed at the proximal end of the sheath 1614', the central portion of the sheath 1614', any location or multiple locations between the proximal end and distal end of the sheath 1614', or in the entire length or substantially the entire length of the sheath 1614'. Therefore, as the catheter 1612 or 1612' translates within the sheath 1614', the guidewire 1620 will continue to excite and/or vibrate.

As described above, for example, with reference to FIG. 13, catheter systems according to embodiments of the present disclosure may have distal tips that are fully or partially closed. In some of these embodiments, it may be desirable to seal the sheath with the guidewire upon introduction of the liquid medium to the closed distal tip. FIGS. 21-22B illustrate a catheter system 1710 according to such an embodiment. That is, the catheter system 1710 includes a sheath 1720 (which is hidden in FIG. 21 to illustrate internal components of the catheter system 1710) that has a partially-closed tip 1722. The sheath 1720 carries a laser catheter, such as the laser catheter 1010 described herein, and the laser catheter 1010 may translate distally and/or proximally within the sheath 1720. As shown in FIG. 21, the sheath 1720 may taper proceeding distally toward the tip 1722. Alternatively, the sheath 1720 may include a flat surface that receives the tip 1722. As another alternative, the tip 1722 may have a similar size to the lumen of the sheath 1720 and be press-fittingly received in the lumen. In order to ensure that a cavity remains between the distal end of the laser catheter 1010 and the proximal end 1724 of the tip 1722 of the sheath 1720, the sheath 1720 may include one or more internal stops 1726. The shape of the tip 1722 may be configured similar to the tips 180 illustrated and described with respect to FIGS. 2-6 such that the catheter system 1710, including the laser catheter tip 1722, is configured such that the energy produced by the pressure waves is captured within the cavity and the forces generated by the pressure waves propagate longitudinally, including in a forward (that is, parallel with the vessel) direction, thereby increasing the tip's ability to disrupt, destroy and/or penetrate the vascular obstructions.

The tip 1722 includes the proximal end 1724, a distal end 1728, and a lumen 1730 extending therethrough from its proximal end 1724 to its distal end 1728. The tip 1722 also includes a valve that seals the intersection of the tip 1722 and the guidewire 1040 as the guidewire 1040 passes through the guidewire lumen 1730. One example of a valve is that which is depicted in FIGS. 22-22B which illustrate a flange 1732 that is disposed at and/or toward the proximal end 1724 of the tip 1722.

Referring back to FIGS. 21-22B, upon introducing the guidewire 1040 through the lumen 1730 of the laser catheter 1010 and into the guidewire lumen 1730 of the tip 1722, the guidewire 1040 and tip 1722 are slidably coupled such that the tip 1722 can slide over the guidewire 1040 (or the guidewire 1040 can slide through a lumen 1730 of the tip 1722), as depicted in FIG. 22A. As illustrated in this figure, there is a gap (or opening) caused by the guidewire lumen 1730 between the flange 1732 and the guidewire 1040. If the gap is maintained during introduction of the liquid medium into the distal end of the catheter system 1710 (via, for example, the opening or gap between the laser catheter 1010 and the sheath 1720), the liquid medium would travel through the guidewire lumen 1730 and into the patient's vasculature, which may be undesirable. The flange 1732, which may include a tapered portion 1734 that tapers from the tip's distal end 1728 toward its proximal end 1724, is configured to radially collapse upon introduction of the liquid medium into the distal end of the catheter system 1710 due to the increased fluid pressure on the flange 1732. The increased fluid pressure on the flange 1732 actuates the flange 1732 and moves it radially inward toward the guidewire lumen 1730 such that the gap between flange 1732 and the guidewire 1040 closes, thereby creating a seal between the between flange 1732 and the guidewire 1040, as depicted in FIG. 22B. The reduced thickness of the tapered portion 1734 of the flange 1732 as the flange 1732 tapers radially inward towards the guidewire lumen 1730 as the flange 1732 progresses from the distal end 1728 toward the proximal end 1724 increases the flange's ability to flex upon exposure to the pressure created upon introduction of the liquid medium. Upon removal of the liquid medium from the distal end of the catheter system 1710, the pressure within the catheter system 1710, the pressure on the flange 1732 decreases, and the flange 1732 naturally retracts to its original position as depicted in FIG. 22A, thereby reestablishing the gap between the tip 1722 and the guidewire 1040 so that the two components may slide with respect to one another. Accordingly, the flange 1732 acts as sealable valve within the tip 1722, and the flange 1732 is actuated with the introduction and removal of the liquid medium into and from the distal end of the catheter system 1710.

Although the tapered portion 1734 illustrated in FIGS. 22A and 22B tapers from the tip's distal end 1728 toward its proximal end 1724, the direction of the taper may be reversed such that the tapered portion 1734 tapers from the tip's proximal end 1724 toward its distal end 1728. Additionally, the flange 1732 may taper towards any portion along its length such that a portion of the flange 1732 is thinner at one or more locations along its length in comparison to other locations along its length. Accordingly, upon an increased fluid pressure being imparted on the flange 1732, thinner portion of the flange 1732 actuates and moves radially inward toward the guidewire lumen 1730 such that the gap between flange 1732 and the guidewire 1040 closes, thereby creating a seal between the between flange 1732 and the guidewire 1040.

The tip 1722 may be constructed from any type of compressible or compliant biopolymers, such as silicones or flouro-polymers, compliant adhesives, etc. The configuration of the tip 1722 depicted in these figures includes an exterior wall 1736 and the flange 1732 disposed radially therein, to create a gap therebetween for the liquid medium to enter and actuate the flange 1732. The flange 1732 is also depicted as being disposed toward the proximal end 1724 of the tip 1722, which itself is depicted as tubular, and its distal end 1728 has an inward taper that tapers distally from the exterior wall 1736 towards the guidewire lumen 1730. Although the tip 1722 is depicted as including particular components and shapes, the present disclosure shall include other shapes and components known to one of skill in the art. Moreover, the tip 1722 may alternatively include a self-sealing tube constructed of any type of compressible or compliant biopolymers, such as silicones or flouro-polymers, compliant adhesives, etc. For example, the tip 1722 may include a tube that has a lumen 1730 passing therethrough such that upon insertion of a guidewire, the lumen expands, and upon removable of the guidewire, the lumen contracts, thereby appearing as a slit.

Continuing to refer to FIGS. 21-22B, the tip 1722 may include one or more openings 1738 through its exterior wall 1736. The openings 1738 allow the liquid medium to reach the flange 1732 not only from the gap between the flange 1732 and the exterior wall 1736 at the proximal end 1724 of the tip 1722 but also at a location distal the proximal end 1724 of the tip 1722. Allowing the liquid medium to reach the flange 1732 at or toward its distal portion, potentially increases the likelihood and effectiveness of actuating the flange 1732. Although the tip 1722 is illustrated as having a tubular section 1740 from its proximal end 1724 and a tapered section 1742 from the end of its tubular section 1740 toward the tip's distal end 1728, the scope of this disclosure shall include other shapes for the tip 1722.

As discussed herein, as the laser light is emitted from the emitter(s), the light interacts with the liquid medium, and the liquid medium absorbs the light energy, thereby creating cavitation bubbles within the catheter system 1710. The openings 1738 within the tip 1722 may reduce the size of the bubble formed within the catheter system 1710 and/or reduce the likelihood that the bubble will expand toward the distal end of the catheter system 1710.

In some embodiments, the devices and methods of the present disclosure can also be used deliver pressure waves to ablate a vascular occlusion using a substantially solid light absorbing material instead of liquid medium. In some circumstances, pairing a laser that emits a specific wavelength of light with a light absorbing material designed to absorb light at that wavelength can significantly increase the energy efficiency of the resultant pressure waves produced by the reaction. The use of such pairings can ultimately reduce the energy input required to treat a vascular occlusion, which can increase the safety of the procedure and reduce costs. For example, the catheters according to embodiments of the present disclosure can be filled with air or a substantially inert liquid medium (for example, saline) instead of contrast medium, which can significantly reduce the amount and size of cavitation bubbles produced along with the pressure waves. Because the pressure waves can propagate outside of the catheter to ablate a vascular occlusion, it can be advantageous in some circumstances to reduce (for example, by filling the catheter with saline) or eliminate (for example, by filling the catheter with air or inert gas) the production of cavitation bubbles. In other cases, liquid medium delivered to the distal end of the catheter can be pre-treated to remove the amount of gas dissolved in it using methods known to one of ordinary skill in the art based on the present disclosure, as this can also reduce the amount of cavitation bubbles generated along with the pressure waves.

Suitable light absorbing material can be any agent capable of absorbing light energy and producing a pressure wave. For example, the light absorbing material can contain an aromatic hydrocarbon with iodine bonded to it, such as iodinated x-ray contrasts. Low osmolar, non-ionic, iodinated, and radio-opaque contrasts are also suitable light absorbing materials that can be used to produce pressure waves. Other light absorbing materials include, but are not limited to, iodinated contrasts such as Diatrizoic acid, Metrizoic acid, lodamide, lotalamic acid, loxitalamic acid, loglicic acid, Acetrizoic acid, locarmic acid, Methiodal, Diodone, Metrizamide, lohexol, loxaglic acid, lopamidol, lopromide, lotrolan, loversol, lopentol, lodixanol, lomeprol, lobitridol, loxilan, lodoxamic acid, lotroxic acid, loglycamic acid, Adipiodone, lobenzamic acid, lopanoic acid, locetamic acid, Sodium iopodate, Tyropanoic acid, Calcium iopodate, lopydol, Propyliodone, lofendylate, Lipiodol, non-iodinated contrasts such as Barium sulfate, MRI contrast agents such as Gadobenic acid, Gadobutrol, Gadodiamide, Gadofosveset, Gadolinium, Gadopentetic acid, Gadoteric acid, Gadoteridol, Gadoversetamide, Gadoxetic acid, Ferric ammonium citrate, Mangafodipir, Ferumoxsil, and Ferristene Iron oxide nanoparticles, Perflubron, Glucose and other carbohydrates, Albumen and other proteins, Nitroglycerin or other vasodilators, Hydrocarbons such as Oils, Alcohols, or other organic functional groups (Amines, Alkanes, Carboxyl, and the like), blood/tissue products such as Platelet Rich Plasma (PRP), packed red cells, plasma, platelet, fat, Charcoal, biocompatible materials such as stainless steel, biopolymers, and bioceramics, or other pharmacological agents which contain a combination of aromatic carbon rings and functional groups such as Salicylic acid, Acetylsalicylic acid, Methyl salicylate, Mesalazine, Aspirin, Acetaminophen, Ibuprofen, Clopidogrel, or other pharmacological and/or biological agents which may be compatible with the medical procedures described herein.

Suitable light absorbing material can also include those materials capable of absorbing wavelengths in the UV spectrum. For example, light absorbing materials can include, but are not limited to, PABA, Padimate 0, Phenylbenzimidazole sulfonic acid, Cinoxate, Dioxybenzone, Oxybenzone, Homosalate, Menthyl anthranilate, Octocrylene, Octyl methoxycinnamate, Octyl salicylate, Sulisobenzone, Trolamine salicylate, Avobenzone, Ecamsule, 4-Methylbenzylidene camphor, Tinosorb M, Tinosorb S, Tinosorb A2B, Neo Heliopan AP, Mexoryl XL, Benzophenone-9, Uvinul T 150, Uvinul A Plus, Uvasorb HEB, Parsol SLX, or Amiloxate, Silicon and its various atomic structures, Cadmium telluride, Copper indium gallium selenide, Gallium arsenide, Ruthenium metalorganic dye, Polyphenylene vinylene, Copper phthaloncyanine, Carbon fullerenes and derivatives, Carbon compounds such as Graphite, Graphene, Diamond, Charcoal, Titianium and oxides, Nickel and oxides, Gold, Silver, Zinc and oxides, Tin and oxides, Aluminum and oxides, or alloys or ceramics of the preceding metals.

Light absorbing material may be combined with various other compounds to facilitate their attachment to a substrate. For example, light absorbing materials may be combined with various compounds (for example, solubilizing agents) that aid in the generation of a solution or mixture comprising the light absorbing material, which can be used to coat the substrate. In some embodiments, a biodegradable and biocompatible hydrophobic polymer may be used as a light absorbing material. For example, the biodegradable and biocompatible hydrophobic polymer may be poly(glycerol sebacate acrylate) (PGSA), or variations and combinations thereof, which can be crosslinked using ultraviolet light. Ultraviolet light may be emitted from the distal end of a catheter, which may be disposed within or outside of a sheath, to activate the PGSA, for example.

Other light absorbing material can also include agents having adhesive-like properties, and in some cases, the light absorbing properties of these agents can be in addition to, or independent of, their use as adhesives. For example, light absorbing materials can include, but are not limited to, cyanoacrylates, bovine serum albumin (BSA)-glutaraldehyde, fibrin sealants, gelatin matrix thrombin, gelatin sponge, oxidized cellulose, collagen sponge, collagen fleece, recombinant factor VIIa, and the like. In some embodiments, the light absorbing material may comprise hydrophobic functional groups, such as hexanoyl (Hx; C6), palmitoyl (Pam; C16), stearoyl (Ste; C18), and oleoyl (Ole; C18 unsaturated) groups, so as to resist being washed out or disengaged from their substrate in predominately aqueous environments (for example, vascular tissue). Such light absorbing materials can include, but are not limited to, 10Ole-disuccinimidyl tartrate, 10Ste-disuccinimidyl, and variations and combinations thereof.

Light absorbing material can be configured to exhibit high absorption of light energy from an emitter. Light energy can be emitted at any suitable wavelength capable of generating pressure waves. Light energy can be emitted between about 1 nanometer and about 1 millimeter. In some cases, light can be emitted from about 10 nanometers to about 5000 nanometers. In some cases, light can be emitted from about 100 nanometers to about 1000 nanometers. In some cases, light can be emitted from about 250 nanometers to about 750 nanometers. In some cases, light can be emitted from about 300 nanometers to about 600 nanometers. In still other cases, light can be emitted from about 300 nanometers to about 350 nanometers.

In general, the light absorbing material can be located anywhere within a catheter, so long as it generally intersects with the path of light emitted from the optical fibers. In some embodiments, the light absorbing material may be substantially solid (for example, stable in a generally solid state, such as metals and metal alloys). Substantially solid light absorbing material can be used to construct various portions of the components of the catheter, and/or substantially solid light absorbing material can be used to construct a separate structure that is independent of another catheter component.

In some embodiments, the light absorbing material can be applied to a separate supporting structure (that is, a support structure that is not predominately made of light absorbing material, or a support structure that is not being used as a light absorbing material) and used to generate pressure waves using the devices and methods of the present disclosure. In some embodiments, the light absorbing materials are stable only in liquid, gel, or semi-liquid forms. In these embodiments, the light absorbing material can be included as part of a formulation or coating that is suitable for application to a support structure, such as impregnated in hydrogel or other solid support matrix. In some embodiments, the light absorbing materials can be part of a formulation or coating containing other agents that facilitate their placement on and/or adherence to a support structure. For example, solid absorbing materials can be formulated with coating agents, thickening agents, adhesive agents, and/or other pharmaceutical or biological agents that are suitable for use with the devices and methods of the present disclosure.

Referring to FIG. 23, the distal end of a catheter system 1810 including a light absorbing material according to an embodiment of the present disclosure is illustrated. The catheter system 1810 is similar to the catheter system 1710. That is, the catheter system 1810 includes a sheath 1820 (which is hidden in FIG. 23 to illustrate internal components of the catheter system 1810) that has a partially-closed tip 1822. The sheath 1820 carries a laser catheter, such as the laser catheter 1010 described herein, and the laser catheter 1010 may translate distally and/or proximally within the sheath 1820. As shown in FIG. 23, the sheath 1820 may taper proceeding distally toward the tip 1822. Alternatively, the sheath 1820 may include a flat surface that receives the tip 1822. As another alternative, the tip 1822 may have a similar size to the lumen of the sheath 1820 and be press-fittingly received in the lumen. In order to ensure that a cavity remains between the distal end of the laser catheter 1010 and the proximal end of the tip 1822 of the sheath 1820, the sheath 1820 may include one or more internal stops 1826. The shape of the tip 1822 may be configured similar to the tips 180 illustrated and described with respect to FIGS. 2-6 such that the catheter system 1810, including the laser catheter tip 1822, is configured such that the energy produced by the pressure waves is captured within the cavity and the forces generated by the pressure waves propagate longitudinally, including in a forward (that is, parallel with the vessel) direction, thereby increasing the tip's ability to disrupt, destroy and/or penetrate the vascular obstructions.

The tip 1822 may include the same features and structures as the tip 1722. In addition, the tip 1822 includes a light absorbing material support structure 1824. The light absorbing material support structure 1824 acts as a substrate for the application of light absorbing material, which may be any of the light absorbing materials described herein. Light absorbing material can be applied as a coating, as described herein, on the proximal end of the tip 1822 within the cavity of the catheter system 1810, and light absorbing material support structure 1824 can be positioned such that it generally intersects with the path of the light emitted from the distal end of the laser catheter 1010.

In some embodiments, the light absorbing material can be applied to various surfaces within the catheter system 1810 itself instead of being applied to a support structure. For example, the light absorbing material can be applied as a coating to the inner surface of the catheter system 1810 or portions thereof (such as the inner surface of the sheath 1820). The laser light emitted from the distal end of the laser catheter 1010 can be directed upward and/or outward such that it can react with the light absorbing material to generate a pressure wave, without the need for an additional support structure.

Referring to FIG. 24, the distal end of a catheter 1910 including a light absorbing material according to an embodiment of the present disclosure is illustrated. The catheter 1910 is similar to the catheter illustrated in FIG. 5A. That is, the catheter 1910 includes a distal end having a tip 1912 that comprises a non-metallic component in lieu of a metallic (for example, stainless steel) solid or hollow construction. The catheter 1910 includes an outer sheath 1914, an inner sheath 1916 disposed concentrically and/or radially within the outer sheath 1914, and one or more optical fibers 1918 disposed concentrically and/or radially within the inner sheath 1916. The distal end of the outer sheath 1914 is directly coupled (via a press fit and/or a weld) to the tip 1912. The inner sheath 1916 and the one or more optical fibers 1918 are not directly coupled to the tip 1912. Rather, the inner sheath 1916 and the one or more optical fibers 1918 are disposed proximate the tip 1912, thereby forming a cavity among the outer sheath 1914, the inner sheath 1916, one or more optical fibers 1918, and the tip 1912.

The catheter 1910 may include a shield 1920 disposed axially between the distal end of the inner sheath 1916 and the proximal end of the tip 1912, and disposed radially between the one or more optical fibers 1918 and the outer sheath 1914. The shield 1920, which is depicted as a generally cylindrical tube, increases the pressure waves' resistance in the radial direction, thereby reducing the ability of the pressure waves to travel radially towards the outer sheath 1914. The configuration of the cylindrically-shaped shield 1920 allows for a reduced resistance in the longitudinal direction, in comparison to the radial direction, thereby increasing the tip's ability to translate in a forward/backward direction. The cylindrically-shaped shield 1920 may also be configured such that its diameter is greater (or less) at its proximal end in comparison to its distal end, thereby potentially tapering in either the proximal or distal direction and concentrating the pressure waves towards the center of the tip 1912. The shield 1920 may also serve to create a sealed cavity at the distal end of the catheter 1910, thereby preventing the leakage of the liquid medium through the outer sheath 1914 because a portion of the shield overlaps with a portion of the outer sheath 1914 that may be porous.

The inner sheath also includes one or more lumens for passage of liquid medium into the cavity. The distal end(s) of the one or more optical fibers 1918 are proximate, at, or distal the distal end of the inner sheath 1916. Again, one or more emitters are disposed at the distal end of the one or more optical fibers 1918. The emitter(s) are in direct contact with the liquid medium, such that when laser light energy is emitted from the emitter(s), the liquid medium absorbs the emitted light, which in turn produces pressure waves and generates pressure waves and/or cavitation bubbles that produce additional pressure waves.

The tip 1912 has a circular construction, thereby creating a collar for the distal end of the outer sheath 1914. The tip 1912 also includes a flexible membrane 1922 at its distal end. For example, the membrane 1922 may be constructed of Mylar and be adhesively bonded to the distal end of the tip 1912 in an orientation perpendicular to the longitudinal axis. In addition the membrane may be compliant in order to deflect and engage the shape of the calcified cap, total occlusion or lesion. In some embodiments, the membrane 1922 may be constructed of an elastic or hyperelastic material (for example, nitinol).

The membrane 1922 carries a light absorbing material support structure 1924 within the cavity. The light absorbing material support structure 1924 acts as a substrate for the application of light absorbing material, which may be any of the light absorbing materials described herein. In other embodiments, the light absorbing material support structure 1924 could be carried by the membrane 1922 outside of the cavity, or light absorbing material can be applied as a coating, as described herein, on the membrane 1922 (with the cavity or outside of the cavity). In any case, the light absorbing material can be positioned such that it generally intersects with the path of the light emitted from the distal end of the optical fiber(s) 1918. In some embodiments and as shown in Fig. 24, the light absorbing material support structure 1924 may cover the entire membrane 1922 within the cavity. In other embodiments, the light absorbing material support structure 1924 may partially cover the membrane 1922 within the cavity. In these embodiments, the position and/or size of the light absorbing material support structure 1924 on the membrane 1922 may affect the location of cavitation bubble formation and collapse, which in turn may affect the deflected shape of the membrane 1922. In some embodiments, the light absorbing material can be applied to various surfaces other within the catheter 1910 instead of being applied to a support structure. For example, the light absorbing material can be applied as a coating to the inner surface of the shield 1920 or portions thereof.

To treat a subject having a vascular occlusion, the distal end of the catheter 1910, particularly the tip 1912 is positioned adjacent to the vascular obstruction with the membrane 1922 adjacent the vascular occlusion. The liquid medium may be delivered to the cavity from the one more lumens within the inner sheath 1916 through one or more liquid medium ports or between the outer sheath and the inner sheath or laser catheter 1910. When the laser system is activated, light energy travels through one or more optical fibers until the light energy is released from the emitter(s) at the end of the one or more optical fibers. As the light absorbing material and the liquid medium absorb the light energy, the liquid medium rapidly expands and contracts, creating a pressure wave and/or a vapor bubbles that propagate pressure waves through the liquid medium. The energy produced by the pressure waves is captured within the closed system provided by the cavity and transferred to the vascular occlusion through the flexible membrane 1922. The transfer of the energy produced by the pressure waves to the vascular occlusion is sufficient to disrupt calcium deposits and/or fibrous tissue within the vascular occlusion. The forces generated by the pressure waves can propagate longitudinally in forward (that is, parallel to the vessel). Pressure waves produced in this manner can also be used to increase vessel compliance prior to performing another procedure, such as a traditional balloon angioplasty.

Although a large portion of this disclosure includes a discussion of laser ablation catheters used in conjunction with a sheath assembly to perform CAD and PAD procedures, other the laser ablation catheter and sheath assembly may be used to perform other types of medical and/or surgical procedures. Laser catheters typically transmit laser energy through optical fibers housed in a relatively flexible tubular catheter inserted into a body lumen, such as a blood vessel, ureter, fallopian tube, cerebral artery and the like to remove obstructions in the lumen. Catheters used for laser angioplasty and other procedures may have a central passageway or tube which receives a guide wire inserted into the body lumen (for example, vascular system) prior to catheter introduction. The guide wire facilitates the advancement and placement of the catheter to the selected portion(s) of the body lumen for laser ablation of tissue.

The present disclosure, in various aspects, embodiments, and configurations, includes components, methods, processes, systems and/or apparatus substantially as depicted and described herein, including various aspects, embodiments, configurations, sub combinations, and subsets thereof. Those of skill in the art will understand how to make and use the various aspects, aspects, embodiments, and configurations, after understanding the present disclosure. The present disclosure, in various aspects, embodiments, and configurations, includes providing devices and processes in the absence of items not depicted and/or described herein or in various aspects, embodiments, and configurations hereof, including in the absence of such items as may have been used in previous devices or processes, for example, for improving performance, achieving ease and\or reducing cost of implementation.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more, aspects, embodiments, and configurations for the purpose of streamlining the disclosure. The features of the aspects, embodiments, and configurations of the disclosure may be combined in alternate aspects, embodiments, and configurations other than those discussed above. This method of disclosure is not to be interpreted as reflecting an intention that the claimed disclosure requires more features than are expressly recited in each claim. Rather, as the following claims reflect, inventive aspects lie in less than all features of a single foregoing disclosed aspects, embodiments, and configurations. Thus, the following claims are hereby incorporated into this Detailed Description, with each claim standing on its own as a separate preferred embodiment of the disclosure.

Moreover, though the description of the disclosure has included description of one or more aspects, embodiments, or configurations and certain variations and modifications, other variations, combinations, and modifications are within the scope of the disclosure, for example, as may be within the skill and knowledge of those in the art, after understanding the present disclosure.

## Claims

1. A catheter comprising:
an outer sheath having a proximal end, a distal end and a porous attenuating member disposed adjacent the distal end, wherein the porous attenuating member comprises a plurality of openings, and wherein the outer sheath comprises a non-porous solid biocompatible layer covering at least a portion of the plurality of openings;
an inner sheath having a proximal end and a distal end, wherein the inner sheath is disposed radially within the outer sheath;
one or more optical fibers disposed within the inner sheath extending from a proximal portion of the inner sheath to the distal end of the inner sheath, wherein a proximal end of the one or more optical fibers is coupled to a laser generator; and
at least one emitter coupled to the one or more optical fibers, wherein the at least one emitter is disposed radially within the outer sheath such that the at least one emitter is disposed radially within the porous attenuating member.

2. The catheter of claim 1, wherein the porous attenuating member is constructed to form a semi-rigid biocompatible structure.

3. The catheter of claim 1, wherein the porous attenuating member is constructed to form a rigid biocompatible structure.

4. The catheter of claim 1, wherein the porous attenuating member is integrally disposed within the solid biocompatible layer.

5. The catheter of claim 1, wherein the porous attenuating member is disposed on the interior of the solid biocompatible layer.

6. The catheter of claim 1, wherein the plurality of openings comprise at least one of the following shapes: circle; oval; triangle; square; rectangle; polygon; diamond; pentagon; hexagon; heptagon; octagon; nonagon; and decagon.

7. The catheter of claim 1, wherein the plurality of openings are formed by a helical structure wound in a first direction.

8. The catheter of claim 7, wherein the porous attenuating member includes a metallic coil.

9. The catheter of claim 1, wherein the at least one emitter is configured to emit laser light energy at wavelengths of between about 300 nanometers to about 350 nanometers, at pulse durations between about 100 nanoseconds to about 150 nanoseconds, and at frequencies between about 1 pulse per second to about 250 pulses per second.

10. The catheter of any preceding claim, wherein the at least one emitter is configured to be activated so as to transmit pulses of light energy into a liquid medium capable of absorbing light and producing pressure waves, wherein transmitting the pulses of light energy generates a plurality of propagating pressure waves that pass through the outer sheath and the openings in the porous attenuating member.

11. The catheter of claim 10, wherein the liquid medium is contrast medium or contrast solution.

12. The catheter of claim 11, wherein the liquid medium is any one of iodine-containing contrast medium or gadolinium contrast medium.

13. The catheter of claim 10, wherein the liquid medium is configured to exhibit high absorption of light energy emitted from the at least one emitter at wavelengths of between about 1 nanometer to about 1 millimeter, at pulse durations between about 1 nanosecond to about 1 second, and at frequencies between about 1 pulse per second to about 500 pulses per second.

14. The catheter of claim 1, wherein the at least one emitter is two or more single-fiber emitters.

15. The catheter of claim 1, wherein the at least one emitter is configured to translate within the outer sheath.

16. The catheter of claim 1, wherein the distal end of the outer sheath comprises an open configuration.

## Patentansprüche

1. Katheter, umfassend:
einen äußeren Schaft mit einem proximalen Ende, einem distalen Ende und einem porösen Dämpfungselement, die neben dem distalen Ende angeordnet sind, wobei das poröse Dämpfungselement mehrere Öffnungen umfasst, und wobei der äußere Schaft eine nicht poröse feste biokompatible Schicht umfasst, die mindestens einen Teil der Vielzahl von Öffnungen bedeckt;
einen inneren Schaft mit einem proximalen Ende und einem distalen Ende, wobei der innere Schaft radial innerhalb des äußeren Schafts angeordnet ist;
eine oder mehrere optische Fasern, die in dem inneren Schaft angeordnet sind und sich von einem proximalen Abschnitt des inneren Schafts bis zum distalen Ende des inneren Schafts erstrecken, wobei ein proximales Ende der einen oder mehreren optischen Fasern mit einem Lasergenerator gekoppelt ist; und
mindestens einen Emitter, der mit der einen oder den mehreren optischen Fasern gekoppelt ist, wobei der mindestens eine Emitter radial innerhalb des äußeren Schafts angeordnet ist, so dass der mindestens eine Emitter radial innerhalb des porösen Dämpfungselements angeordnet ist.

2. Katheter nach Anspruch 1, wobei das poröse Dämpfungselement so aufgebaut ist, dass es eine halbstarre biokompatible Struktur bildet.

3. Katheter nach Anspruch 1, wobei das poröse Dämpfungselement so aufgebaut ist, dass es eine starre biokompatible Struktur bildet.

4. Katheter nach Anspruch 1, wobei das poröse Dämpfungselement einstückig in der festen biokompatiblen Schicht angeordnet ist.

5. Katheter nach Anspruch 1, wobei das poröse Dämpfungselement im Inneren der festen biokompatiblen Schicht angeordnet ist.

6. Katheter nach Anspruch 1, wobei die mehreren Öffnungen mindestens eine der folgenden Formen umfassen: Kreis; Oval; Dreieck; Viereck; Rechteck; Polygon; Diamant; Fünfeck; Sechseck; Siebeneck; Achteck; Neuneck; und Zehneck.

7. Katheter nach Anspruch 1, wobei die mehreren Öffnungen durch eine in eine erste Richtung gewickelte spiralförmige Struktur gebildet sind.

8. Katheter nach Anspruch 7, wobei das poröse Dämpfungselement eine Metallspule enthält.

9. Katheter nach Anspruch 1, wobei der mindestens eine Emitter konfiguriert ist, um Laserlichtenergie bei Wellenlängen zwischen ungefähr 300 Nanometern bis ungefähr 350 Nanometern, bei Pulsdauern zwischen ungefähr 100 Nanosekunden bis ungefähr 150 Nanosekunden und bei Frequenzen zwischen ungefähr 1 Impuls pro Sekunde bis ungefähr 250 Impulse pro Sekunde zu emittieren.

10. Katheter nach einem der vorhergehenden Ansprüche, wobei der mindestens eine Emitter so konfiguriert ist, dass er aktiviert wird, um Impulse von Lichtenergie in ein flüssiges Medium zu übertragen, das Licht absorbieren und Druckwellen erzeugen kann, wobei das Übertragen der Impulse der Lichtenergie eine Vielzahl von sich ausbreitenden Druckwellen erzeugt, die durch den äußeren Schaft und die Öffnungen in dem porösen Dämpfungselement gehen.

11. Katheter nach Anspruch 10, wobei das flüssige Medium Kontrastmittel oder Kontrastlösung ist.

12. Katheter nach Anspruch 11, wobei das flüssige Medium eines von Iod enthaltendem Kontrastmittel oder Gadolinium-Kontrastmittel ist.

13. Katheter nach Anspruch 10, wobei das flüssige Medium konfiguriert ist, um eine hohe Absorption von Lichtenergie zu zeigen, die von dem mindestens einen Emitter bei Wellenlängen zwischen ungefähr 1 Nanometer bis ungefähr 1 Millimeter, bei Pulsdauern zwischen ungefähr 1 Nanosekunde bis ungefähr 1 Sekunde und bei Frequenzen zwischen ungefähr 1 Impuls pro Sekunde bis ungefähr 500 Impulsen pro Sekunde emittiert wird.

14. Katheter nach Anspruch 1, wobei der mindestens eine Emitter zwei oder mehr Einzelfaseremitter ist.

15. Katheter nach Anspruch 1, wobei der mindestens eine Emitter konfiguriert ist, um sich innerhalb des äußeren Schafts zu verschieben.

16. Katheter nach Anspruch 1, wobei das distale Ende des äußeren Schafts eine offene Konfiguration aufweist.

## Revendications

1. Cathéter comprenant:
une gaine externe ayant une extrémité proximale, une extrémité distale et un élément d'atténuation poreux disposé adjacent à l'extrémité distale, où l'élément d'atténuation poreux comprend une pluralité d'ouvertures, et où la gaine externe comprend une couche biocompatible solide non poreuse recouvrant au moins un partie de la pluralité d'ouvertures;
une gaine interne ayant une extrémité proximale et une extrémité distale, où la gaine interne est disposée radialement à l'intérieur de la gaine externe;
une ou plusieurs fibres optiques disposées à l'intérieur de la gaine interne s'étendant d'une partie proximale de la gaine interne à l'extrémité distale de la gaine interne, où une extrémité proximale de l'une ou des plusieurs fibres optiques est couplée à un générateur laser; et
au moins un émetteur couplé à l'une ou aux plusieurs fibres optiques, où l'au moins un émetteur est disposé radialement à l'intérieur de la gaine extérieure de telle sorte que l'au moins un émetteur est disposé radialement à l'intérieur de l'élément d'atténuation poreux.

2. Cathéter selon la revendication 1, dans lequel l'élément d'atténuation poreux est construit pour former une structure biocompatible semi-rigide.

3. Cathéter selon la revendication 1, dans lequel l'élément d'atténuation poreux est construit pour former une structure biocompatible rigide.

4. Cathéter selon la revendication 1, dans lequel l'élément d'atténuation poreux est disposé intégralement à l'intérieur de la couche biocompatible solide.

5. Cathéter selon la revendication 1, dans lequel l'élément d'atténuation poreux est disposé à l'intérieur de la couche solide biocompatible.

6. Cathéter selon la revendication 1, dans lequel la pluralité d'ouvertures comprend au moins l'une des formes suivantes: cercle; ovale; triangle; carré; rectangle; polygone; diamant; pentagone; hexagone; heptagone; octogone; nonagone; et décagone.

7. Cathéter selon la revendication 1, dans lequel la pluralité d'ouvertures est formée par une structure hélicoïdale enroulée dans une première direction.

8. Cathéter selon la revendication 7, dans lequel l'élément d'atténuation poreux comprend une bobine métallique.

9. Cathéter selon la revendication 1, dans lequel l'au moins un émetteur est configuré pour émettre de l'énergie lumineuse laser à des longueurs d'onde entre environ 300 nanomètres et environ 350 nanomètres, à des durées d'impulsion entre environ 100 nanosecondes et environ 150 nanosecondes, et à des fréquences entre environ 1 impulsion par seconde et environ 250 impulsions par seconde.

10. Cathéter selon l'une quelconque des revendications précédentes, dans lequel l'au moins un émetteur est configuré pour être activé de manière à transmettre des impulsions d'énergie lumineuse dans un milieu liquide capable d'absorber la lumière et de produire des ondes de pression, où la transmission des impulsions d'énergie lumineuse génère une pluralité d'ondes de pression se propageant qui traversent la gaine extérieure et les ouvertures dans l'élément d'atténuation poreux.

11. Cathéter selon la revendication 10, dans lequel le milieu liquide est un milieu de contraste ou une solution de contraste.

12. Cathéter selon la revendication 11, dans lequel le milieu liquide est l'un quelconque parmi un milieu de contraste contenant de l'iode ou un milieu de contraste au gadolinium.

13. Cathéter selon la revendication 10, dans lequel le milieu liquide est configuré pour présenter une absorption élevée de l'énergie lumineuse émise de l'au moins un émetteur à des longueurs d'onde entre environ 1 nanomètre et environ 1 millimètre, à des durées d'impulsion entre environ 1 nanoseconde et environ 1 seconde, et à des fréquences entre environ 1 impulsion par seconde et environ 500 impulsions par seconde.

14. Cathéter selon la revendication 1, dans lequel l'au moins un émetteur est constitué de deux ou plusieurs émetteurs monofibres.

15. Cathéter selon la revendication 1, dans lequel l'au moins un émetteur est configuré pour se déplacer par translation dans la gaine extérieure.

16. Cathéter selon la revendication 1, dans lequel l'extrémité distale de la gaine externe comprend une configuration ouverte.
